(19) Europäisches Patentamt
European Patent Office
Office européen des brevets

(11) **EP 2 305 827 A1**

(12) **EUROPEAN PATENT APPLICATION**

(43) Date of publication:
**06.04.2011 Bulletin 2011/14**

(21) Application number: **10189300.6**

(22) Date of filing: **14.11.2006**

(51) Int Cl.:
***C12P 21/08*** *(2006.01)* ***A61K 31/7088*** *(2006.01)*
***A61K 39/00*** *(2006.01)* ***A61K 39/395*** *(2006.01)*
***A61K 48/00*** *(2006.01)* ***A61P 35/00*** *(2006.01)*
***C07K 16/18*** *(2006.01)* ***C12N 5/12*** *(2006.01)*
***C12Q 1/02*** *(2006.01)* ***C12Q 1/68*** *(2006.01)*
***G01N 33/53*** *(2006.01)* ***G01N 33/574*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **14.11.2005 US 735859 P**

(62) Document number(s) of the earlier application(s) in accordance with Art. 76 EPC:
**06817649.4 / 1 951 891**

(71) Applicant: **Université Laval**
**Québec,**
**Québec G1K 7P4 (CA)**

(72) Inventors:
- **Bergeron, Alain**
  **Quebec, Québec G1H 1T4 (CA)**
- **Picard, Valérie**
  **Quebec, Québec G1H 1T4 (CA)**
- **Larue, Hélène**
  **Quebec, Québec G1H 1T4 (CA)**
- **Fradet, Yves**
  **Quebec, Québec G1K 3W1 (CA)**

(74) Representative: **Maureau, Philippe et al**
**Cabinet Germain & Maureau**
**12, rue Boileau**
**B.P. 6153**
**69466 Lyon Cedex 06 (FR)**

Remarks:
This application was filed on 28-10-2010 as a divisional application to the application mentioned under INID code 62.

(54) **Cancer antigen MAGE-A9 and uses thereof**

(57) The present invention relates to the detection of MAGE-A9 in a biological sample and to the diagnosis, prevention and treatment of MAGE-A9-associated aberrant cell growth, through the targeting of the MAGE-A9 polypeptide and/or polynucleotide. Diagnosis is accomplished by examining or monitoring cells for perturbations in MAGE-A9 expression. Prevention and treatment are accomplished by administering to a subject MAGE-A9 as a protein or a fragment thereof or under form of a DNA expression vector, as well as by administering to a subject a MAGE-A9 gene product-binding agent. Compositions and commercial kits are also provided.

EP 2 305 827 A1

## Description

### TECHNICAL FIELD

The present invention relates to the field of tumor antigens for diagnosis, prognosis, and treatment of cancer, in particular bladder cancer, but also ovarian cancer, kidney cancer, lung cancer, liver cancer, testis cancer, skin cancer, blood cancer and lymphomas.

### BACKGROUND OF THE INVENTION

Bladder cancer is the fifth most common cancer in the Western world. Histologically, most of these tumors are transitional cell carcinomas (TCCs) and the majority of them (75%) are superficial tumors confined to the bladder mucosa. Although these tumors present a good prognosis, they are associated with a high rate of recurrence since more than 60% of them will reappear in a short period of time after surgery, and a significant proportion of these tumors (5 to 25%) will even progress toward more aggressive disease. Because of the recurring nature of this cancer, the treatment of superficial bladder tumors represents a heavy burden for our health systems and new treatments that could improve the management of this cancer are needed.

Intravesical instillation of bacille Calmette-Guérin (BCG) is presently the most effective agent to prevent the recurrence of superficial bladder tumors. In fact, about half of the subjects at high risk of recurrence will remain free of disease after receiving a BCG immunotherapy following tumor resection. However BCG is associated with important side effects which reduce the compliance of the subjects to the treatment. Nevertheless, the efficacy of this non-specific immunotherapy suggests that immunotherapeutic treatments such as cancer vaccines targeting specific tumor-associated antigens might be more effective and associated with less secondary effects than BCG treatment.

Cancer/Testis (CT) antigens are expressed in a large variety of tumors but their expression in normal tissues is mostly restricted to gametogenic tissues which are immunoprivileged because of their lack or low expression of HLA molecules. Because of their tumor-restricted expression, CT antigens appear to be ideal targets for immunotherapy. Moreover, several studies have shown the existence of natural cellular and humoral responses against some CT antigens, indicating that they are relevant targets for cancer immunotherapy. However, a major drawback to their use as target antigens is the low level of expression of some of these proteins and their highly heterogeneous expression between and within tumor tissues.

CT gene expression has been shown to be regulated by epigenetic mechanisms such as DNA methylation and histone deacetylation (HDAC). Several new chemotherapeutic agents aiming at inhibiting DNA methylation or HDAC are presently being clinically tested. Some of these drugs, such as the DNA methylation inhibitor 5-aza-2-deoxycytidine (5-AZA-DC), have been shown to be potent inducers of the expression of several CT genes. It has also been shown that the combination of 5-AZA-DC with HDAC inhibitors such as trichostatin A or depsipetide could even induce stronger expression of CT genes. It can be considered that a treatment combining the use of these drugs to modulate the expression of CT antigens with a cancer vaccine targeting these same antigens might be efficient to improve the immune response and lead to clinical responses.

Tumor specific T cells, derived from cancer subjects, bind and lyse tumor cells. This specificity is based on their ability to recognize short amino acid sequences (epitopes) presented on the surface of the tumor cells by MHC class I and, in some cell types, class II molecules. These epitopes are derived from the proteolytic degradation of cellular proteins called tumor antigens encoded by genes that are either uniquely or aberrantly expressed in tumor or cancer cells.

The availability of specific anti-tumor T cells has enabled the identification of tumor antigens and subsequently the generation of cancer vaccines designed to provoke an anti-tumor immune response. Anti-tumor T cells can be found in cancer subjects, including in the blood (where they can be found in the peripheral blood mononuclear cell fraction), in primary and secondary lymphoid tissue (e.g., the spleen), in ascites fluid (tumor-associated lymphocytes or TALs) or within the tumor itself (tumor-infiltrating lymphocytes or TILs). Of these, TILs have been the most useful in the identification of tumor antigens and tumor antigen-derived peptides recognized by T cells. The introduction of an antigen into an animal has been widely used for the purposes of modulating the immune response, or lack thereof, to the antigen for a variety of purposes. These include vaccination against pathogens, induction of an immune response to a cancerous cell, reduction of an allergic response, reduction of an immune response to a self antigen that occurs as a result of an autoimmune disorder, reduction of allograft rejection, and induction of an immune response to a self antigen for the purpose of contraception.

In the treatment of cancer, a variety of immunotherapeutic approaches have been taken to generate populations of cytotoxic T lymphocytes which specifically recognize and lyse tumor cells. Many of these approaches depend in part on identifying and characterizing tumor-specific antigens.

Regarding bladder cancer, few cancer-specific antigens have yet been identified for the purpose of diagnosis and being capable to induce in a subject an efficient immune response against the tumor.

Considering the state of the art, there is still a need for new tumor antigens with new properties for diagnosis, prognosis and therapy of different cancers.

SUMMARY OF THE INVENTION

One aim of the present invention is to provide methods and means for the detection of MAGE-A9 antigens and for diagnosing and treating MAGE-A9-associated aberrant cell growth disorders such as MAGE-A9-associated cancers.
Another aim of the present invention is to provide an antibody produced by a cell line identified at the International Depositary Authority of Canada under accession number 020805-01 or 020805-02.
Another aim of the present invention is to provide a cell line identified at the International Depositary Authority of Canada under accession number 020805-01 or 020805-02.
Another aim of the present invention is to provide method for detecting an aberrant cell growth in a subject comprising:

a) determining a parameter in a biological sample obtained from said subject, said parameter being at least one of:

- a MAGE-A9-encoding polynucleic acid;
- a MAGE-A9 polypeptide;
- a MAGE-A9 activity;
- the localization of a MAGE-A9-encoding polynucleic acid; and
- the localization of a MAGE-A9 polypeptide; and

b) comparing said parameter to a control parameter, wherein a difference between said parameter and said control parameter is indicative of aberrant cell growth in said subject.

Another aim of the present invention is to provide a method for predicting the susceptibility of a subject to a MAGE-A9-associated cancer comprising:

a) determining a parameter in a biological sample obtained from said subject, said parameter being at least one of:

- a MAGE-A9-encoding polynucleic acid;
- a MAGE-A9 polypeptide level;
- a MAGE-A9 activity;
- the localization of a MAGE-A9-encoding polynucleic acid; and
- the localization of a MAGE-A9 polypeptide; and

b) comparing said parameter to a control parameter, wherein a difference between said parameter and said control parameter is indicative of the susceptibility of the subject to a MAGE-A9-associated cancer.

Another aim of the present invention is to provide a method for determining the aggressiveness of a MAGE-A9-associated cancer in a subject comprising:

a) determining a parameter in a biological sample obtained from said subject, said parameter being at least one of:

- a MAGE-A9-encoding polynucleic acid;
- a MAGE-A9 polypeptide level;
- a MAGE-A9 activity;

- the localization of a MAGE-A9-encoding polynucleic acid; and
- the localization of a MAGE-A9 polypeptide; and

b) comparing said parameter to a control parameter, wherein a difference between said parameter and said control parameter is indicative of the aggressiveness of a MAGE-A9-associated cancer in said subject.

In an embodiement of the present invention, the MAGE-A9-encoding polynucleic acid selected from the group consisting of genomic DNA, cDNA, mRNA, variants thereof and fragments thereof. In a further embodiement of the present invention, the MAGE-A9-encoding polynucleic acid comprises the nucleic acid sequence of SEQ ID NO:1 or a fragment thereof.

In an embodiement of the present invention, the determination is performed with a MAGE-A9-encoding polynucleic acid-binding agent selected from the group consisting of a primer, a probe, a riboprobe, and a complementary nucleic acid. In a further embodiement of the present invention, the determination is performed with a technique selected from the group consisting of genomic analysis, Northern blot analysis, Southern blot analysis, *in situ* hybridization technique, PCR assay, RT-PCR assay, branched DNA analysis, and tissue array analysis.

In an embodiement of the present invention, the MAGE-A9 polypeptide is selected from the group consisting of a MAGE-A9 polypeptide, a MAGE-A9 polypeptide variant and a MAGE-A9 polypeptide fragment. In a further embodiment, the MAGE-A9 polypeptide comprises the amino acid sequence of SEQ ID NO:2 or a fragment thereof.

In an embodiement of the present invention, the determination is performed with a MAGE-A9 polypeptide-binding agent selected from the group consisting of a monoclonal antibody, and a polyclonal antibody. In a further embodiement of the present invention, the monoclonal antibody is produced by a cell line selected from the group consisting of cell lines identified at the International Depositary Authority of Canada under accession number 020805-01 and 020805-02.

In an embodiement of the present invention, the determination is performed with a technique selected from the group consisting of immunohistochemistry, radio-immunoassay, ELISA assay, ELIFA assay, and Western blot analysis.

In another embodiement of the present invention, the aberrant cell growth is indicative of a MAGE-A9-associated hyperplasia or MAGE-A9-associated cancer. In a further embodiement of the present invention, the MAGE-A9-associated cancer is selected from the group consisting of bladder cancer, ovarian cancer, kidney cancer, lung cancer, liver cancer, testis cancer, skin cancer, blood cancer and lymphomas.

In another embodiement of the present invention, the control parameter is a previously determined corresponding parameter from the same subject, and wherein said difference between the determined parameter and the control parameter is indicative of a variation in aberrant cell growth in said subject.

In another embodiement of the present invention, the subject is human.

Another aim of the present invention is to provide a method for preventing a MAGE-A9-associated aberrant cell growth in a subject, said method comprising administering a MAGE-A9 gene product to said subject, thereby inducing an immunization to a MAGE-A9 polypeptide in said subject.

In an embodiement of the present invention, the immunization is a genetic immunization and the MAGE-A9 gene product is a MAGE-A9-encoding polynucleic acid selected from the group consisting of genomic DNA, cDNA, mRNA, variants thereof and fragments thereof. In a further embodiement, the MAGE-A9-encoding polynucleic acid comprises the nucleic acid sequence of SEQ ID NO:1 or a fragment thereof.

In another embodiement of the present invention, the immunization is a cellular immunization. In a further embodiement, the MAGE-A9 gene product is selected from the group consisting of a MAGE-A9 polypeptide, a MAGE-A9 polypeptide variant and a MAGE-A9 polypeptide fragment. In yet a further embodiement, the MAGE-A9 polypeptide comprises the amino acid sequence of SEQ ID NO:2 or a fragment thereof. Various embodiments of the contemplated MAGE-A9-associated hyperplasia or MAGE-A9-associated cancers have been described herein. Various embodiments of the subject have been described herein.

Another aim of the present invention is to provide the use of a MAGE-A9 gene product in the prevention of a MAGE-A9-associated aberrant cell growth in a subject. Various embodiments of the MAGE-A9 gene product, MAGE-A9-encoding polynucleic acid, MAGE-A9 polypeptide, MAGE-A9-associated hyperplasia, MAGE-A9 associated cancer and/or subjet have been described herein.

Another aim of the present invention is to provide the use of a MAGE-A9 gene product in the preparation of a medicament for the prevention of a MAGE-A9-associated aberrant cell growth in a subject. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide a method for inducing an immune response to a MAGE-A9 polypeptide in a subject, said method comprising administering a MAGE-A9 gene product to said subject. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide the use of a MAGE-A9 gene product for inducing an immune response to a MAGE-A9 polypeptide in a subject.

In an embodiement of the present invention, the induced immune response is a cellular immune response. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide the use of a MAGE-A9 gene product in the preparation of a medicament for inducing an immune response to a MAGE-A9 polypeptide in a subject. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide a method for treating a MAGE-A9-associated aberrant cell growth in a subject, said method comprising administering a MAGE-A9 gene product to said subject, thereby inducing an immune response to a MAGE-A9 polypeptide in said subject. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide the use of a MAGE-A9 gene product for the treatment of a MAGE-A9-associated aberrant cell growth in a subject. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide the use of a MAGE-A9 gene product in the preparation of a medicament for treating a MAGE-A9-associated aberrant cell growth in a subject. Various embodiments of the MAGE-A9 gene product, MAGE-A9-encoding polynucleic acid, MAGE-A9 polypeptide, MAGE-A9-associated hyperplasia, MAGE-A9 associated cancer and subjet have been described herein.

Another aim of the present invention is to provide a method for treating a MAGE-A9-associated aberrant cell growth in a subject, said method comprising administering a MAGE-A9 gene product-binding agent and a therapeutic agent to said subject.

In an embodiement of the present invention, the MAGE-A9 gene product-binding agent is physically or chemically linked to said effective therapeutic agent. In a further embodiement of the present invention, the MAGE-A9 gene product-binding agent is selected from the group consisting of a monoclonal antibody, and a polyclonal antibody. In yet a further embodiement of the present invention, the monoclonal antibody is produced by a cell line selected from the group consisting of cell lines identified at the International Depositary Authority of Canada under accession number 020805-01 and 020805-02. In still a further embodiment, the therapeutic agent is a chemotherapeutic agent.

Another aim of the present invention is to provide a composition for treating a MAGE-A9-associated aberrant cell growth in a subject, wherein said composition comprises a MAGE-A9 gene product-binding agen and at least one effective therapeutic agent. Various embodiments of the MAGE-A9 gene product-binding, MAGE-A9-associated hyperplasia, MAGE-A9 associated cancer, therapeutic agent and subjet have been described herein.

In an embodiement of the present invention, the composition is a vaccine composition.

Another aim of the present invention is to provide a kit comprising an antibody produced by a cell line identified in the International Depositary Authority of Canada under accession number 020805-01 or 020805-02 and instruments for its use in the detection of a MAGE-A9 polypeptide in a biological sample from a subject. In an embodiment, the detection of a MAGE-A9 polypeptide is indicative of an aberrant cell growth, of a MAGE-A9-associated hyperplasia and/or a MAGE-A9-associated cancer. Various embodiments of the MAGE-A9 associated cancer have been described herein.

## BRIEF DESCRIPTION OF THE DRAWINGS

Fig. 1 illustrates an acrylamide gel electrophoresis of radioactive RT-PCR products. Amplicons obtained after MAGE-A3, MAGE-A4, MAGE-A8, MAGE-A9 and β-actin cDNA amplification in superficial (Ta-T1) (A), invasive ≥ T2) (B) bladder tumor samples, and in normal urothelia (C). A 473-bp (*) and a 399-bp amplicons (**) are obtained with primers specific for MAGE-A8.

Fig. 2 shows Western blot analysis of the specificity of mAbs 14A11 (A) and 14A12 (B). 293 cells were transiently transfected with plasmids encoding MAGE-A1, -A2, -A3, -A4, -A6, -A8, -A9, -A10, -A11 and -A12. Lysates of the transfectants were tested with mAbs 14A11, 14A12 and as controls for antigen expression, 57B (C) and 6C1 (D), two mAbs cross-reacting with several MAGE-A antigens. NT: Non-transfected cells.

Fig. 3 shows immunohistochemical analysis of MAGE-A9 and MAGE-A4 expression in human testis (A and B) and in a Ta grade 2 tumor (Tum-623) (C and D). Sections from fixed and paraffin-embedded tissues were stained with mAb 14A11 (A and C) or with mAb 57B (B and D) to respectively detect the expression of MAGE-A9 and MAGE-A4. In testis, mAb 14A11 reactivity is associated with primary spermatocytes (Sp) (A) while that of 57B is associated with spermatogonia (Sg) (B). In the Tum-623 sample, mAb 14A11 stains nearly 100% of the cells (C) while mAb 57B stains about 50% of the cells (D). In this tumor sample, staining for both antigens was localized in the cytoplasm but could also be observed in the nuclei. The same area of Tum-623 is shown in C and D. Magnification, A and B: 40X; C and D: 20X.

Fig. 4 illustrates (A) RT-PCR analysis of MAGE-A9 gene expression induction in bladder cancer cell lines treated or not with methylase and/or histone deacetylase inhibitors Apicidin, MS-275 and 4-PB. PCR products were electrophoresed on acrylamide gel, stained with ethidium bromide and visualized under UV, and (B) Western blot analysis of MAGE-A9 polypeptide induction after cell treatments with drugs. Protein were electrophoresed, transferred to nitrocellulose and tested with mAb 14A11. Treatment conditions were: 1, Control (untreated cells); 2, Cells treated with 1 μM 5-AZA-DC for 96 h; 3, Cells treated with 1 μM 5-AZA-DC for 96 h and 0.5 μM Apicidin for 48 h; 4, Cells treated with 0.5 μM Apicidin for 48 h; 5, Cells treated with 1 μM 5-AZA-DC for 96 h and 1 μM MS-275 for 48 h; 6, Cells treated with 1 μM MS-275 for 48 h; 7, Cells treated with 1μM 5-AZA-DC for 96 h and 2 mM 4-PB for 48 h; 8, Cells treated with 2 mM 4-PB for 48 h.

Fig. 5 shows a western blot showing the reactivity of 5 serum samples with the recombinant human MAGE-A9. Only serum sample #0290-S (lane 3) is reactive as a clear band at 45 kDa can be observed. As positive control, a rabbit polyclonal antibody against MAGE-A9 was used to show the presence of the antigen (Ctrl).

Fig. 6 shows the mean expression of MAGE-A4 (light gray) and MAGE-A9 (black) in bladder tumors according to grade and stage, as determined by IHC using mAbs 57B and 14A11.

Fig. 7 shows Kaplan-Meier curves showing recurrence-free survival in function of expression of MAGE-A4 (A) and MAGE-A9 (B) in superficial (Ta-T1) bladder tumors as analysed by IHC using mAbs 57B and 14A11. Absence of MAGE-A9 expression is significantly associated with a longer recurrence-free survival.

Fig. 8 shows a normal kidney tissue section (A) and the immunohistochemical staining of renal cell carcinoma for MAGE-A9 (mAb 14A11) (B, D) and MAGE-A4 (mAb 57B) (C), showing cytoplasmic staining with both mAbs.

Fig. 9 shows immunohistochemical staining of ovarian carcinomas tissue sections for MAGE-A9 (mAb 14A11) and MAGE-A4 (mAb 57B) showing one MAGE-A9 negative tumor (A) and MAGE-A4 (B, D) or MAGE-A9 (C) positive tumors.

DESCRIPTION OF THE PREFERRED EMBODIMENT

The present invention generally relates to methods and means for the detection of MAGE-A9 antigens and for diagnosing, preventing and treating MAGE-A9-associated aberrant cell growth disorders such as MAGE-A9-associated cancers. It has been found by the present inventors that MAGE-A9 polypeptides or peptides, or proteins or peptides closely related to MAGE-A9, are specifically produced by different types of cancer cells. For example, MAGE-A9 is the most frequently expressed marker of superficial and invasive bladder tumors. In this sense, different MAGE-A9-associated cancer cells have been found. A MAGE-A9-associated cancer produces MAGE-A9 at its surface or intracellularly. Cancers that can be considered as MAGE-A9-associated cancers are, for example, but not limited to, bladder cancer, ovarian cancer, kidney cancer, lung cancer, liver cancer, testis cancer, skin cancer, blood cancer and lymphomas. It is understood to someone skilled in the art that the MAGE-A9-associated cancer can be at different stages of development or malignancy, being for example as a primary tumor in an organ or being under the form of a metastasic cancer. Therefore, and as an example, the term "bladder cancer" as used herein is intended to include a bladder tumor as well as a metastasis originating from a bladder tumor.

The present invention now will be described more fully hereinafter with reference to the accompanying drawings, in which preferred embodiments of the invention are shown. This invention, may, however, be embodied in many different forms and should not be construed as limited to the embodiments set forth herein; rather, these embodiments are provided so that this disclosure will be thorough and complete, and will fully convey the scope of the invention to those skilled in the art.

All patents, patent applications, articles and publications mentioned herein, both *supra* and *infra*, are hereby incorporated by reference.

In accordance with the present invention, there is provided monoclonal antibodies produced by a cell line deposited on August 2, 2005 at the International

Depositary Authority of Canada and identified under accession number 020805-01 or 020805-02.

Further in accordance with the present invention, there is provided a method for detecting an aberrant cell growth in a subject by determining a parameter in a biological sample obtained from the subject. The expression "aberrant cell growth" as used herein is intended to represent characteristics of cell growth that are different from the normal or expected growth characteristics of normal cells. The expression is intended to represent unregulated cell growth in the way that the growth of such cells escape the traditional and normal growth regulation mechanisms of normal cells in a healthy organism. It is also intended to reflect characteristics of cell growth that are different from the cell growth characteristics one would expect of cells in culture. Non-limitative examples of aberrant cell growth are hyperplasia and cancer, both states involving cells that are no longer regulated by normal cell growth mechanisms. The expressions "MAGE-A9-associated hyperplasia" and "MAGE-A9-associated cancer" as used herein are intended to represent hyperplasia disorders and cancer where hyperplasic or cancerous cells express (e.g. over-express) MAGE-A9 gene and/or protein. Non-limitative examples of MAGE-A9 cancers include bladder cancer, ovarian cancer, kidney cancer, lung cancer, liver cancer, testis cancer, skin cancer, blood cancer and lymphomas. The expression "biological sample" as used herein is intended to represent any sample that can be taken from a biological organism, including a biopsy. Because of the nature of the present invention, such a sample must contain cells that are able to grow under normal or pathological conditions.

According to the invention, the parameter to be detected in the method described herein can be a MAGE-A9-encoding polynucleic acid, a MAGE-A9 polypeptide, a MAGE-A9 activity, the localization of a MAGE-A9-encoding polynucleic acid and/or the localization of a MAGE-A9 polypeptide.

The terms "polynucleic acid" and "polynucleotide" as used herein are intended to include, without being limited to, prokaryotic and eukaryotic polynucleic acid, DNA, genomic DNA, synthetic DNA, cDNA, mRNA, mRNA splice variants, synthetic mRNA, ribozyme, antisense molecules, recombinant DNA or RNA containing a MAGE-A9-encoding polynucleotide or a fragment of a MAGE-A9-encoding polynucleotide , and polynucleotide including peptide nucleic acids (PNAs) or non-nucleic acid molecules such as phosphorothioate derivatives that specifically bind DNA or RNA in a base pair-dependent manner. Sense and antisense polynucleotide are intended to be included in those terms. The terms also capture nucleic acid sequences that include any of the known base analogs of DNA and RNA. The terms are further intended to mean a polymeric form of nucleotides of at least 10 bases or base pairs in length, either ribonucleotides or deoxynucleotides, or a modified form of either type of nucleotides, and is meant to include single and double stranded forms of DNA and RNA. In the art, this term if often used interchangeably with oligonucleotide. Variants of polynucleotides are intended to represent polynucleotides in which one or more nucleotide is deleted, substituted or added. Variants may be naturally or non-naturally occurring allelic variants. Variant sequences preferably exhibit at least 50%, more preferably at least 75%, more preferably yet at least 90%, even more preferably at least 95%, and most preferably at least 98% identity to the naturally occurring sequence of interest. Fragments of polynucleotides are intended to represent polynucleotides in which at least two sequential nucleotide have been deleted. For the purpose of the present invention, polynucleotide fragments are intended to include at least the minimal portion of a polynucleotide capable of producing the expected effect. For example, for genetic immunization, only a fragment of a polynucleic acid encoding a highly immunogenic portion of the polypeptide can be used instead of the entire polynucleic acid. In both those examples, the skilled man in the art those techniques are pertaining to will have the necessary knowledge and tools to rapidly and easily identify the useful fragments that are adequate for the projected use.

The term "polypeptide" as used herein is intended to mean a polymer of at least 4 amino acids. In the present application, this term is used as an equivalent to terms such as "peptide" and "protein" and should be understood as such. Variants of polypeptides are intended to represent polypeptides in which one or more amino acid is deleted, substituted or added. Variants may be occurring naturally or non-naturally. Variants can include a MAGE-A9 polypeptide originating from a different animal species than the one it is intended to be used into, the protein having slightly different amino-acid or are encoded by various nucleotide sequences. Variant sequences preferably exhibit at least 50%, more preferably at least 75%, more preferably yet at least 90%, even more preferably at least 95%, even more preferably at least 98%, and most preferably at least 99% identity to the naturally occurring polypeptide of interest. Fragments of polypeptides are intended to represent polypeptides in which at least two sequential amino acids have been deleted. For the purpose of the present invention, polypeptide fragments are intended to include at least the minimal portion of a polypeptide capable of producing the expected effect. For example, when aiming at inducing an immune response towards MAGE-A9, only a fragment of a MAGE-A9 polypeptide, such as a MAGE-A9-specific epitope, can be used, providing this fragment is sufficiently immunogenic to induce an immune response and that the immune response is specific towards MAGE-A9. In such an example, the skilled man in the art this technique is pertaining to will have the necessary knowledge and tools to rapidly and easily identify the useful fragments that are adequate for the projected use. Fusion proteins containing a MAGE-A9 polypeptide fragment can also be used with the present invention.

MAGE-A9 polypeptides contains epitope that can be recognized by an antibody are used in methods of monitoring MAGE-A9. MAGE-A9 polypeptide fragments and polypeptide analogs or variants can also be used in an analogous manner, as long as they contain an epitope, that is a portion of the original polypeptide that can be recognized by the immune system, such as by an anti-MAGE-A9 antibody. It is known in the art that an epitope can be cleaved in different fragments that can be used as antigens or immunogens to raise an immune response against the whole protein or peptide they are originating from. Also, analogs can be used for building an immune defense system in an organism, such as a human, against the MAGE-A9 polypeptide. In this sense, analogs are intended here to mean polypeptides or proteins having a certain level of homology with the original MAGE-A9 polypeptide, as for example from 60 to 99% homology, 70 to 99% homology, 80 to 99% homology, 90 to 99% homology, 95 to 99% homology and 98 to 99% homology to the native MAGE-A9 polypeptide. An analog can be a synthetic MAGE-A9 polypeptide with a biochemical modification improving its immunogenic characteristics. Typically, a skilled artisan will create a variety of different polypeptide fragments that can be used in order to generate an immune response specific for different portions of the polypeptide of interest. For example it may be preferable to use a polypeptide comprising one of the MAGE-A9 biological motifs. Polypeptide fragments, variants or analogs are typically useful in this context as long as they comprise a specific portion of a MAGE-A9 polypeptide capable of generating the production of an antibody specific for targeting the MAGE-A9 polypeptide sequence of interest.

Another aspect of the invention is to allow for the evaluation of the integrity of a MAGE-A9 nucleotide or amino acid sequence in a biological sample, in order to identify perturbations such as insertions, deletion, substitution or mutations in the structure of these molecules. The presence of one or more perturbations in MAGE-A9 gene products in the sample can be an indication of cancer susceptibility (or the emergence or existence of a tumor).

The present invention enables for the comparison of a parameter in a biological sample obtained from a subject with a control parameter in order to detect or diagnose an aberrant cell growth. This can be done by contacting the biological sample obtained from the subject with a MAGE-A9 gene product-binding agent. For the purpose of the present invention, a "MAGE-A9 gene product" as used herein is intended to represent any molecule that can be produced using a MAGE-A9 gene as a template for its production. Polynucleotides based on the sequence of the MAGE-A9 gene, in whole or in parts, or complementary polynucleotides capable of hybridizing under high stringency conditions to at least a part of a MAGE-A9 gene are included in this expression. Similarly, polypeptides that encoded by those polynucleotides are also included in the expression. A "MAGE-A9 gene product-binding agent" as used herein is intended to represent any agent that can specifically bind to a MAGE-A9 gene product. For polynucleotides, this includes, without restriction, oligonucleotide primers, specific probes and radioprobes, antisense sequences, complementary sequences, and any other molecules that will recognize the sequence of a MAGE-A9-associated polynucleotide and will specifically bind to it under appropriate conditions. For polypeptides, this includes, without restriction, monoclonal and polyclonal antibodies, and also any other molecule that will recognize the sequence of a MAGE-A9-associated polypeptide and will specifically bind to it under appropriate conditions.

The binding of the MAGE-A9 gene product-binding agent can be detected by a variety of technique known in the art. For polynucleic acids, some of the detections techniques that can be used include, without limitations, genomic analysis, Northern blot analysis, Southern blot analysis, *in situ* hybridization technique, PCR assay, RT-PCR assay, branched DNA analysis, and tissue array analysis. MAGE-A9 gene product-binding agents that can be used for the detection of polynucleic acids can include, without limitation, primers, probes, riboprobes, and complementary nucleic acids. consisting of fragments of the MAGE-A9 cDNA sequence. Illustrating this, primers used to PCR amplify a MAGE-A9 polynucleotide must include less than the whole MAGE-A9 sequence to correctly function in the polymerase chain reaction. In the context of such PCR reactions, skilled artisans generally create a variety of different polynucleotide fragments that can be used as primers in order to amplify different portions of a polynucleotide of interest or to optimize amplification reactions. An additional illustration of the use of such fragments is provided in Example 1, where MAGE-A9 polynucleotide fragments are used as probes, or primers, to detect the expression of MAGE-A9 RNAs in cancer cells. In addition, variant polynucleotide sequences are typically used as primers and probes for the corresponding mRNAs in PCR and Northern analyses. Polynucleotide fragments and variants are useful in this context where they are capable of binding to a target polynucleotide sequence under conditions of high stringency.

The binding of a polynucleotide fragment or variant to a target polynucleotide sequence of interest, or "hybridization", is meant to refer to conventional hybridization conditions. The "stringency" of an hybridization reaction is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured nucleic acid sequences to re-anneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, or highly stringent, while lower temperatures less so.

As discussed above, a MAGE-A9 gene product can also be a MAGE-A9 polypeptide. Polypeptide detection techniques well known in the art can be used in determining MAGE-A9 polypeptide, including, but not restricting to, immunohistochemistry, radio-immunoassay, enzyme-linked immunosorbent assays (ELISA), enzyme-linked immunofluorescent assays (ELIFA), radioscintigraphic imaging methods, and Western blot analysis. Immunological non-antibody assays of the invention also comprise T cell immunogenicity assays (inhibitory or stimulatory) as well as major histocompatibility complex (MHC) binding assays. MAGE-A9 gene product-binding agents that can be used for the detection of polynucleic acids can include, without limitation, antibodies such as monoclonal antibodies and polyclonal antibodies. Monoclonal antibodies such as the ones produced by the cell lines identified at the International Depositary Authority of Canada under accession number 020805-01 and 020805-02 are particularly well suited for detecting MAGE-A9 polypeptides.

A comparison between the determined parameter from the biological sample and a control parameter is then performed. The control parameter, as used herein, is intended to represent a definite set of data, based on previous observations, measurements or prediction, that are used as a template for evaluating the aberrant cell growth state of a subject. Alternatively, the control parameter can be measured or determined after the determination of the parameter in the biological sample. Typically, a control parameter will scale from values representative of the absence of aberrant cell growth to values representative of a highly aberrant cell growth. The difference between the parameter as determined in the biological sample and the control parameter will be indicative of aberrant cell growth in the subject. The possible determination regarding aberrant cell growth following such a detection can range from the absence of aberrant cell growth to a highly aberrant cell growth, including the determination of an improving or deteriorating state of aberrant cell growth over time in the subject. The improvement or deterioration of aberrant cell growth over time can be used either to monitor the evolution of an aberrant cell growth-related disorder, or to monitor the effectiveness of a treatment for an aberrant cell growth-related disorder. In an embodiment, in the method herewith disclosed, the parameter is compared to a corresponding biological sample or a control parameter. The expression "corresponding biological sample" as used herein is intended to represent a biological sample that is corresponding to another biological sample, either by being the same kind of sample (e.g. blood, bladder tissue) taken at a different time in a same subject, or by being a similar kind of sample taken from a different subject which present an interest of being compared to (e.g. comparing a sample of a subject suspected of having an aberrant cell growth disorder to a sample of a subject known not to have the suspected cell growth disorder).

The expression profile of MAGE-A9 makes it a diagnostic marker for local and/or metastasized disease, and provides useful information on aberrant cell growth or oncogenic potential of a biological sample. In particular, the status of MAGE-A9 provides information useful for predicting susceptibility to particular disease stages, progression, evolution, tumor aggressiveness and/or response to treatment. The present invention provides, amongst other things, methods for determining MAGE-A9 status and diagnosing cancers that express MAGE-A9, such as cancers of the bladder for example. Because MAGE-A9 mRNA is over-expressed in bladder cancers when compared to its expression level in normal bladder tissue, the determination of MAGE-A9 mRNA transcripts or polypeptides level in a biological sample can be used to diagnose a bladder disease associated with MAGE-A9-associated aberrant cell growth disorder, and can provide prognostic information useful in defining appropriate therapeutic options.

Another aspect of the present invention is to provide a method for predicting the susceptibility of a subject to a MAGE-A9-associated cancer. Such a method involves the determination of a parameter in a biological sample obtained from the subject and a comparison between the determined parameter from the biological sample and the control parameter is then performed, as described above. The difference between the determined parameter from the biological sample with the control parameter is indicative of the susceptibility of the subject to MAGE-A9-associated cancer. The "susceptibility" of a subject to a MAGE-A9-associated cancer as used herein is intended to represent a predictive measure of the risk for a subject to develop a MAGE-A9-associated cancer. The predictive measure can be calculated by any statistical or mathematical mean well known in the art, as long as they include a value representative of the level of aberrant cell growth in the subject, said value being linked to the risk of developing a MAGE-A9-associated cancer by the subject. The susceptibility can also be evaluated for MAGE-A9-associated hyperplasia.

Another aspect of the present invention is to provide a method for determining the aggressiveness of a MAGE-A9-associated cancer in a subject. Such a method involves the determination of a parameter in a biological sample obtained from the subject and a comparison between the determined parameter from the biological sample and a control parameter is then performed, as described above. The difference between the determined parameter from the biological sample with the control parameter is indicative of the aggressiveness of the MAGE-A9-associated cancer in the subject. The term "aggressiveness" as used herein in relation to the "aggressiveness" of a MAGE-A9-associated cancer is intended to reflect on the aggressiveness of a cancer or a tumor as traditionally intended in oncology. Typically, highly aggressive cancers will have high chances of producing metastasic cancer and will have a low potential of being responsive to traditional treatment, while a non-aggressive cancer will often present higher responsiveness to traditional treatments.

Another aspect of the present invention is to provide a method for determining a variation in aberrant cell growth in a subject over a time period. Such a method involves the determination of a parameter in a biological sample obtained from the subject at a given point in time and its comparison with a control parameter, as described above. In this case, the control parameter will be a parameter from a previously collected sample from the same patient, in order to compare the state of aberrant cell growth at the time of determination with the state of aberrant cell growth at a previous point in time. The difference between the determined parameter from the biological sample with the control parameter is indicative of the variation of aberrant cell growth over time. A "variation" in aberrant cell growth over time is intended to reflect changes in the characteristics of cell growth of cells escaping the normal growth regulatory mechanisms of cell growth in a given length of time. Positive variations between a previously estimated growth state and a freshly estimated growth state will typically be reflective of an increase in aberrant cell growth, while a negative variation will typically be reflective of a decrease in aberrant cell growth to values closer to a normal state of growth. The determination of a variation in aberrant cell growth can be used for determining the evolution of an aberrant cell growth-related disorder over time, e.g. an increase or a decrease of the disorder. Another possible use of this method is the assessment of the efficiency of a treatment for aberrant cell growth-related disorder. In this respect, aberrant cell growth can be determined before a therapeutic treatment is started, and another determination of aberrant cell growth can be made after a given length of treatment time in order to evaluate the efficiency of the treatment prescribed. Any other justifications for determining aberrant cell growth variations in a subject are meant to be included by this aspect of the present invention, this aspect being directed towards a method for determining such a variation, regardless of the interest in determining such a variation.

The above determination and diagnostic methods can be combined with any one of a wide variety of prognostic and diagnostic protocols known in the art. For example, and without being limitative, another aspect of the invention is directed toward methods for observing a coincidence between the over-expression of a MAGE-A9 gene or a MAGE-A9 gene product (or perturbations in the expression of a MAGE-A9 gene or a MAGE-A9 gene product) with another factor that is associated with malignancy, as a means for diagnosing and prognosticating whether a tissue sample has cancer cells or cells prompted to develop into cancer cells, or how cancer cells will respond to therapeutic treatment. A wide variety of factors associated with malignancy can be utilized, such as the expression of other genes associated with malignancy, as well as gross cytological observations.

Another aspect of the present invention provides a method for inducing an immune response to a MAGE-A9 polypeptide in a subject, and to inducing an immunization to a MAGE-A9 polypeptide in a subject. The term "immunization" as used herein is intended to relate to its traditional definition in the art of immunology, that is the training of an organism to respond either more rapidly or more efficiently to a particular threat. This include genetic immunization as well as cellular and humoral immunization. The term "genetic immunization" as used herein is intended to represent the introduction of a MAGE-A9-encoding polynucleic acid, which comprises a coding sequence encoding the MAGE-A9 protein, or part of the MAGE-A9 protein, in an organism in order to endogenously induce an immune response in the organism towards the MAGE-A9 encoding nucleic acid or part thereof or the MAGE-A9 protein or part thereof. In addition, naked DNA immunization techniques known in the art can be used, with or without purified MAGE-A9 polypeptide or MAGE-A9 expressing cells, to generate an immune response to the encoded immunogen. The term "cellular immunization" as used herein is intended to represent the activation of macrophages and natural killer cells, the production of antigen-specific T lymphocytes and the release of various cytokines in response to the exposure of an organism to an antigen, such as a MAGE-A9 polypeptide or a variant or a fragment of a MAGE-A9 polypeptide that will be either sufficiently immunogenic to induce an immune response, or that will either be injected together with an immunogenic molecule or an agent that will increase the immune response towards the MAGE-A9 polypeptide. The term "humoral immunization" as used herein is intended to represent the production of antibodies by B lymphocytes in response to the exposure of an organism to an antigen, such as a MAGE-A9 polypeptide or a variant or a fragment of a MAGE-A9 polypeptide that will be either sufficiently immunogenic to induce an immune response, or that will either be injected together with an immunogenic molecule or an agent that will increase the immune response towards the MAGE-A9 polypeptide. It also involves the generation of memory cells and the production of various cytokines.

Methods based on administration of peptides or proteins as antigens for inducing such immune response are well known in the art. For example, the protein MAGE-A9 alone, linked to a cytokine, combined to an adjuvant or expressed in an antigen presenting cells, or any combination of these, can be administered in its whole, or fragmented. Combination of fragments can also be used for this purpose to induce a more polyvalent anti-MAGE-A9 immune response.

MAGE-A9 antibodies are also used in methods for purifying a MAGE-A9-associated protein and for isolating MAGE-A9 homologues and related molecules. For example, a method of purifying a MAGE-A9 polypeptide comprises incubating a MAGE-A9 antibody, which may have been coupled to a solid matrix, with a cellular lysate or other solution containing a MAGE-A9 polypeptide under conditions that permit the MAGE-A9 antibody to bind to the MAGE-A9 polypeptide; washing the solid matrix to eliminate impurities; and eluting the MAGE-A9 polypeptide from the coupled antibody. Other uses of MAGE-A9 antibodies in accordance with the invention include generating anti-idiotypic antibodies that mimic a MAGE-A9 polypeptide.

Various methods for the preparation of monoclonal or polyclonal antibodies known in the art can be used. For example, antibodies can be prepared by immunizing a suitable mammalian host using a MAGE-A9 polypeptide, or a fragment or a variant thereof, in isolated or immunoconjugated form. In addition, fusion proteins of MAGE-A9 can also be used, such as a MAGE-A9 GST-fusion protein. In a particular aspect, a GST fusion protein comprising all or most of the amino acid sequence of MAGE-A9 is produced, then used as an immunogen to generate appropriate antibodies.

MAGE-A9 antibodies can be introduced into a patient such that the antibody binds to MAGE-A9 and modulates a function, such as an interaction with a binding partner, and consequently mediates destruction of the tumor cells and/or inhibits the growth of the tumor cells. Mechanisms by which such antibodies exert a therapeutic effect can include complement-mediated cytolysis, antibody-dependent cellular cytotoxicity, modulation of the physiological function of MAGE-A9, inhibition of ligand binding or signal transduction pathways, modulation of tumor cell differentiation, alteration of tumor angiogenesis factor profiles, and/or apoptosis.

Those skilled in the art understand that antibodies can be used to specifically target and bind immunogenic molecules such as an immunogenic region of a MAGE-A9 sequence (SEQ ID NO:2). In addition, skilled artisans understand that it is routine to conjugate antibodies to cytotoxic agents. When cytotoxic and/or therapeutic agents are delivered directly to cells, such as by conjugating them to antibodies specific for a molecule expressed by that cell (e.g. MAGE-A9), the cytotoxic agent will exert its known biological effect (i.e. cytotoxicity) on those cells or their vicinity.

Another aspect of the present invention provides the use of a MAGE-A9 gene product for the induction of an immune response to a MAGE-A9-associated aberrant cell growth in a subject. A further aspect of the present invention provides the use of a MAGE-A9 gene product in the preparation of a medicament for the induction of an immune response to a MAGE-A9-associated aberrant cell growth in a subject.

Another aspect of the present invention provides a method for preventing a MAGE-A9-associated aberrant cell growth in a subject by administering a MAGE-A9 gene product to the subject in order to induce an immunization to a MAGE-A9 polypeptide in said subject. The prevention of a MAGE-A9-associated aberrant cell growth is related to the art of prophylaxis, that is decreasing the risks of apparition or recurrence of a particular disease or disorder, such as the ones associated with a MAGE-A9 over-expression. By being immunized to a MAGE-A9 polypeptide, the subject will naturally produce an immune response directed toward cells over-expressing the MAGE-A9 polypeptide against which the subject is immunized. Therefore, the immune system of the subject will prevent or lower MAGE-A9-associated aberrant cell growth, including MAGE-A9-associated hyperplasia and MAGE-A9-associated cancer.

Another aspect of the present invention provides the use of a MAGE-A9 gene product for the prevention of a MAGE-A9-associated aberrant cell growth in a subject. A further aspect of the present invention provides the use of a MAGE-A9 gene product in the preparation of a medicament for the prevention of a MAGE-A9-associated aberrant cell growth in a subject.

Another aspect of the present invention is the treatment of a MAGE-A9-associated aberrant cell growth in a subject by administering a MAGE-A9 gene product to the subject, which will induce an immune response to a MAGE-A9 polypeptide in the subject. As described above, the administration of a MAGE-A9 gene product will induce an immune response to a MAGE-A9 polypeptide, therefore boosting the immune system against such a MAGE-A9 polypeptide. In consequence, cells over-expressing such a MAGE-A9 polypeptide, such as cells from MAGE-A9-associated cancer, will be targeted by the immune system of the host.

Another aspect of the present invention also provides for the use of a MAGE-A9 gene product for the treatment of a MAGE-A9-associated aberrant cell growth in a subject. A further aspect of the present invention provides for the use of a MAGE-A9 gene product in the preparation of a medicament for treating a MAGE-A9-associated aberrant cell growth in a subject.

Based on its cellular localization and its protein structure, it is possible that MAGE-A9 is involved in modulating the activity of tumor-promoting genes or genes that have some role in tumorigenesis. Accordingly, another aspect of the invention is to provide therapeutic approaches aimed at modulating or reducing the expression of the MAGE-A9 polypeptide are expected to be useful for a subject suffering from bladder cancer, and other cancers which exhibit increased levels of MAGE-A9 expression.

The immunization can also be induced through recombinant technologies, such as *in vivo, ex vivo,* or *in situ* gene therapy. A large variety of vectors for use in the therapeutic methods described herein can be generated by any of the methods known to the art for the insertion of DNA fragments into a gene expression vector consisting of appropriate transcriptional/translational control signals and the desired MAGE-A9 cDNA sequence.

Expression of a nucleic acid sequence encoding a MAGE-A9 may be regulated by a second nucleic acid sequence so that the MAGE-A9 is expressed in a host infected or transfected with the recombinant DNA molecule. For example, expression of MAGE-A9 may be controlled by any promoter/enhancer element known in the art and appropriate for such a use. The promoter activation may be tissue specific or inducible by a metabolic product or administered substance. Promoters/enhancers which may be used to control MAGE-A9 gene expression include, but are not limited to, the native MAGE-A9 promoter, the cytomegalovirus (CMV) promoter/enhancer, the human β-actin promoter, the glucocorticoid-inducible promoter present in the mouse mammary tumor virus long terminal repeat (HHTV LTR), the long terminal repeat sequences of Moloney murine leukemia virus (MULV LTR), the SV40 early region promoter, the promoter contained in the 3' long terminal repeat of Rous sarcoma virus (RSV), the herpes simplex virus (HSV) thymidine kinase promoter/enhancer, the regulatory sequences of the metallothionine gene, the adenovirus promoter, and the herpes simplex virus LAT promoter.

The invention further provides a host-vector system comprising a recombinant DNA molecule containing a MAGE-A9 polynucleotide, fragment, analog or variant thereof within a suitable prokaryotic or eukaryotic host cell. Examples of suitable eukaryotic host cells include a yeast cell, a plant cell, or an animal cell, such as a mammalian cell or an insect cell (e.g., a baculovirus-infectible cell such as an Sf9 or HighFive cell). Examples of suitable mammalian cells include various cancer cell lines such as DU145 and TsuPr1, other transfectable or transducible cancer cell lines, primary cells (PrEC), as well as a number of mammalian cells routinely used for the expression of recombinant proteins (e.g., COS, CHO, 293, and/or 293T cells). More particularly, a polynucleotide comprising the coding sequence of MAGE-A9 or a fragment, analog or homolog thereof can be used to generate MAGE-A9 polypeptides or fragments thereof using any number of host-vector systems routinely used and widely known in the art.

A wide range of host-vector systems suitable for the expression of MAGE-A9 polypeptides or fragments thereof are available in the art. Preferred vectors for mammalian expression include but are not limited to pcDNA 3.1 myc-His-tag (Invitrogen) and the retroviral vector pSR.quadrature.tkneo. Using these expression vectors, MAGE-A9 can be expressed in several cancer and non-bladder cell lines, including for example 293, 293T, rat-1, NIH 3T3 and/or TsuPr1. The host-vector systems of the invention are useful for the production of a MAGE-A9 polypeptide or fragment thereof. Such host-vector systems can be employed to study the functional properties of MAGE-A9 and MAGE-A9 mutations or analogs.

Vaccinia virus can be used, for example, as a vector to express nucleotide sequences that encode the peptides of the invention. Upon introduction into a host, the recombinant vaccinia virus expresses the protein immunogenic peptide, and thereby elicits a host immune response. A wide variety of other vectors useful for therapeutic administration or immunization of the peptides of the invention, e.g. adeno and adeno-associated virus vectors, retroviral vectors, BCG vectors, *Salmonella typhi* vectors, detoxified anthrax toxin vectors, and the like, will be apparent to those skilled in the art from the description herein.

Thus, gene delivery systems can be used to deliver a MAGE-A9-encoding polynucleotide. In one aspect, the full-length human MAGE-A9 cDNA is employed. In another aspect, MAGE-A9-encoding polynucleotide encoding specific cytotoxic T lymphocytes (CTL) antigens and/or antibody epitopes are employed.

Another aspect of the present invention is a method for the treatment of a MAGE-A9-associated aberrant cell growth in a subject by administering a MAGE-A9 gene product-binding agent and a therapeutic agent to said subject. Such therapeutic agent can be a chemotherapeutic agent or any other agent that will be effective toward a MAGE-A9-associated cancer cell. The term "therapeutic agent" as used herein is intended to represent any agent that can be therapeutically effective towards an aberrant cell growth disorder, such as a cytotoxic agent, and that can be used with either aspects of the present invention. The therapeutic agent can be either administered jointly with the MAGE-A9 gene product-binding agent, or be physically or chemically linked to the MAGE-A9 gene product-binding agent. In another embodiment, the therapeutic agent can be administered separately from the MAGE-A9 gene product-binding agent. Preferably, and regarding this aspect of the invention, the MAGE-A9 gene product-binding agent will be a monoclonal or a polyclonal antibody. More preferably, the MAGE-A9 gene product-binding agent will be a monoclonal antibody produced by one of the cell lines identified at the International Depositary Authority of Canada under accession number 020805-01 and 020805-02. By binding to MAGE-A9 in MAGE-A9 over-expressing cells, the MAGE-A9 gene product-binding agent, and more particularly the monoclonal antibodies described above, will facilitate the delivery of an effective therapeutic agent directly to a MAGE-A9 over-expressing cell. Those monoclonal antibodies can also be used as drug targeting system or as drug delivery systems since they allow for the specific delivery of an effective therapeutic agent directly and specifically to a MAGE-A9 over-expressing cell.

Another aspect of the present invention is to provide a composition for treating a MAGE-A9-associated aberrant cell growth in a subject, wherein the composition comprises a MAGE-A9 gene product-binding agent, such as described above, with at least one effective therapeutic agent and a physiologically acceptable carrier.

In subjects with MAGE-A9-associated or related cancer, the vaccine compositions of the invention can also be used in conjunction with other treatments used for cancer, e.g., surgery, chemotherapy, drug therapies, radiation therapies, etc. including use the combination of the vaccine composition with immune adjuvants such as IL-2, IL-12, GM-CSF, and the like.

The expression pattern of MAGE-A9 in a given tissue, qualitatively, quantitatively, or regionally, can be used for predicting the potential immune response to MAGE-A9-expressing cells that can be obtained in an individual. This approach, also called theranostics (therapy-specific diagnostics), is the integration of rapid diagnostic testing and therapy involving a feedback loop to monitor and improve the efficiency of a treatment and to minimize its side-effects. In traditional medical practice, therapeutic choices follow diagnosis, which may be based on clinical signs alone, or may be made in conjunction with an *in vivo* or *in vitro* diagnostic test. However, the effectiveness of the therapy and the likelihood of side effects often cannot be predicted for individual subjects. Now, the present invention provides theranostic tests allowing the mixed use of medical diagnosis and treatment into a predictive application.

The theranostic aspect of the present invention in specifically developed for predicting and assessing anti-MAGE-A9 immune response in individual subjects rather than diagnosing disease. Theranostic testing can be used to specifically design treatments with an optimal efficiency and minimal side-effects for specific subjects, and inversely to select subjects that are particularly likely to benefit from specific treatments, and unlikely to suffer from drug-induced side-effects.

Another aspect of the present invention is to provide a kit comprising an antibody produced by a cell line identified in the International Depositary Authority of

Canada under accession number 020805-01 or 020805-02 and instructions for its use in the detection of a MAGE-A9 polypeptide in a biological sample from a subject.

In another aspect of the invention, there is provided cancer vaccines comprising a MAGE-A9-related protein or fragments and variant thereof, or a MAGE-A9-related nucleic acid or fragments and variant thereof. In view of the expression of MAGE-A9, cancer vaccines prevent and/or treat MAGE-A9-expressing cancers with minimal or no effects on non-target tissues. The use of a tumor antigen in a vaccine that generates humoral and/or cell-mediated immune responses as anticancer therapy is well known in the art.

Such methods can be readily practiced by employing a MAGE-A9-related protein, or a MAGE-A9-encoding nucleic acid molecule and recombinant vectors capable of expressing and presenting the MAGE-A9 immunogen (which typically comprises an antibody or T cell epitopes). Skilled artisans understand that a wide variety of vaccine systems for delivery of immunoreactive epitopes are known in the art. Briefly, such methods of generating an immune response (e.g. humoral and/or cell-mediated) in a mammal, comprise the steps of: exposing the mammal's immune system to an immunoreactive epitope (e.g. an epitope present in a MAGE-A9 protein (SEQ ID NO:2) or analog or homolog thereof) so that the mammal generates an immune response that is specific for that epitope (e.g. generates antibodies that specifically recognize that epitope).

The entire MAGE-A9 protein, immunogenic regions or epitopes thereof can be combined and delivered by various means. Such vaccine compositions can include, for example, lipopeptides, peptide compositions encapsulated in poly(DL-lactide-co-glycolide) ("PLG") microspheres, peptide compositions contained in immune stimulating complexes (ISCOMS), multiple antigen peptide systems (MAPs), peptides formulated as multivalent peptides; peptides for use in ballistic delivery systems, typically crystallized peptides, viral delivery vectors, particles of viral or synthetic origin, adjuvants, liposomes, or, naked or particle absorbed cDNA. Toxin-targeted delivery technologies, also known as receptor mediated targeting, such as those of Avant Immunotherapeutics, Inc. (Needham, Mass.) may also be used.

In patients with MAGE-A9-associated cancer, the vaccine compositions of the invention can also be used in conjunction with other treatments used for cancer, e.g., surgery, chemotherapy, drug therapies, radiation therapies, etc. including use in combination with immune adjuvants such as IL-2, IL-12, GM-CSF, and the like.

Another aspect of the invention is to provide cellular vaccines. CTL epitopes can be determined using specific algorithms to identify peptides within MAGE-A9 protein that bind corresponding HLA alleles. In a preferred embodiment, a MAGE-A9 immunogen contains one or more amino acid sequences identified using techniques well known in the art, or a peptide of 8, 9, 10 or 11 amino acids specified by an HLA Class I motif/supermotif (e.g., Tables 5, 6 and 7) and/or a peptide of at least 9 amino acids that comprises an HLA Class II motif/supermotif (e.g., Table 8). As is appreciated in the art, the HLA Class I binding groove is essentially closed ended so that peptides of only a particular size range can fit into the groove and be bound, generally HLA Class I epitopes are 8, 9, 10, or 11 amino acids long. In contrast, the HLA Class II binding groove is essentially open ended; therefore a peptide of about 9 or more amino acids can be bound by an HLA Class II molecule. HLA Class II epitopes are often 9, 10, 11, 12, 13, 14, 15, 16, 17, 18, 19, 20, 21, 22, 23, 24, or 25 amino acids long, or longer than 25 amino acids.

Another aspect of the invention is to provide *ex vivo* vaccines. Various *ex vivo* strategies can also be employed to generate an immune response. One approach involves the use of antigen presenting cells (APCs) such as dendritic cells (DC) to present MAGE-A9 antigen to a patient's immune system. Dendritic cells express MHC class I and II molecules, B7 co-stimulator, and IL-12, and are thus highly specialized antigen presenting cells. In bladder cancer, autologous dendritic cells pulsed with peptides of MAGE-A3 have been used in a Phase I clinical trial to stimulate bladder cancer patients' immune systems. Thus, dendritic cells can be used to present MAGE-A9 peptides to T cells in the context of MHC class I or II molecules. In one embodiment, autologous dendritic cells are pulsed with MAGE-A9 peptides capable of binding to MHC class I and/or class II molecules. In another embodiment, dendritic cells are pulsed with the complete MAGE-A9 protein. Yet another embodiment involves engineering the overexpression of a MAGE-A9 gene in dendritic cells using various implementing vectors known in the art, such as, for example, adenovirus, retrovirus, lentivirus, adeno-associated virus, DNA transfection, or tumor-derived RNA transfection. Cells that express MAGE-A9 can also be engineered to express immune modulators, such as GM-CSF, and used as immunizing agents.

Vaccines and methods of preparing vaccines that contain an immunogenically effective amount of one or more HLA-binding peptides as described herein are further embodiments of the invention. Furthermore, vaccines in accordance with the invention encompass compositions of one or more of the claimed peptides. A peptide can be present in a vaccine individually. Alternatively, the peptide can exist as a homopolymer comprising multiple copies of the same peptide, or as a heteropolymer of various peptides. Polymers have the advantage of increased immunological reaction and, where different peptide epitopes are used to make up the polymer, the additional ability to induce antibodies and/or CTLs that react with different antigenic determinants of the pathogenic organism or tumor-related peptide targeted for an immune response. The composition can be a naturally occurring region of an antigen or can be prepared, e.g., recombinantly or by chemical synthesis.

Carriers that can be used with vaccines of the invention are well known in the art, and include, e.g., thyroglobulin, albumins such as human serum albumin, tetanus toxoid, polyamino acids such as poly L-lysine, poly L-glutamic acid, influenza, hepatitis B virus core protein, and the like. The vaccines can contain a physiologically tolerable (i.e., acceptable) diluent such as water, or saline, preferably phosphate buffered saline. The vaccines also typically include an adjuvant. Adjuvants such as incomplete Freund's adjuvant, aluminum phosphate, aluminum hydroxide, or alum are examples of materials well known in the art. Additionally, as disclosed herein, CTL responses can be primed by conjugating peptides of the invention to lipids, such as tripalmitoyl-S-glycerylcysteinlyserylserine ($P_3CSS$). Moreover, an adjuvant such as a synthetic cytosine-phosphorothiolated-guanine-containing (CpG) oligonucleotides has been found to increase CTL responses 10- to 100-fold.

Upon immunization with a peptide composition in accordance with the invention, via injection, aerosol, oral, transdermal, transmucosal, intrapleural, intrathecal, or other suitable routes, the immune system of the host responds to the vaccine by producing large amounts of CTLs and/or helper T lymphocytes (HTLs) specific for the desired antigen. Consequently, the host becomes at least partially immune to later development of cells that express or overexpress MAGE-A9 antigen, or derives at least some therapeutic benefit when the antigen is tumor-associated.

In some embodiments, it may be desirable to combine the class I peptide components with components that induce or facilitate neutralizing antibody and or helper T cell responses directed to the target antigen. A preferred embodiment of such a composition comprises class I and class II epitopes in accordance with the invention. An alternative embodiment of such a composition comprises a class I and/or class II epitope in accordance with the invention, along with a cross reactive HTL epitope such as PADRE™. (Epimmune, San Diego, Calif.) molecule.

A vaccine of the invention can also include antigen-presenting cells (APC), such as dendritic cells (DC), as a vehicle to present peptides of the invention. Vaccine compositions can be created *in vitro,* following dendritic cell mobilization and harvesting, whereby loading of dendritic cells occurs *in vitro.* For example, dendritic cells are transfected, e g, with a minigene in accordance with the invention, or are pulsed with peptides. The dendritic cells can then be administered to a patient to elicit immune responses *in vivo.* Vaccine compositions, either DNA- or peptide-based, can also be administered *in vivo* in combination with dendritic cell mobilization whereby loading of dendritic cells occurs *in vivo.*

Preferably, the following principles are utilized when selecting an array of epitopes for inclusion in a polyepitopic composition for use in a vaccine, or for selecting discrete epitopes to be included in a vaccine and/or to be encoded by nucleic acids such as a minigene. It is preferred that each of the following principles be balanced in order to make the selection. The multiple epitopes to be incorporated in a given vaccine composition may be, but need not be, contiguous in sequence in the native antigen from which the epitopes are derived.

1.) Epitopes are selected which, upon administration, mimic immune responses that have been observed to be correlated with tumor clearance. For HLA Class I this includes 3-4 epitopes that come from at least one tumor-associated antigen (TAA). For HLA Class II a similar rationale is employed; again 3-4 epitopes are selected from at least one TAA. Epitopes from one TAA may be used in combination with epitopes from one or more additional TAAs to produce a vaccine that targets tumors with varying expression patterns of frequently-expressed TAAs.

2.) Epitopes are selected that have the requisite binding affinity established to be correlated with immunogenicity: for HLA Class I an $IC_{50}$ of 500 nM or less, often 200 nM or less; and for Class II an $IC_{50}$ of 1000 nM or less.

3.) Sufficient supermotif bearing-peptides, or a sufficient array of allele-specific motif-bearing peptides, are selected to give broad population coverage. For example, it is preferable to have at least 80% population coverage. A Monte Carlo analysis, a statistical evaluation known in the art, can be employed to assess the breadth, or redundancy of, population coverage.

4.) When selecting epitopes from cancer-related antigens it is often useful to select analogs because the patient may have developed tolerance to the native epitope.

5.) Of particular relevance are epitopes referred to as "nested epitopes." Nested epitopes occur where at least two epitopes overlap in a given peptide sequence. A nested peptide sequence can comprise B cell, HLA class I and/or HLA class II epitopes. When providing nested epitopes, a general objective is to provide the greatest number of epitopes per sequence. Thus, an aspect is to avoid providing a peptide that is any longer than the amino terminus of the amino terminal epitope and the carboxyl terminus of the carboxyl terminal epitope in the peptide. When providing a multi-epitopic sequence, such as a sequence comprising nested epitopes, it is generally important to screen the sequence in order to insure that it does not have pathological or other deleterious biological properties.

6.) If a polyepitopic protein is created, or when creating a minigene, an objective is to generate the smallest peptide that encompasses the epitopes of interest. This principle is similar, if not the same as that employed when selecting a peptide comprising nested epitopes. However, with an artificial polyepitopic peptide, the size minimization objective is balanced against the need to integrate any spacer sequences between epitopes in the polyepitopic protein. Spacer amino acid residues can, for example, be introduced to avoid junctional epitopes (an epitope recognized by the immune system, not present in the target antigen, and only created by the man-made juxtaposition of epitopes), or to facilitate cleavage between epitopes and thereby enhance epitope presentation. Junctional epitopes are generally to be avoided because the recipient may generate an immune response to that non-native epitope. Of particular concern is a junctional epitope that is a "dominant epitope." A dominant epitope may lead to such a zealous response that immune responses to other epitopes are diminished or suppressed.

7.) Where the sequences of multiple variants of the same target protein are present, potential peptide epitopes can also be selected on the basis of their conservancy. For example, a criterion for conservancy may define that the entire sequence of an HLA class I binding peptide or the entire core of a class II binding peptide be conserved in a designated percentage of the sequences evaluated for a specific protein antigen.

For minigene vaccines, a number of different approaches are available which allow simultaneous delivery of multiple epitopes. Nucleic acids encoding the peptides of the invention are a particularly useful embodiment of the invention. Epitopes for inclusion in a minigene are preferably selected according to the guidelines set forth in the previous section. Preferred nucleic acids encoding the peptides of the invention uses minigene constructs encoding a peptide comprising one or multiple epitopes of the invention.

The use of multi-epitope minigenes is described below. For example, a multi-epitope DNA plasmid encoding supermotif- and/or motif-bearing epitopes derived from MAGE-A9, the PADRE.RTM. universal helper T cell epitope or multiple HTL epitopes from MAGE-A9 (see e.g.,Tables 5, 6, 7 and 8), and an endoplasmic reticulum-translocating signal sequence can be engineered. A vaccine may also comprise epitopes that are derived from other TAAs.

An embodiment of a vaccine composition in accordance with the invention comprises *ex vivo* administration of a cocktail of epitope-bearing peptides to PBMC, or isolated DC therefrom, from the patient's blood. A pharmaceutical that facilitates the harvesting of DC can also be used, such as Progenipoietin™ (Pharmacia-Monsanto, St. Louis, Mo.) or GM-CSF/IL-4. After pulsing the DC with peptides and prior to reinfusion into patients, the DC are washed to remove unbound peptides. In this embodiment, a vaccine comprises peptide-pulsed DCs which present the pulsed peptide epitopes complexed with HLA molecules on their surfaces.

The DC can be pulsed *ex vivo* with a cocktail of peptides, some of which stimulate CTL responses to MAGE-A9. Optionally, a helper T lymphocyte (HTL) peptide, such as a natural or artificial loosely restricted HLA Class II peptide, can be included to facilitate the CTL response. Thus, a vaccine in accordance with the invention is used to treat a cancer which expresses or overexpresses MAGE-A9.

For adoptive immunotherapy, antigenic MAGE-A9-related peptides are used to elicit a CTL and/or HTL response *ex vivo,* as well. The resulting CTL or HTL cells, can be used to treat tumors in patients that do not respond to other conventional forms of therapy, or will not respond to a therapeutic vaccine peptide or nucleic acid in accordance with the invention. *Ex vivo* CTL or HTL responses to a particular antigen are induced by incubating in tissue culture the patient's, or genetically compatible, CTL or HTL precursor cells together with a source of antigen-presenting cells (APC), such as dendritic cells, and the appropriate immunogenic peptide. After an appropriate incubation time (typically about 7-28 days), in which the precursor cells are activated and expanded into effector cells, the cells are infused back into the patient, where they will destroy (CTL) or facilitate destruction (HTL) of their specific target cell (e.g., a tumor cell expressing MAGE-A9). Transfected dendritic cells may also be used as antigen presenting cells.

Pharmaceutical and vaccine compositions of the invention are typically used to treat and/or prevent a cancer that expresses or overexpresses MAGE-A9. In therapeutic applications, peptide and/or nucleic acid compositions are administered to a patient in an amount sufficient to elicit an effective B cell, CTL and/or HTL response to the antigen and to cure or at least partially arrest or slow symptoms and/or complications. An amount adequate to accomplish this is defined as "therapeutically effective dose." Amounts effective for this use will depend on, e.g., the particular composition administered, the manner of administration, the stage and severity of the disease being treated, the weight and general state of health of the patient, and the judgment of the prescribing physician.

For pharmaceutical compositions, the immunogenic peptides of the invention, or DNA encoding them, are generally administered to an individual already bearing a tumor that expresses MAGE-A9. The peptides or DNA encoding them can be administered individually or as fusions of one or more peptide sequences. Patients can be treated with the immunogenic peptides separately or in conjunction with other treatments, such as surgery, as appropriate.

For therapeutic use, administration should generally begin at the first diagnosis of MAGE-A9-associated cancer. This is followed by boosting doses until at least symptoms are substantially abated and for a period thereafter. The embodiment of the vaccine composition (i.e., including, but not limited to embodiments such as peptide cocktails, polyepitopic polypeptides, minigenes, or TAA-specific CTLs or pulsed dendritic cells) delivered to the patient may vary according to the stage of the disease or the patient's health status. For example, in a patient with a tumor that expresses MAGE-A9, a vaccine comprising MAGE-A9-specific CTL may be more efficacious in killing tumor cells in patient with advanced disease than alternative embodiments.

It is generally important to provide an amount of the peptide epitope delivered by a mode of administration sufficient to stimulate effectively a cytotoxic T cell response; compositions which stimulate helper T cell responses can also be given in accordance with this embodiment of the invention.

The dosage for an initial therapeutic immunization generally occurs in a unit dosage range where the lower value is about 1, 5, 50, 500, or 1,000 μg and the higher value is about 10,000; 20,000; 30,000; or 50,000 μg. Dosage values for a human typically range from about 500 μg to about 50,000 μg per 70 kilogram patient. Boosting dosages of between about 1.0 μg to about 50,000 μg of peptide pursuant to a boosting regimen over weeks to months may be administered depending upon the patient's response and condition as determined by measuring the specific activity of CTL and HTL obtained from the patient's blood. Administration should continue until at least clinical symptoms or laboratory tests indicate that the neoplasia, has been eliminated or reduced and for a period thereafter. The dosages, routes of administration, and dose schedules are adjusted in accordance with methodologies known in the art.

In certain embodiments, the peptides and compositions of the present invention are employed in serious disease states, that is, life-threatening or potentially life threatening situations. In such cases, as a result of the minimal amounts of extraneous substances and the relative non-toxic nature of the peptides in preferred compositions of the invention, it is possible and may be felt desirable by the treating physician to administer substantial excesses of these peptide compositions relative to these stated dosage amounts.

The vaccine compositions of the invention can also be used purely as prophylactic agents. Generally the dosage for an initial prophylactic immunization generally occurs in a unit dosage range where the lower value is about 1, 5, 50, 500, or 1000 μg and the higher value is about 10,000; 20,000; 30,000; or 50,000 μg. Dosage values for a human typically range from about 500 μg to about 50,000 μg per 70 kilogram patient. This is followed by boosting dosages of between about 1.0 μg to about 50,000 μg of peptide administered at defined intervals from about four weeks to six months after the initial administration of vaccine. The immunogenicity of the vaccine can be assessed by measuring the specific activity of CTL and HTL obtained from a sample of the patient's blood.

The pharmaceutical compositions for therapeutic treatment are intended for parenteral, topical, oral, nasal, intrathecal, or local (e.g. as a cream or topical ointment) administration. Preferably, the pharmaceutical compositions are administered parentally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier.

A variety of aqueous carriers may be used, e.g., water, buffered water, 0.8% saline, 0.3% glycine, hyaluronic acid and the like. These compositions may be sterilized by conventional, well-known sterilization techniques, or may be sterile filtered. The resulting aqueous solutions may be packaged for use as is, or lyophilized, the lyophilized preparation being combined with a sterile solution prior to administration.

The compositions may contain pharmaceutically acceptable auxiliary substances as required to approximate physiological conditions, such as pH-adjusting and buffering agents, tonicity adjusting agents, wetting agents, preservatives, and the like, for example, sodium acetate, sodium lactate, sodium chloride, potassium chloride, calcium chloride, sorbitan monolaurate, triethanolamine oleate, etc.

The concentration of peptides of the invention in the pharmaceutical formulations can be of 0.01 % to 50%, of 0.01 % to 20%, of 0.01 % to 5%, of 0.01 % to 2%, or of 0.01 % to 0.1 % by weight in accordance with the particular mode of administration selected, and will be selected primarily by fluid volumes, viscosities, etc.

A human unit dose form of a composition is typically included in a pharmaceutical composition that comprises a human unit dose of an acceptable carrier, in one embodiment an aqueous carrier, and is administered in a volume/quantity that is known by those of skill in the art to be used for administration of such compositions to humans. For example a peptide dose for initial immunization can be from about 1 to about 50,000 μg, generally from about 100 to 5,000 μg, for a 70 kg patient. For example, for nucleic acids, an initial immunization may be performed using an expression vector in the form of naked nucleic acid administered IM (or SC or ID) in the amounts of 0.5 to 5 mg at multiple sites. The nucleic acid (0.1 to 1000 μg) can also be administered using a gene gun. Following an incubation period of 3-4 weeks, a booster dose is then administered. The booster can be recombinant fowlpox virus administered at a dose of $5\text{-}10^7$ to $5x10^9$ pfu.

For antibodies, a treatment generally involves repeated administration of the anti-MAGE-A9 antibody preparation, via an acceptable route of administration such as intravenous injection (IV), typically at a dose in the range of about 0.1 to about 10 mg/kg body weight. In general, doses in the range of 10-500 mg mAb per week are effective and well tolerated. Moreover, an initial loading dose of approximately 4 mg/kg patient body weight IV, followed by weekly doses of about 2 mg/kg IV of the anti-MAGE-A9 mAb preparation represents an acceptable dosing regimen. As appreciated by those of skill in the art, various factors can influence the ideal dose in a particular case. Such factors include, for example, half life of a composition, the binding affinity of an Ab, the degree of MAGE-A9 expression in the patient, the extent of circulating shed MAGE-A9 antigen, the desired steady-state concentration level, frequency of treatment, and the influence of chemotherapeutic or other agents used in combination with the treatment method of the invention, as well as the health status of a particular patient. Non-limiting preferred human unit doses are, for example, 500 μg-1 mg, 1 mg-50 mg, 50 mg-100 mg, 100 mg-200 mg, 200 mg-300 mg, 400 mg-500 mg, 500 mg-600 mg, 600 mg-700 mg, 700 mg-800 mg, 800 mg-900 mg, 900 mg-1 g, or 1 mg-700 mg. In certain embodiments, the dose is in a range of 2-5 mg/kg body weight, e.g., with follow on weekly doses of 1-3 mg/kg; 0.5 mg, 1, 2, 3, 4, 5, 6, 7, 8, 9, 10 mg/kg body weight followed, e.g., in two, three or four weeks by weekly doses; 0.5-10 mg/kg body weight, e.g., followed in two, three or four weeks by weekly doses; 225, 250, 275, 300, 325, 350, 375, 400 mg $m^2$ of body area weekly; 1-600 mg $m^2$ of body area weekly; 225-400 mg $m^2$ of body area weekly; these does can be followed by weekly doses for 2, 3, 4, 5, 6, 7, 8, 9, 19, 11, 12 or more weeks.

In one embodiment, human unit dose forms of polynucleotides comprise a suitable dosage range or effective amount that provides any therapeutic effect. As appreciated by one of ordinary skill in the art a therapeutic effect depends on a number of factors, including the sequence of the polynucleotide, molecular weight of the polynucleotide and route of administration. Dosages are generally selected by the physician or other health care professional in accordance with a variety of parameters known in the art, such as severity of symptoms, history of the patient and the like. Generally, for a polynucleotide of about 20 bases, a dosage range may be selected from, for example, an independently selected lower limit such as about 0.1, 0.25, 0.5, 1, 2, 5, 10, 20, 30, 40, 50, 60, 70, 80, 90, 100, 200, 300, 400 or 500 mg/kg up to an independently selected upper limit, greater than the lower limit, of about 60, 80, 100, 200, 300, 400, 500, 750, 1000, 1500, 2000, 3000, 4000, 5000, 6000, 7000, 8000, 9000 or 10,000 mg/kg. For example, a dose may be about any of the following: 0.1 to 100 mg/kg, 0.1 to 50 mg/kg, 0.1 to 25 mg/kg, 0.1 to 10 mg/kg, 1 to 500 mg/kg, 100 to 400 mg/kg, 200 to 300 mg/kg, 1 to 100 mg/kg, 100 to 200 mg/kg, 300 to 400 mg/kg, 400 to 500 mg/kg, 500 to 1000 mg/kg, 500 to 5000 mg/kg, or 500 to 10,000 mg/kg. Generally, parenteral routes of administration may require higher doses of polynucleotide compared to more direct application to the nucleotide to diseased tissue, as do polynucleotides of increasing length.

In one embodiment, human unit dose forms of T-cells comprise a suitable dosage range or effective amount of T-cells that provides any therapeutic effect. As appreciated by one of ordinary skill in the art, a therapeutic effect depends on a number of factors. Dosages are generally selected by the physician or other health care professional in accordance with a variety of parameters known in the art, such as severity of symptoms, history of the patient and the like. A dose may be about $10^4$ cells to about $10^6$ cells, about $10^6$ cells to about $10^8$ cells, about $10^8$ to about $10^{11}$ cells, or about 10 to about $5x10^{10}$ cells. A dose may also be about $10^6$ cells/$m^2$ to about $10^{10}$ cells/$m^2$, or about $10^6$ cells/$m^2$ to about $10^6$ cells/$m^2$.

Proteins(s) of the invention, and/or nucleic acids encoding the protein(s), can also be administered via liposomes, which may also serve to: 1) target the proteins(s) to a particular tissue, such as lymphoid tissue; 2) to target selectively to diseases cells; or, 3) to increase the half-life of the peptide composition. Liposomes include emulsions, foams, micelles, insoluble monolayers, liquid crystals, phospholipid dispersions, lamellar layers and the like. In these preparations, the peptide to be delivered is incorporated as part of a liposome, alone or in conjunction with a molecule which binds to a receptor prevalent among lymphoid cells, such as monoclonal antibodies which bind to the MAGE-A9 antigen, or with other therapeutic or immunogenic compositions. Thus, liposomes either filled or decorated with a desired peptide of the invention can be directed to the site of lymphoid cells, where the liposomes then deliver the peptide compositions. Liposomes for use in accordance with the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream.

For targeting cells of the immune system, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, *inter alia,* the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, immunogenic peptides are preferably supplied in finely divided form along with a surfactant and propellant. Typical percentages of peptides are about 0.01%-20% by weight, preferably about 1%-10%. The surfactant must, of course, be non-toxic, and preferably soluble in the propellant. Representative of such agents are the esters or partial esters of fatty acids containing from about 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute about 0.1%-20% by weight of the composition, preferably about 0.25-5%. The balance of the composition is ordinarily propellant. A carrier can also be included, as desired, as with, e.g., lecithin for intranasal delivery.

The present invention will be more readily understood by referring to the following examples which are given to illustrate the invention rather than to limit its scope.

EXAMPLE 1 : MAGE-A9 mRNA and protein expression in bladder cancer.

Twenty-four superficial (Ta or T1) and 22 invasive (≥T2) bladder tumors were collected between 1984 and 1990. One part of the tumor was sent to the pathology department of the hospital to be fixed and paraffin-embedded for routine analysis and the second part was frozen in liquid nitrogen and stored at - 80°C. Normal urothelia (mucosa) were isolated from bladder of organ donors. Normal testis specimens were either obtained from organ donors or orchiectomies.

Bladder cancer cell lines (MGH-U3, SW780, RT4, 5637 and VMCUB-3) were cultured in Minimal Essential Medium (MEM, Gibco/BRL, Burlington, ON) containing 10% fetal calf serum. CTA inductions were performed by treating cells with 5-aza-2'-deoxycytidine (5-AZA-DC) (Sigma Chemical Company, St-Louis, MO) and/or the histone deacetylase (HDAC) inhibitors Apicidin, MS-275 or 4-phenylbutyrate (4-PB) (all from Calbiochem, San Diego, CA). Cells were plated in T75 flasks to obtain a 25-50% confluence. Twenty-four hours later, the medium was replaced by one containing 1 μM 5-AZA-DC and cells were cultured for an additional forty-eight hours. Then the medium was removed and replaced by one containing 1 μM 5-AZA-DC alone or in combination with 0.5 μM Apicidin, 1 μM MS-275 or 2 mM 4-PB and incubation pursued for forty eight hours. Total RNA and proteins were extracted from cells after 96 hours of treatment.

Total RNA from frozen tumor specimens or cultured cells was isolated using the TRIzol™ reagent (Invitrogen, Burlington, ON). The amount of isolated RNA was determined by optical density at 260 nm and its quality was evaluated by visualization after formaldehyde agarose gel electrophoresis and ethidium bromide staining. All samples used were considered of good quality as 28S and 18S ribosomal RNAs were present in normal or near normal proportions. Contaminating DNA was eliminated from 10 μg RNA samples by treatment with 2 units of DNAse I (Ambion, Austin, TX) in a final volume of 20 μl. The enzyme was inactivated using the DNAse Inactivating Reagent (Ambion) following manufacturer's recommendations. To control for the presence of residual DNA contaminants, all RNA samples were submitted to direct PCR amplification omitting the RT reaction. The absence of β-actin amplicon confirmed the absence of contaminating DNA. Moreover, the β-actin primers used span a 94-bp intron, but the 222-bp product expected from genomic DNA was never observed with the 128-bp produced from the cDNA following RT-PCR amplification of the various samples. Total proteins from cultured cells treated with drugs or from MAGE-A transfectants were extracted with a lysis buffer containing 1% IGEPAL™ (ICN, Irvine, CA) and Mini-Complete™ as protease inhibitor (Roche, Laval, QC). Protein concentration was determined by the Bradford assay.

RT reactions were performed using 1 μg of DNA-free RNA, 100 ng of random hexanucleotide primers (Amersham BioSciences, Baie d'Urfé, Qc) and 200 units of MMLV reverse transcriptase (Invitrogen) in a final volume of 20 μl. To measure expression of MAGE-A genes in clinical specimens, PCR reactions were subsequently carried out using pairs of primers for the human β-actin and MAGE-A genes. PCR primers for β-actin were F-5'-TCATCACCATTGGCAATGAG-3' (SEQ ID NO:3) and R-5'-GATGTCCACGTCACACTTC-3' (SEQ ID NO:4) and those for specific MAGE-A were F-5'-TGGAGGACCAGAGGCCCCC-3' (SEQ ID NO:5) and R-5'-GGACGATTATCAGGAGGCCTGC-3' (SEQ ID NO:6) for MAGE-A 3; F-5'-GAGCAGACAGGCCAACCG-3' (SEQ ID NO:7) a n d R-5'-AAGGACTCTGCGTCAGGC-3' (SEQ ID NO:8) for MAGE-A4; F-5'-CCCCAGAGAAGCACTGAAGAAG-3 ' (SEQ ID NO:9) a n d R-5'-GGTGAGCTGGGTCCGGG-3' (SEQ ID NO:10) for MAGE-A8; F-5'-CCCCAGAGCAGCACTGACG-3 ' (SEQ ID NO:11) and R-5'-CAGCTGAGCTGGGTCGACC-3' (SEQ ID NO:12) for MAGE-A9 as previously described.

For assessment of the cDNA quantity used as template, PCR amplification of β-actin transcripts was first performed. Radioactive nucleotides were used in PCR reactions to evaluate the amount of amplicon produced. The quantity of cDNA was adjusted for each sample in order to obtain signals of similar intensity, below the level of saturation. MAGE-A gene expression was measured using 300 times more cDNA than for β-actin. PCR reactions were performed using between 1 and 5 μl of RT reaction and 0.5 units of Platinum™ Taq DNA polymerase (Invitrogen) in presence of PCR buffer containing $MgCl_2$, 0.2 μM of both primers, 0.2 mM each of dCTP, dGTP and dTTP, 0.1 mM of dATP and 2.5 μCi of dATP-[a32P] (3000 Ci/mmole, Mandel Scientific Co. Ltd., Guelph, ON) in a final volume of 25 μl. The final concentration of $MgCl_2$ and annealing temperatures that were used were as follows: β-actin: 5 mM and 52°C; MAGE-A3: 7 mM and 71°C; MAGE-A4: 3 mM and 68°C; MAGE-A8: 4 mM and 65°C; MAGE-A9: 1 mM and 63°C. Twenty-eight amplification cycles were performed for β-actin and 32 for MAGE-A genes. PCR reactions were conducted in a Perkin Elmer 9600 thermocycler (Norwalk, CT). Amplicons were analyzed on 10% polyacrylamide gels. Dried gels were exposed to a phosphor screen for 1 h before being analyzed with a PhosphoreImager Storm™ 860 (Molecular Dynamics, Sunnyvale, CA). For signal equalization, β-actin amplicons were quantified using the ImageQuant™ software (Molecular Dynamics).

Expression of MAGE-A9 in cultured cells treated with DNA methylation and HDAC inhibitors was measured by standard RT-PCR analysis. All PCR reactions were performed using 1 μl of cDNA in conditions identical to those described above but in the absence of radioactive nucleotides. PCR products were subjected to electrophoresis on 10% polyacrylamide gels and monitored under UV light after ethidium bromide staining.

For monoclonal antibodies production, MAGE-A9 cDNA was cloned using the GATEWAY™ Cloning Technology (Invitrogen). MAGE-A9 coding sequence was amplified by PCR from an EST IMAGE clone (ID: 3943015) using primers containing *attB* sequences. It was transferred into pDONR201 via a BP reaction and then transferred from this recombinant plasmid to the prokaryotic pDEST17 expression vector via a LR reaction. This expression vector enables the production of an N-terminal 6-histidine-tagged MAGE-A9 polypeptide. The recombinant protein was produced in BL21(DE3)pLysS cells (Novagen, EMD Biosciences, WI) and purified with the TALON™ Metal Affinity resin (Clontech, Mountain View, CA) following manufacturer's recommendations. Balb/c mice were immunized s.c. three times with 20 μg of recombinant MAGE-A9 mixed with 10 μg of QUIL-A (Superfos Biosector, Frederikssund, Denmark) and a fourth time *i.v.* with the same amount of immunogen alone. Splenocytes from the best responder were fused with SP2 cells on day 62. Eleven days later, more than 6000 hybridomas were tested for reactivity with the recombinant protein. Two hybridomas, 14A11 and 14A12, were selected for specificity analysis using MAGE-A transfectants. Both clones were subcloned three times. Isotypes were determined using an antibody isotyping kit from Sigma Chemical Co.

To perform competition assays, mAb 14A12 was labeled with biotin and tested in ELISA on recombinant MAGE-A9 polypeptide in presence of 0, 5, 10 and 100-fold more unlabeled competing antibodies, i.e. mAbs 14A12, 14A11 and an irrelevant antibody. Bound antibodies were revealed using peroxidase-labeled streptavidin (Jackson Immunoresearch, West Grove, PA) and 2,2'-azino-bis (3-ethylbenzthiazoline-6-sulfonic acid) (ABTS) reagent (Roche), followed by reading optical density at 405 nm.

Plasmids containing full-length cDNA encoding MAGE-A antigens under the control of a CMV promoter were provided by Dr. De Plaen (Brussels, Belgium) (MAGE-A1, A2, A3, A4, A6, A12) or purchased from Invitrogen (MAGE-A8, A9, A10, A11). These purified plasmids were used to transiently transfect 293 cells using Lipofectamine™ 2000 (Invitrogen). After 48 h, cells were lysed and tested by Western blotting. Expression of MAGE-A antigens was controlled with mAb 57B, kindly provided by Dr G. Spagnoli (Basel, Switzerland), and with mAb 6C1 (Novocastra, Newcastle upon Tyne, UK).

Immunohistochemistry was performed using 5 μm sections of paraffin-embedded tumor blocks were deparaffinized, and rehydrated. For mAb 14A11, antigen retrieval in 0.1 M Tris-HCl pH8.0, 1 mM EDTA pH8.0 and 0.01 M sodium citrate buffer pH 6.0 were compared to omitting the antigen retrieval step. While use of 1 mM EDTA caused a strong background, no antigen retrieval or use of 0.1 M Tris-HCl gave much weaker signals than 0.01 M sodium citrate buffer. Antigen retrieval was thus carried out by heating to 100°C in 0.01 M sodium citrate buffer pH 6.0 in a pressure cooker for 12 min followed by overnight incubation with hybridoma supernatants 14A11 and 57B respectively diluted 1: 50 and 1:200. Bound antibodies were revealed using the IDetect™ Ultra HRP Detection System kit (ID Labs, London, Ontario). Percent of positive cells and staining intensity were scored by two independent observers.

Western blots were performed using 50 micrograms of proteins were separated on 10% SDS-PAGE under reducing conditions and then transferred onto Hybond™ C nitrocellulose membranes (Amersham BioSciences). Filters were blocked with TBS containing 5% skim milk and incubated at room temperature for 1 h with hybridoma supernatant 14A11, 14A12, 57B or 6C1 diluted 1:10-1:50. Membranes were washed and then incubated for 1 h in presence of horseradish peroxidase-labelled goat anti-mouse secondary antibody (Jackson Immunoresearch, West Grove, PA) diluted 1:5000. Bound antibodies were revealed using Western Lightning™ Chemiluminescence Reagent (Perkin Elmer).

In order to better characterize the expression of MAGE-As in bladder tumors, we analyzed the expression of MAGE-A3, A4, A8 and A9 mRNAs. This RT-PCR analysis using specific primers was performed on the same panel of tissues used in our previous analysis. It was composed of 6 Ta, 18 T1, 9 T2, 10 T3 and 3 T4 tumors and of 10 normal urothelium (mucosa only) samples. Figure 1 shows the amplicons obtained after amplification of β-actin, and the four MAGE-A sequences. MAGE-A3 transcripts were observed in 9 out of 24 (37%) superficial tumors and in 5 out of 22 (23%) invasive tumors. MAGE-A4 mRNA was detected in 10 out of 24 (42%) superficial tumors and in 5 of the 22 (23%) invasive tumors. PCR amplification of MAGE-A8 mRNA is expected to result in a 399-bp product according to De Plaen et al. (Immunogenetics 1994;40:360-9). However, although this product could be observed in 3 superficial tumors and in the testis sample, a 473-bp product was more often observed in most superficial tumors and in some invasive tumors but also in 7 out of 10 normal urothelia. The existence of this product is explained by the fact that the PCR primers used span a 74-bp intron that appears to be frequently not excised. Sequencing of both PCR products confirmed the presence of the 74-bp intron in the 473-bp product. To ensure that this band does not result from amplification of contaminating genomic DNA, a second round of DNAse treatment was performed on a selection of RNA samples presenting the 473-bp product. PCR amplification of MAGE-A8 mRNA from RT reactions made with these RNA still showed the presence of the 473-bp product while PCR reactions performed on the same RT reactions in which the reverse transcriptase was omitted showed no amplification, confirming that this product results from RNA and not contaminating DNA. Thus, the expression of MAGE-A8 transcript was found in 21 out of 24 (87%) superficial and in 5 out of 22 (23%) invasive tumors but in most cases the level of expression was not much higher than in the 7 positive normal urothelium samples. MAGE-A9 mRNA was specifically expressed in 17 out 24 (71 %) superficial tumors and in 8 out of 22 (36%) invasive tumors. Moreover, the level of expression observed in about half of the positive tumors was comparable to that found in testis. No MAGE-A gene other than MAGE-A8 was expressed in normal urothelia.

The RT-PCR expression analysis showed that MAGE-A9 might be good target for superficial bladder cancer immunotherapy because of its frequent and rather strong expression in these tumors. MAGE-A9 is an antigen that has been poorly characterized so far. To further study its expression, we undertook the production of a MAGE-A9-specific mAb. A recombinant histidine-tagged MAGE-A9 polypeptide was produced in *E. coli* and used to immunize mice. Human MAGE-A9 was found to be highly immunogenic since a large majority of the hybridomas produced after fusion of the mouse splenocytes with SP2 cells reacted with this antigen. Two hybridomas, 14A11 and 14A12, both IgG2a, were selected for further characterization. Each mAb reacted with a distinct epitope as no competition was observed between the two antibodies in competition assays. They were shown to react specifically with lysate of MAGE-A9 293 transfectants and not with lysates of other MAGE-A 293 transfectants (Figure 2). Expression of MAGE-A antigens in these cells was controlled with 57B and 6C1, two mAbs cross-reacting with several MAGE-A antigens.

MAbs 14A11 and 14A12 were used to stain sections of fixed and paraffin-embedded human testis specimens. MAb 57B was used as a positive control for MAGE-A expression. Of the two anti-MAGE-A9 antibodies, mAb 14A11 was selected for further analysis as it gave a clear and specific staining after antigen retrieval. MAb 14A11 reactivity was mainly observed on primary spermatocytes (Fig. 3A) whereas mAb 57B was positive mostly on spermatogonia (Fig. 3B). The 14A11 staining was nuclear on primary spermatocytes but a weaker staining was also observed on some spermatogonia nuclei. No reactivity with spermatids or spermatozoa was observed suggesting that MAGE-A9 expression was associated with early spermatogenesis. No reactivity was either observed on other testicular cells such as Sertoli and Leydig cells.

Immunohistochemical staining with mAbs 14A11 and 57B was then carried out on 36 of the 46 tumors previously analysed for gene expression. Reactivity of the mAbs with tumors showed a good concordance with mRNA expression but with some disparities. Fifteen of the 20 tumors expressing MAGE-A9 transcript, also expressed the protein as detected with mAb 14A11 while 5 showed no reactivity with the antibody (Table 1). However, none of the tumors that were found negative in RT-PCR analyses was positive with mAb 14A11. Overall, 10 out of 21 superficial (48%) and 5 out of 15 invasive tumors (33%) were found positive with this mAb. The staining in tumors was mostly heterogeneous ranging between 1 and 100% of positive cells. However, nearly half of the positive cases (*i.e.* 8/15) showed expression of MAGE-A9 in 75% or more of the tumor cells. In most tumors, the staining was cytoplasmic but occasionally also observed in nuclei (Fig. 3C). As a comparison, Table 1 also shows the results of expression of MAGE-A4, which is predominantly detected by mAb 57B by immunohistochemical analysis (Landry C. et al., Int J Cancer 2000;86:835-41). Eleven tumors expressed MAGE-A4 mRNA, while 13 were positive with mAb 57B. Overall, 4 tumors that did not express MAGE-A4 mRNA were positive with this mAb. As for mAb 14A11, staining was heterogeneous. However, contrary to MAGEA9, only 3 out of 13 positive tumors showed more than 75% of tumor cells stained. As for MAGE-A9, MAGE-A4 staining was mostly cytoplasmic but in some cases, nuclei were also stained (Fig. 3D). Of the 4 tumors that were positive with mAb 57B with no MAGE-A4 transcript expression, 3 expressed MAGE-A9 transcript (Tum-614, 642 and 646). To rule out a possible cross reactivity of 57B with MAGE-A9, we tested the two mAbs on xenografts of the bladder cancer cell line RT4, previously shown to strongly express MAGE-A9 transcript. MAb 14A11 strongly reacted with this tissue while 57B showed no reactivity. Other immunohistochemistry analyses showed that MAGE-A9 expression was tumor specific as mAb 14A11 showed no reactivity on 4 samples of normal urothelium and liver and on 2 samples of normal kidney, colon and lung tissues.

CTA gene expression has been shown to be regulated by epigenetic mechanisms such as DNA methylation and histone acetylation/deacetylation. In order to assess a possible induction of the expression of MAGE-A9 by inhibition of DNA methylation and HDAC, MGH-U3, RT4, SW780, 5637 and VMCUB-3 bladder cancer cells were treated with 1 $\mu$M of the methylation inhibitor 5-AZA-DC for 96 hours and/or with either 0.5 $\mu$M Apicidin, 1 $\mu$M MS-275 or 2 mM 4-PB for the last 48 hours. RT-PCR analysis of MAGEA9 expression showed that it was constitutively expressed in RT4 and SW780 and was very slightly modulated by the addition of the drugs. However, MAGE-A9 mRNA could be strongly induced in MGH-U3, 5637 and VMCUB-3 after 96 hours of treatment with 5-AZA-DC. Treatment of these cells with the 3 HDAC inhibitors alone did not induce MAGE-A9 expression. However, addition of MS-275 or 4-PB to cells previously treated for 48 hours with 5-AZA-DC augmented considerably the level of MAGE-A9 mRNA expression while addition of Apicidin had little or no effect (Figure 4A).

In order to analyze induction of the MAGE-A9 polypeptide, we tested by Western blotting, mAb 14A11 on lysates of cells treated with the combination of methylation and HDAC inhibitors. Figure 4B shows that expression of the MAGE-A9 polypeptide correlated with mRNA expression. MAGE-A9 was strongly expressed in RT4 and SW780 cells and was not significantly modulated by the drugs. However, MAGE-A9 induction could be observed in MGH-U3, 5637 and in VMCUB-3.

In our previous analysis using primers able to amplify MAGE-A1, -A2, -A3, -A4, - A6, -A8, -A9, -A12 but not -A10 and -A11 we observed a very weak expression of MAGE-A mRNAs in 7 out 10 normal urothelium samples. However 67% of superficial and 64% of invasive bladder tumors expressed MAGE-A mRNAs above the level observed in normal urothelia. In the present study we further characterized the expression of four specific MAGE-As. We found that, overall, MAGE-A3, -A4, -A8 and -A9 transcripts were respectively expressed in 30%, 33% 56% and 54% of the 46 bladder tumors analyzed. Patard et al. (Int J Cancer 1995;64:60-4) found expression of MAGE-A3 and/or A4 in 24/57 (42%) bladder tumors which is similar to our 19/46 (41 %). As reported by Bar-Haim et al. (Br J Cancer 2004;91:398-407), we also found high occurrence of MAGE-A8 with 21 out of 24 (87%) superficial but much fewer invasive tumors, *i.e.* 5 out of 22 (23%) expressing this transcript. However, contrary to these authors, we found that MAGE-A8 transcript was also expressed in 7 out of the 10 normal urothelia tested at a level similar to that observed in tumors. Only 4 out of the 24 (15%) superficial tumors over-expressed MAGE-A8 compared to normal urothelium. These results undermine the potential of this antigen as a relevant target for bladder cancer immunotherapy.

Most interestingly, we report here that the MAGE-A9 transcript was found in 71 % of superficial and 36% of invasive tumors. Since this transcript was not found in normal urothelium, this makes MAGE-A9 a highly relevant target for superficial bladder cancer immunotherapy. Such a frequent expression of MAGE-A9 was unexpected as it has always been considered to be rarely expressed (De Plaen E. et al., Immunogenetics 1994;40:360-9). However, Eichmüller et al. (Int J Cancer 2003;104:482-7) observed its expression in 27% of cutaneous T-cell lymphomas while Lin et al. (Clin Cancer Res 2004;10:5708-16) found MAGE-A9 overexpression in 6.7% of esophageal adenocarcinomas13,14. More recently, Oehlrich et al. (Int J Cancer 2005;117:256-64) reported that MAGE-A9 mRNA was found in 38% renal carcinomas. These results and ours suggest that MAGE-A9 might be expressed more frequently than first expected.

In order to study MAGE-A9 polypeptide expression, we produced a mAb able to detect the antigen in fixed and paraffin-embedded tissues. Reactivity of mAb 14A11 on testis specimens was compared with reactivity of mAb 57B, known to detect preferentially MAGE-A4-positive cells using IHC. While mAb 57B mainly stained spermatogonia, 14A11 was mostly reactive with the more differentiated primary spermatocytes. Other mAbs to CTAs such as MAGE-A116, NY-ESO-117,18, MAGE-C119, SSX2-420 have been produced and analysis of their reactivity with human testes has shown that none of them react primarily with primary spermatocytes. Such a distinct pattern of expression of MAGE-A9 in germ cells suggests that MAGE-A9 function is not redundant with that of other CTAs. It is thus tempting to speculate that tumors expressing predominantly MAGE-A9 may have a different biological behavior from those expressing predominantly other CTAs. In this regard, it is noteworthy that we observed MAGE-A9 expression more frequently in the more differentiated superficial bladder tumors while to the opposite, expression of other CTAs such as NY-ESO-1, BAGE, MAGE-C1, SSX-2 and -4 was more frequent in the poorly differentiated invasive tumors.

In a sub-panel of 36 tumors, we compared MAGE-A4 and -A9 protein expression as detected by IHC using mAbs 57B and 14A11 with transcript expression as detected by RT-PCR analysis (Table 1). The few discrepancies observed between protein and mRNA expression can possibly be explained by the highly heterogeneous expression of CTAs. The frozen pieces of tissue used for RNA extraction and the fixed pieces used for IHC analysis might originate from distinct parts of the tumors. Unfortunately, it was not possible to carry out Western blot analysis with mAb 14A11 on the proteins extracted from the same tumor pieces as was RNA. Another explanation for discrepancies observed in some tumors could be the cross reactivity of the mAbs with other MAGE-A antigens. However, we have shown here that mAb 14A11 reacts specifically on Western blot with lysates of MAGE-A9 transfectants but not MAGE-A1, A2, A3, A4, A6, A8, A10, A11 and A12 transfectants. Thus, lack of specificity does not seem to explain the discrepancy in data for this mAb. On the other hand, in the same experiment, mAb 57B was shown to cross-react with MAGE-A2, A3, A4, A6 and A12, as expected, and not with -A9 and -A10. Although it was reported that mAb 57B detects predominantly MAGE-A4-positive tumors because of the higher expression of this antigen (Landry C et al., Int J Cancer 2000;86: 835-41), we can not rule out that mAb 57B reactivity might be due to MAGE-A antigens other than MAGE-A4. In our panel, 3 out of the 4 57B-positive tumors did not express MAGE-A4 mRNA but did express MAGE-A9 transcript suggesting that 57B might cross-react with MAGE-A9 in fixed and paraffin-embedded tissues. However, several tumors that were 14A11 positive were 57B negative. Moreover, no reactivity of 57B was observed when tested on xenografts of the RT4 cell line which strongly and homogeneously expresses MAGE-A9 and react with mAb 14A11. All together, these results indicate that 57B does not react with MAGE-A9 but could, in some tumors, detect MAGE-A antigens other than MAGE-A4.

Expression of CTAs is regulated by epigenetic mechanisms such as DNA methylation and histone acetylation/deacetylation. In the present study we tried to identify drugs or combination of drugs that could be used to modulate the expression of MAGE-A9. We showed that treatment of cells with 5-AZA-DC 22 strongly induced the expression of MAGE-A9 in cell lines that did not express this antigen. Addition of drugs inhibiting histone deacetylation, especially MS-275 and 4-PB, could even push up the induction to a higher level of expression while treatment of cells with these same drugs alone had no effect. The use of these drugs to induce expression of CTA is particularly interesting in the context of superficial bladder cancer since they could be administered locally by intravesical instillation. In addition, when used after transurethral resection, the drugs are expected to reach a maximal number of tumors cells since after surgery, the tumor burden is very low. When combined with an appropriate vaccination strategy targeting MAGE-A9, one might expect a high efficacy of this chemoimmunotherapeutic approach.

Thus, the frequent expression of MAGE-A9 in superficial bladder tumors and the possibility of inducing its expression using chemotherapeutic drugs suggest that it is a relevant target for the development of a vaccine to prevent recurrence of bladder cancer after surgery.

Table 1: Immunohistochemical analysis of MAGE-A9, using mAb 14A11, and MAGEA4, using mAb 57B, on the same tumors analyzed by RT-PCR.

| | | | | RT-PCR* | IHC mAb 14A11 | | | RT-PCR* | IHC mAb 57b | | |
|---|---|---|---|---|---|---|---|---|---|---|---|
| | Samples | Grade | Stage | MAGE-A9 | Staining** | Intensity*** | Localization**** | MAGE-A4 | Staining** | Intensity** | Localization**** |
| Superficial tumours | Tum-600 | 2 | Ta | - | - | | | - | - | | |
| | Tum-607 | 2 | T1 | - | - | | | - | - | | |
| | Tum-616 | 1 | Ta | - | - | | | - | - | | |
| | Tum-617 | 2 | T1 | - | - | | | ++ | - | | |
| | Tum-620 | 2 | T1 | - | - | | | - | - | | |
| | Tum-622 | 2 | T1 | - | - | | | - | + | - | cytoplasm |
| | Tum-624 | 2 | Ta | - | - | | | - | - | | |
| | Tum-602 | 2 | T1 | + | - | | | - | - | | |
| | Tum-604 | 3 | T1 | + | - | | | - | - | | |
| | Tum-615 | 3 | T1 | + | - | | | - | - | | |
| | Tum-618 | 2 | T1 | + | - | | | - | - | | |
| | Tum-621 | 3 | T1 | +++ | + | +/++ | cyt. + nuc. | +++ | ++++ | +/++ | cyt. + nuc. |
| | Tum-605 | 2 | T1 | +++ | + | + | cytoplasm | ++ | ++ | +/++ | cytoplasm |
| | Tum-608 | 2 | T1 | ++ | ++ | ++ | cytoplasm | - | - | | |
| | Tum-606 | 3 | Ta | +++ | +++ | + | cytoplasm | - | - | | |
| | Tum-613 | 3 | T1 | +++ | ++++ | +/++ | cytoplasm | +++ | + | +/++ | cytoplasm |
| | Tum-614 | 2 | T1 | +++ | ++++ | +/++ | cyt. + nuc. | - | + | +/++ | cytoplasm |
| | Tum-611 | 2 | T1 | +++ | ++++ | + | cytoplasm | ++ | + | + | cyt. + nuc. |
| | Tum-623 | 2 | Ta | +++ | ++++ | ++ | cyt. + nuc. | +++ | +++ | ++ | cyt. + nuc. |
| | Tum-644 | 2 | T1 | +++ | ++++ | +/++ | cytoplasm | + | + | + | cytoplasm |
| | Tum-619 | 1 | T1 | +++ | ++++ | + | cytoplasm | + | - | | |
| | Tum-603 | 3 | T1 | +++ | N.D. | | | - | N.D. | | |
| | Tum-609 | 2 | T1 | ++ | N.D. | | | ++ | N.D. | | |
| | Tum-612 | 3 | Ta | +++ | N.D. | | | + | N.D. | | |
| | Total superficial tumours | | | 17/24 (71%) | 10/21 (45%) | | | 10/24 (42%) | 8/21 (38%) | | |
| Invasive tumours | Tum-645 | 3 | T3a | - | - | | | - | - | | |
| | Tum-647 | 3 | T3a | - | - | | | - | - | | |
| | Tum-650 | 2 | T2 | - | - | | | - | - | | |
| | Tum-651 | 2 | T4a | - | - | | | - | - | | |
| | Tum-652 | 3 | T2a | - | - | | | - | - | | |
| | Tum-655 | 3 | T2b | - | - | | | - | - | | |
| | Tum-656 | 3 | T3a | - | - | | | - | - | | |
| | Tum-657 | 3 | T2 | - | - | | | - | - | | |
| | Tum-658 | 3 | T2 | - | - | | | ++ | ++++ | +/++ | cytoplasm |
| | Tum-661 | 2 | T3a | + | - | | | - | - | | |
| | Tum-642 | 3 | T3a | ++ | + | + | cytoplasm | - | + | + | cytoplasm |
| | Tum-659 | 2 | T2 | +++ | ++ | ++/+++ | cytoplasm | ++ | + | +/++ | cytoplasm |
| | Tum-646 | 3 | T2b | + | ++ | + | cytoplasm | - | + | + | cytoplasm |
| | Tum-643 | 2 | T4a | ++ | ++++ | + | cytoplasm | - | - | | |
| | Tum-660 | 2 | T2 | +++ | ++++ | ++ | cyt. + nuc. | ++ | ++++ | +/++ | cyt. + nuc. |
| | Tum-638 | 3 | T3a | - | N.D. | | | - | N.D. | | |
| | Tum-639 | 3 | T3a | ++ | N.D. | | | +++ | N.D. | | |
| | Tum-641 | 3 | T3a | - | N.D. | | | - | N.D. | | |
| | Tum-649 | 3 | T2 | - | N.D. | | | - | N.D. | | |
| | Tum-653 | 3 | T4a | - | N.D. | | | ++ | N.D. | | |
| | Tum-654 | 3 | T3a | ++ | N.D. | | | - | N.D. | | |
| | Tum-662 | 3 | T3 | - | N.D. | | | - | N.D. | | |
| | Total invasive tumours | | | 8/22 (36%) | 5/15 (33%) | | | 5/22 (23%) | 5/15 (33%) | | |
| | Total all tumours | | | 25/46 (54%) | 15/36 (42%) | | | 15/46 (33%) | 13/36 (36%) | | |

* Results of RT-PCR analysis (Fig. 1) expressed as -, +, ++ or +++ are reproduced here for comparison

** +: 1-24%; ++: 25-49%; +++: 50-74%; ++++: 75-100% of stained cells. -: no staining

*** +: weak; ++, moderate; +++, strong expression. A combination of two staining intensities may be observed in a same tissue.

**** cytoplasm: cytoplasmic staining; cyt. + nuc.: cytoplasmic and nuclear staining

N.D.: Not determined

Discrepancies between RT-PCR and IHC results are boxed

EXAMPLE 2 : Humoral response against MAGE-A9.

Serum samples from subjects with bladder cancer (n=163) and from healthy individuals (n=13) were collected between 1985 and 2005 at the L'H6tel-Dieu de Quebec and stored aliquoted at -20°C.

ELISA assays were performed using Maxisorp™ plates (NUNC) that were coated overnight at 37°C with 0.1 μg of recombinant MAGE-A9 polypeptide. Plates were washed with TBS and blocked with TBS containing 5% skimmed milk for 1 hour. Sera diluted 1:100 in TBS containing 1% skimmed milk were added at reason of 50 μl per well and incubated for 90 min. After several washes with TBS, peroxidase-conjugated goat anti-human antibody (Jackson ImmunoResearch Laboratories) was added to each well at a dilution of 1:5000 in TBS containing 1% skimmed milk. After several washed bound antibodies were revealed by addition of a solution of ABTS 1mg/ml. The reaction was stopped after 15 min and O.D. 405 nm was obtained.

For Western blotting, 250 ng of MAGE-A9 recombinant protein were separated on 10% SDS-PAGE and then transferred onto Hybond™ C nitrocellulose membranes (Amersham BioSciences). Filters were blocked with TBS containing 5% skimmed milk and incubated at room temperature for 90 min with human sera diluted 1:100 in TBS containing 1 % skimmed milk or with a rabbit polyclonal antibody against MAGE-A9 (Orbigen) diluted 1:500. Membranes were washed and then incubated for 1 h in presence of horseradish peroxidase-labelled goat anti-human IgG secondary antibody (Jackson Immunoresearch Laboratory) diluted 1:5000 in TBS containing 1% non-fat dry milk. Bound antibodies were revealed using an enhanced chemiluminescence kit (Perkin Elmer).

To identify subjects that have mounted a humoral response against MAGE-A9, we tested in ELISA the reactivity of 163 serum samples from subjects with bladder cancer and 13 serum samples from healthy individuals as controls. Twenty-four serum samples from bladder cancer subjects (15%) showed a signal that was above the background (identified by the reactivity of normal sera and of most of the subject serum samples). To ensure that these sera were reactive with MAGE-A9, we tested them in Western blot. From these, only one serum sample (Sample #0290-S) showed reactivity with the recombinant MAGE-A9 (Fig. 5).

The Western blot result clearly shows that at least one subject has mounted an IgG antibody response against MAGE-A9 indicating that this antigen is immunogenic as it can induce a humoral response.

EXAMPLE 3 : PREDICTIVE VALUE OF MAGE-A9 IN BLADDER CANCER

In this example, analysis by immunohistochemistry (IHC), using mAb 14A11, the expression of MAGE-A9 in retrospective cohorts of superficial and invasive bladder tumors was performed in order to assess a possible prognostic value associated with MAGE-A9 expression. As a point of comparison, the expression of MAGE-A4, as detected by mAb 57b, was also included in this study as this antigen is also frequently expressed in bladder tumors.

The "Laboratoire d'uro-oncologie expérimentale" at the L'Hôtel-Dieu de Québec has collected sample throughout several years to create a superficial (Ta-T1) and an invasive (T2-T4) tumor banks. Our superficial tumor bank is composed of 381 primary Ta-T1 tumors. The median follow-up of these patients is 5.6 years. Nearly 65% of the patients had a recurrence and 5.8% had seen their disease progress. The muscle invasive tumor bank includes tumors form 288 patients with a mean follow-up of 33 months. Local recurrence was observed in 28 cases (11.9 %) and metastatic recurrence in 55 cases (23.3 %). Disease free survival at 3 years was 59.0 %. Beside these large tumour banks, a small panel of 10 Tis samples is also available.

Five μm sections of 193 superficial tumors (Ta-T1) and sections from 6 tissue microarray blocks (4 cores/ tumor) of 288 invasive (T2+) bladder tumors (fixed and paraffin-embedded) were tested with mAbs 14A11 (to MAGE-A9) and 57b (to MAGE-A4). Detection was performed after an antigen retrieval step carried out by heating at 100°C in 0.01 M sodium citrate pH 6.0 in a pressure cooker for 12 min. MAb 14A11 and 57b hybridoma supernatants were used diluted respectively at 1:50 and 1:200. After overnight incubation at room temperature, bound antibodies were revealed using the IDetect™ Ultra HRP Detection System Kit (ID labs). Percent of positive cells and staining intensity were scored by two independent observers.

MAGE-A9 expression (defined as 1% or more positive cells) was found in 63% of Ta-T1 and in 57% of T2-T4 tumors while MAGE-A4 was found in 38% of Ta-T1 and 50% T2-T4 tumors (Table 2). Using a cut-off of 30% or more positive cells, MAGE-A9 expression was found in 29% of Ta-T1 and in 45% of T2-T4 tumours while that of MAGE-A4 was found in 8% of Ta-T1 and 34% of T2-T4 tumours (Table 2). Strong expression of MAGE-A9 or MAGE-A4 was found more frequently in invasive than in superficial tumors. Strong expression of MAGE-A9 or MAGE-A4 was found in similar proportions of invasive cancer whereas in superficial tumors strong MAGE-A9 expression was found 3 to 4 times more frequently than that of MAGE-A4 (Table 3 and Figure 6). Mean expression of both MAGE-A4 and MAGE-A9 correlated with stage and grade (Figure 6). Mean expression of MAGE-A9 compared to MAGE-A4 was significantly higher in superficial tumors but was comparable in T2 and T4 tumors. Reccurence-free survival was calculated for MAGE-A4 and MAGE-A9 positive and negative superficial bladder tumors. Figure 7 shows no difference in behaviour between MAGE-A4 positive and negative tumors (7A) however patients with MAGE-A9 positive tumors (7B) seems to recur more frequently than patients with MAGE-A9 negative tumors (p=0.01). Thus absence of MAGE-A9 expression is significantly associated with a longer recurrence-free survival.

MAGE-A9 is more frequently expressed in bladder tumors than MAGE-A4 (Table 2, 3 and Fig. 6).

In superficial tumors, MAGE-A9 is 2-3 times more frequently expressed than MAGE-A4 while in invasive tumors the difference of expression is not significant (Figure 6).

In superficial tumors, absence of MAGE-A9, but not MAGE-A4, is associated with a longer recurrence-free survival (Figure 7).

Table 2 : Distribution of MAGE-A4 and MAGE-A9 expression according to percentage of positive cells.

| | **MAGE-A4 (57b)** | | | | **MAGE-A9 (14A11)** | | | |
|---|---|---|---|---|---|---|---|---|
| Stage | n | 0% | ≥ 5% | ≥ 30% | n | 0% | ≥ 5% | ≥ 30% |
| Ta-T1 | 185 | 114 (62%) | 38 (21%) | 15 (8%) | 186 | 69 (37%) | 85 (46%) | 54 (29%) |
| T2+ | 269 | 135 (50%) | 118 (44%) | 91 (34%) | 280 | 121 (43%) | 146 (52%) | 125 (45%) |

Table 3 : Distribution of MAGE-A4 and MAGE-A9 expression in categories combining percentage of positive cells and staining intensity.

| | MAGE-A4 (57b) | | | | MAGE-A9 (14A11) | | | | |
|---|---|---|---|---|---|---|---|---|---|
| Stage | *n* | *Negative* | *Weak* | *Strong* | *n* | *Negative* | *Weak* | *Strong* | p |
| | | 94 | 43 | 5 | | 58 | 68 | 17 | |
| Ta | 142 | (66%) 20 | (30%) 18 | (4%) 2 | 143 | (40%) 11 | (48%) 22 | (12%) 8 | p < 0,0001 |
| T1 | 40 | (50%) 26 | (45%) 11 | (5%) 13 | 41 | (27%) 23 | (54%) 11 | (19%) 18 | p = 0,02 |
| T2 | 50 | (52%) 72 | (22%) 32 | (26%) 28 | 52 | (44%) 58 | (21 %) 40 | (35%) 42 | p = 0,006 |
| T3 | 132 | (55%) 21 | (24%) 14 | (21%) 17 | 140 | (41%) 24 | (29%) 12 | (30%) 23 | p = 0,4 |
| T4 | 52 | (40%) 39 | (27%) 17 | (33%) 1 | 59 | (41%) 33 | (20%) 22 | (39%) 2 | p = 0,02 |
| G1 | 57 | (68%) 79 | (30%) 42 | (2%) 9 | 57 | (58%) 44 | (39%) 60 | (3%) 26 | p < 0,0001 |
| G2 | 130 | (61%) 109 | (32%) 59 | (7%) 54 | 130 | (34%) 84 | (46%) 68 | (20%) 70 | p < 0,0001 |
| G3 | 222 | (49%) | (27%) | (24%) | 222 | (38%) | (31%) | (31%) | p = 0,003 |

EXAMPLE 4 : Expression of MAGE-A9 in renal tumors.

The "Laboratoire d'uro-oncologie expérimentale" at the L'Hôtel-Dieu de Quebec has access to a cohort of 465 patients who underwent nephrectomy for RCC between January 1990 and December 2000 in two hospitals in Quebec City. This cohort is representative of incident RCCs in the whole region. For each patient, clinical data including patient characteristics, circumstances of tumor discovery, radiologic workup, biologic profile, treatment and TNM staging have been compiled in a database with a 6.5 years median follow-up. Regular updates are obtained, up to the latest contact with the patient or death. Informed consent was obtained from patients. Formalin-fixed, paraffin-embedded tissue blocks are available for all patients.

Tumors from 369 patients were included into tissue microarray (TMA) blocks. TMAs were built by arraying 0.6 mm diameter tissue cores, 0.3 mm distant in a receiver paraffin block. For each patient, we used 4 highest grade tumor cores on the arrays. Cores from one to four normal kidneys were also included in each block to control for inhibition of endogenous biotin. This allowed the inclusion of tissues from 56-59 patients per block. Hematoxylin and eosin (H&E) stained tissue sections from each tumor block were used to identify tissue areas suitable for inclusion in the array.

Five μm sections from 3 tissue microarray blocks were tested with mAbs 14A11 (to MAGE-A9) and 57b (to MAGE-A4). Inhibition of endogenous biotins was carried out using the Dako Biotin Blocking System (Dako Corporation #cat. X0590) after an antigen retrieval step carried out by heating at 100°C in 0.01 M sodium citrate pH 6.0 in a pressure cooker for 12 min. Staining was carried out using Mabs 14A11 and 57b hybridoma supernatants, diluted respectively at 1:50 and 1:200. After overnight incubation at room temperature, bound antibodies were revealed using the IDetect™ Ultra HRP Detection System Kit (ID labs). Percent of positive cells and staining intensity were scored by two independent observers.

Expression of MAGE-A4 and MAGE-A9 as detected respectively by mAbs 57b and 14A11 was analyzed in 369 renal cell tumors. Adequate data for MAGE-A4 and MAGE-A9 staining was available respectively for 358 and 357 tumors because of loss of cores or absence of tumor in cores. Expression of MAGE-A4 was found in 8.4% (30/358) of tumors while that of MAGE-A9 was found in 17.5% (63/359) of tumors. MAGE-A4 and MAGE-A9 staining was exclusively observed in the cytoplasm of tumor cells (Figure 8).

EXAMPLE 5 : Expression of MAGE-A9 in ovarian tumors.

Tumors from 158 patients operated on for sero-papillary ovarian cancers at L'Hôtel-Dieu de Québec between 1998 and 2003 were analysed in this study. Mean age of patients was 61 years. Tumors were mostly high grade (66% grade 3) and stage III or IV according to the FIGO staging system (Fédération Internationale de Gynécologie et d'Obstétrique). Following surgery, all patients received chemotherapy consisting in a combination of a platinum derived agent with taxol in most cases (112/158). Patient and tumor characteristics at surgery, and follow-up, until death or last visit (median 26.1 months) are available. Sixty-four patients died of their cancer.

Tissue microarray blocks were built using 0.6 mm cores from formaldehyde fixed, paraffin embedded tumor tissues. In most cases, primary tumors were used but when the primary tumors was small, cores were also taken from peritoneal implants. Three cores per tumors were taken.

Five μm sections from 3 tissue microarray blocks were tested with mAbs 14A11 (to MAGE-A9) and 57b (to MAGE-A4). Detection was performed after an antigen retrieval step carried out by heating at 100°C in 0.01 M sodium citrate pH 6.0 in a pressure cooker for 12 min. Mab 14A11 and 57b hybridoma supernatants were used diluted respectively at 1:50 and 1:200. After overnight incubation at room temperature, bound antibodies were revealed using the IDetect™ Ultra HRP Detection System Kit (ID labs). Percent of positive cells and staining intensity were scored by two independent observers.

Expression of MAGE-A4 and MAGE-A9 as detected respectively by mAbs 57b and 14A11 was analyzed in 158 ovarian tumors. Adequate data for MAGE-A4 and MAGE-A9 staining was available respectively for 147 and 148 tumors because of loss of cores or absence of tumor in cores. Expression of MAGE-A4 was found in 41% (61/148) of tumors while that of MAGE-A9 was found in 30% (44/147) of tumors. MAGE-A4 and MAGE-A9 staining was predominantly found in the cytoplasm but occasionally in both cytoplasm and nucleus (Figure 9).

EXAMPLE 6 : Expression of MAGE-A9 in various cancer cell lines.

Various cancer cell lines of human origin were analyzed by RT-PCR for the presence of MAGE-A9 transcript, accordingly to the method of example 1. The results are presented on table 4.

Table 4. Expression of MAGE-A9 transcript in various cancer cell line of human origin (RT-PCR results).

| ***Tissue*** | **Cell line** | **MAGE-A9 expression** |
|---|---|---|
| *Teratocarcinoma* | Tera 1 | - |
| | Tera 2 | - |
| *Brain (astrocytoma)* | AJ | - |
| | Becker | - |
| | Echevaria | - |
| | Healy | - |
| | U251 | + |
| | U373 | + |
| | Machi Cau | - |
| *Colon* | Colo 205 | - |
| | HT29 | - |
| | LS174T | - |
| | LS180 | - |
| | SK-CO-11 | - |
| | SW1417 | - |
| | Talleri | - |
| | LoVo | - |
| *Ovary* | 2774 | - |
| | SK-OV3 (OV3) | - |
| | ROAC | - |
| *Pancreas* | Capan-1 | - |
| | Capan-2 | - |
| | Hs 766T | - |
| *Skin (melanoma)* | SK-Mel-19 (Arnofsky) | N.D. |
| | SK-Mel-103 (Levy) | - |
| | SK-Mel-37 (Murawsky) | - |
| | SK-Mel-28 (Effron) | - |
| *Lung* | H1299 | - |

(continued)

| *Tissue* | Cell line | MAGE-A9 expression |
|---|---|---|
| | Luci 1 | - |
| | Luci 5 | +++ |
| | Luci 6 | - |
| | Luci 8 | - |
| | Luci 13 | +++ |
| *Prostate* | DU145 | - |
| | LNCAP | - |
| | PC-3 | - |
| *Kidney* | Caki I | - |
| | SK-RC-1 (Nemeth) | - |
| | SK-RC-39 (Parson) | - |
| | SK-RC-45 (Rupp II) | - |
| | SK-RC-10 (Cellini) | - |
| | SK-RC-18 (Epstein) | - |
| | SK-RC-2 (Brokatsy) | - |
| | SK-RC-29 (Shaughessy) | - |
| | SK-RC-48 (Chodoff) | - |
| | SK-RC-7 (Scattole) | - |
| | SK-RC-9 (Dematolay) | - |
| *Breast* | BT-20 | - |
| | CAMA-1 | - |
| | HBL-100 | - |
| | MCF7 | ++ |
| | MDA-MB-231 | - |
| | MDA-MB-361 | - |
| | MDA-MB-453 | - |
| | SK-BR-3 | - |
| | T-47D | - |
| *Uterus* | Ca ski | - |
| | Hela | - |
| | Me180 | - |
| | Siha | - |
| *Bladder* | 5637 | - |
| | 253J | - |
| | 575A | - |
| | 639V | - |
| | 647V | - |
| | J82 | - |
| | JON | - |
| | MGH-U3 | - |
| | MGH-U4 | - |
| | RT4 | +++ |
| | SW1710 | - |
| | SW780 | ++ |
| | SW800 | ++ |
| | T24 | - |
| | VMCUB-1 | - |

(continued)

| *Tissue* | Cell line | MAGE-A9 expression |
|---|---|---|
| | VMCUB-2 | N.D. |
| | VMCUB-3 | - |
| *Blood* | | |
| | Jurkat | +++ |
| | HMy2.C1 R | ++ |
| | Raj i | - |
| | HL-60 | - |
| | CCRF-SB | - |
| | CCRF-HSB-2 | - |
| | Molt-4 | - |
| | DG-75 | + |
| | AL-B1 | + |
| | K-562 | +++ |
| | U937 | - |
| *Normal Tissues* | | |
| | Liver | - |
| | Small Intestine | - |
| | Suprarenal gland | - |
| | Uterus | - |
| | Ovary | - |
| | Prostate | - |
| | Muscle | - |
| | Heart | - |
| | Spleen | - |
| | Bladder | - |

EXAMPLE 7 : Identification of HLA supermotif- and motif-bearing CTL candidate epitopes

HLA vaccine compositions of the invention can include multiple epitopes. The multiple epitopes can comprise multiple HLA supermotifs or motifs to achieve broad population coverage. This example illustrates the identification and confirmation of supermotif- and motif-bearing epitopes for the inclusion in such a vaccine composition.

The searches performed to identify the motif-bearing peptide sequences in the Tables 5, 6, 7 and 8 employ the protein sequence data from the gene product of MAGE-A9 (SEQ ID NO:2).

Table 5. Class I (9 mer) potential MAGE-A9 epitopes - RANK PEP RESULTS.

| | HLA-A0101 | | | HLA-A0201 | | |
|---|---|---|---|---|---|---|
| RANK | POS. | SEQUENCE | SCORE | POS. | SEQUENCE | SCORE |
| 1 | 167 | EVDPAGHSY (SEQ ID NO:13) | 162.0 | 107 | ALKLKVAEL (SEQ ID NO:20) | 78.0 |
| 2 | 262 | GSDPAHYEF (SEQ ID NO:14) | 126.0 | 199 | ALLIIVLGV (SEQ ID NO:21) | 76.0 |
| 3 | 94 | SVDPAQLEF (SEQ ID NO:15) | 117.0 | 223 | ALSVMGVYV (SEQ ID NO:22) | 75.0 |
| 4 | 41 | SSDSKEEEV (SEQ ID NO:16) | 85.0 | 194 | SMPKAALLI (SEQ ID NO:23) | 73.0 |
| 5 | 35 | EEETTSSSD (SEQ ID NO:17) | 78.0 | 284 | KVINYLVML (SEQ ID NO:24) | 72.0 |
| 6 | 78 | QFDEGSSSQ (SEQ ID NO:18) | 75.0 | 219 | VIWEALSVM (SEQ ID NO:25) | 68.0 |
| 7 | 28 | AQEPTGEEE (SEQ ID NO:19) | 73.0 | 200 | LLIIVLGVI (SEQ ID NO:26) | 67.0 |

| | HLA-A0301 | | | HLA-A1101 | | |
|---|---|---|---|---|---|---|
| RANK | POS. | SEQUENCE | SCORE | POS. | SEQUENCE | SCORE |
| 1 | 302 | SLYEEVLGE (SEQ ID NO:27) | 74.0 | 276 | AHAETSYEK (SEQ ID NO:34) | 79.0 |
| 2 | 37 | ETTSSSDSK (SEQ ID NO:28) | 69.0 | 203 | IVLGVILTK (SEQ ID NO:35) | 79.0 |
| 3 | 276 | AHAETSYEK (SEQ ID NO:29) | 68.0 | 37 | ETTSSSDSK (SEQ ID NO:36) | 75.0 |
| 4 | 118 | FLLHKYRVK (SEQ ID NO:30) | 68.0 | 225 | SVMGVYVGK (SEQ ID NO:37) | 71.0 |
| 5 | 203 | IVLGVILTK (SEQ ID NO:31) | 63.0 | 302 | SLYEEVLGE (SEQ ID NO:38) | 66.0 |
| 6 | 114 | ELVHFLLHK (SEQ ID NO:32) | 63.0 | 114 | ELVHFLLHK (SEQ ID NO:39) | 64.0 |
| 7 | 70 | SVYYTLWSQ (SEQ ID NO:33) | 59.0 | 70 | SVYYTLWSQ (SEQ ID NO:40) | 63.0 |

RANKPEP: http://bio.dfci.harvard.edu/Tools/rankpep.html

Table 5. Class I (9 mer) potential MAGE-A9 epitopes - RANK PEP RESULTS (continued).

| | HLA-A2402 | | | HLA-A6801 | | |
|---|---|---|---|---|---|---|
| RANK | POS. | SEQUENCE | SCORE | POS. | SEQUENCE | SCORE |
| 1 | 281 | SYEKVINYL (SEQ ID NO:41) | 110.0 | 203 | IVLGVILTK (SEQ ID NO:48) | 78.0 |
| 2 | 71 | VYYTLWSQF (SEQ ID NO:42) | 89.0 | 37 | ETTSSSDSK (SEQ ID NO:49) | 72.0 |
| 3 | 299 | CYPSLYEEV (SEQ ID NO:43) | 88.0 | 225 | SVMGVYVGK (SEQ ID NO:50) | 70.0 |
| 4 | 229 | VYVGKEHMF (SEQ ID NO:44) | 88.0 | 276 | AHAETSYEK (SEQ ID NO:51) | 68.0 |
| 5 | 141 | NYKRYFPVI (SEQ ID NO:45) | 87.0 | 114 | ELVHFLLHK (SEQ ID NO:52) | 68.0 |
| 6 | 237 | FYGEPRKLL (SEQ ID NO:46) | 79.0 | 70 | SVYYTLWSQ (SEQ ID NO:53) | 63.0 |
| 7 | 236 | MFYGEPRKL (SEQ ID NO:47) | 77.0 | 235 | HMFYGEPRK (SEQ ID NO:54) | 59.0 |

| | HLA-A3101 | | |
|---|---|---|---|
| RANK | POS. | SEQUENCE | SCORE |
| 1 | 70 | SVYYTLWSQ (SEQ ID NO:55) | 90.0 |
| 2 | 276 | AHAETSYEK (SEQ ID NO:56) | 88.0 |
| 3 | 133 | EMLESVIKN (SEQ ID NO:57) | 87.0 |
| 4 | 302 | SLYEEVLGE (SEQ ID NO:58) | 82.0 |
| 5 | 144 | RYFPVIFGK (SEQ ID NO:59) | 78.0 |
| 6 | 136 | ESVIKNYKR (SEQ ID NO:60) | 75.0 |
| 7 | 235 | HMFYGEPRK (SEQ ID NO:61) | 73.0 |

RANKPEP: http://bio.dfci.harvard.edu/Tools/rankpep.html

Table 6. Class I (9 mer) potential MAGE-A9 epitopes - BIMAS RESULTS.

| | HLA-A01 | | | HLA-A0201 | | |
|---|---|---|---|---|---|---|
| RANK | POS. | SEQUENCE | SCORE | POS. | SEQUENCE | SCORE |
| 1 | 167 | EVDPAGHSY (SEQ ID NO:62) | 250.000 | 199 | ALLIIVLGV (SEQ ID NO:82) | 591.888 |
| 2 | 94 | SVDPAQLEF (SEQ ID NO:63) | 250.000 | 223 | ALSVMGVYV (SEQ ID NO:83) | 382.536 |
| 3 | 262 | GSDPAHYEF (SEQ ID NO:64) | 150.000 | 111 | KVAELVHFL (SEQ ID NO:84) | 339.313 |
| 4 | 134 | MLESVIKNY (SEQ ID NO:65) | 45.000 | 102 | FMFQEALKL (SEQ ID NO:85) | 262.591 |
| 5 | 153 | ASEFMQVIF (SEQ ID NO:66) | 27.000 | 307 | VLGEEQEGV (SEQ ID NO:86) | 237.541 |
| 6 | 189 | LGDGHSMPK (SEQ ID NO:67) | 12.500 | 270 | FLWGSKAHA (SEQ ID NO:87) | 189.678 |
| 7 | 238 | YGEPRKLLT (SEQ ID NO:68) | 11.250 | 175 | YILVTALGL (SEQ ID NO:88) | 49.993 |
| 8 | 112 | VAELVHFLL (SEQ ID NO:69) | 4.500 | 157 | MQVIFGTDV (SEQ ID NO:89) | 45.555 |
| 9 | 246 | TQDWVQENY (SEQ ID NO:70) | 3.750 | 140 | KNYKRYFPV (SEQ ID NO:90) | 44.240 |
| 10 | 280 | TSYEKVINY (SEQ ID NO:71) | 3.750 | 219 | VIWEALSVM (SEQ ID NO:91) | 37.265 |
| 11 | 249 | WVQENYLEY (SEQ ID NO:72) | 2.500 | 290 | VMLNAREPI (SEQ ID NO:92) | 23.223 |
| 12 | 2 | SLEQRSPHC (SEQ ID NO:73) | 1.800 | 284 | KVINYLVML (SEQ ID NO:93) | 15.047 |
| 13 | 254 | YLEYRQVPG (SEQ ID NO:74) | 1.800 | 221 | WEALSVMGV (SEQ ID NO:94) | 14.308 |
| 14 | 65 | ASSSISVYY (SEQ ID NO:75) | 1.500 | 24 | GLMGAQEPT (SEQ ID NO:95) | 13.510 |
| 15 | 250 | VQENYLEYR (SEQ ID NO:76) | 1.350 | 187 | SMLGDGHSM (SEQ ID NO:96) | 13.276 |
| 16 | 19 | QGEDLGLMG (SEQ ID NO:77) | 1.125 | 194 | SMPKAALLI (SEQ ID NO:97) | 7.535 |
| 17 | 203 | IVLGVILTK (SEQ ID NO:78) | 1.000 | 152 | KASEFMQVI (SEQ ID NO:98) | 7.451 |
| 18 | 114 | ELVHFLLHK (SEQ ID NO:79) | 1.000 | 100 | LEFMFQEAL (SEQ ID NO:99) | 4.865 |
| 19 | 267 | HYEFLWGSK (SEQ ID NO:80) | 0.900 | 207 | VILTKDNCA (SEQ ID NO:100) | 4.297 |
| 20 | 277 | HAETSYEKV (SEQ ID NO:81) | 0.900 | 202 | IIVLGVILT (SEQ ID NO:101) | 4.006 |

BIMAS: http://bimas.dcrt.nih.gov/molbio/hla_bind/

Table 6. Class I (9 mer) potential MAGE-A9 epitopes - BIMAS RESULTS (continued).

| | HLA-A03 | | | HLA-A1101 | | |
|---|---|---|---|---|---|---|
| RANK | POS. | SEQUENCE | SCORE | POS. | SEQUENCE | SCORE |
| 1 | 235 | HMFYGEPRK (SEQ ID NO:102) | 100.000 | 144 | RYFPVIFGK (SEQ ID NO:122) | 7.200 |
| 2 | 114 | ELVHFLLHK (SEQ ID NO:103) | 81.000 | 203 | IVLGVILTK (SEQ ID NO:123) | 6.000 |
| 3 | 203 | IVLGVILTK (SEQ ID NO:104) | 20.250 | 225 | SVMGVYVGK (SEQ ID NO:124) | 4.000 |
| 4 | 225 | SVMGVYVGK (SEQ ID NO:105) | 6.750 | 158 | QVIFGTDVK (SEQ ID NO:125) | 3.000 |
| 5 | 102 | FMFQEALKL (SEQ ID NO:106) | 6.000 | 235 | HMFYGEPRK (SEQ ID NO:126) | 0.800 |
| 6 | 134 | MLESVIKNY (SEQ ID NO:107) | 4.500 | 267 | HYEFLWGSK (SEQ ID NO:127) | 0.400 |
| 7 | 158 | QVIFGTDVK (SEQ ID NO:108) | 3.000 | 114 | ELVHFLLHK (SEQ ID NO:128) | 0.360 |
| 8 | 118 | FLLHKYRVK (SEQ ID NO:109) | 3.000 | 37 | ETTSSSDSK (SEQ ID NO:129) | 0.300 |
| 9 | 199 | ALLIIVLGV (SEQ ID NO:110) | 2.700 | 250 | VQENYLEYR (SEQ ID NO:130) | 0.120 |
| 10 | 107 | ALKLKVAEL (SEQ ID NO:111) | 1.800 | 228 | GVYVGKEHM (SEQ ID NO:131) | 0.120 |
| 11 | 148 | VIFGKASEF (SEQ ID NO:112) | 1.500 | 287 | NYLVMLNAR (SEQ ID NO:132) | 0.120 |
| 12 | 270 | FLWGSKAHA (SEQ ID NO:113) | 1.500 | 132 | AEMLESVIK (SEQ ID NO:133) | 0.120 |
| 13 | 284 | KVINYLVML (SEQ ID NO:114) | 1.215 | 101 | EFMFQEALK (SEQ ID NO:134) | 0.120 |
| 14 | 109 | KLKVAELVH (SEQ ID NO:115) | 1.200 | 103 | MFQEALKLK (SEQ ID NO:135) | 0.100 |
| 15 | 249 | WVQENYLEY (SEQ ID NO:116) | 1.200 | 284 | KVINYLVML (SEQ ID NO:136) | 0.090 |
| 16 | 194 | SMPKAALLI (SEQ ID NO:117) | 1.200 | 118 | FLLHKYRVK (SEQ ID NO:137) | 0.060 |
| 17 | 144 | RYFPVIFGK (SEQ ID NO:118) | 1.012 | 111 | KVAELVHFL (SEQ ID NO:138) | 0.060 |
| 18 | 290 | VMLNAREPI (SEQ ID NO:119) | 0.900 | 3 | LEQRSPHCK (SEQ ID NO:139) | 0.060 |
| 19 | 302 | SLYEEVLGE (SEQ ID NO:120) | 0.900 | 124 | RVKEPVTKA (SEQ ID NO:140) | 0.060 |
| 20 | 280 | TSYEKVINY (SEQ ID NO:121) | 0.900 | 135 | LESVIKNYK (SEQ ID NO:141) | 0.060 |

BIMAS: http://bimas.dcrt.nih.gov/molbio/hla_bind/

Table 6. Class I (9 mer) potential MAGE-A9 epitopes - BIMAS RESULTS (continued).

| | HLA-A24 | | | HLA-A68.1 | | |
|---|---|---|---|---|---|---|
| RANK | POS. | SEQUENCE | SCORE | POS. | SEQUENCE | SCORE |
| 1 | 281 | SYEKVINYL (SEQ ID NO:142) | 504.000 | 225 | SVMGVYVGK (SEQ ID NO:162) | 240.000 |
| 2 | 237 | FYGEPRKLL (SEQ ID NO:143) | 240.000 | 203 | IVLGVILTK (SEQ ID NO:163) | 240.000 |
| 3 | 229 | VYVGKEHMF (SEQ ID NO:144) | 150.000 | 158 | QVIFGTDVK (SEQ ID NO:164) | 240.000 |
| 4 | 71 | VYYTLWSQF (SEQ ID NO:145) | 120.000 | 136 | ESVIKNYKR (SEQ ID NO:165) | 90.000 |
| 5 | 141 | NYKRYFPVI (SEQ ID NO:146) | 60.000 | 37 | ETTSSSDSK (SEQ ID NO:166) | 90.000 |
| 6 | 236 | MFYGEPRKL (SEQ ID NO:147) | 22.000 | 114 | ELVHFLLHK (SEQ ID NO:167) | 27.000 |
| 7 | 284 | KVINYLVML (SEQ ID NO:148) | 12.000 | 218 | EVIWEALSV (SEQ ID NO:168) | 24.000 |
| 8 | 111 | KVAELVHFL (SEQ ID NO:149) | 11.520 | 235 | HMFYGEPRK (SEQ ID NO:169) | 9.000 |
| 9 | 122 | KYRVKEPVT (SEQ ID NO:150) | 10.000 | 111 | KVAELVHFL (SEQ ID NO:170) | 8.000 |
| 10 | 299 | CYPSLYEEV (SEQ ID NO:151) | 9.900 | 284 | KVINYLVML (SEQ ID NO:171) | 8.000 |
| 11 | 197 | KAALLIIVL (SEQ ID NO:152) | 9.600 | 228 | GVYVGKEHM (SEQ ID NO:172) | 6.000 |
| 12 | 112 | VAELVHFLL (SEQ ID NO:153) | 8.400 | 259 | QVPGSDPAH (SEQ ID NO:173) | 6.000 |
| 13 | 67 | SSISVYYTL (SEQ ID NO:154) | 8.400 | 118 | FLLHKYRVK (SEQ ID NO:174) | 6.000 |
| 14 | 201 | LIIVLGVIL (SEQ ID NO:155) | 7.200 | 250 | VQENYLEYR (SEQ ID NO:175) | 5.000 |
| 15 | 193 | HSMPKAALL (SEQ ID NO:156) | 7.200 | 124 | RVKEPVTKA (SEQ ID NO:176) | 4.000 |
| 16 | 17 | EAQGEDLGL (SEQ ID NO:157) | 6.000 | 306 | EVLGEEQEG (SEQ ID NO:177) | 3.600 |
| 17 | 181 | LGLSCDSML (SEQ ID NO:158) | 6.000 | 234 | EHMFYGEPR (SEQ ID NO:178) | 3.000 |
| 18 | 15 | DLEAQGEDL (SEQ ID NO:159) | 6.000 | 101 | EFMFQEALK (SEQ ID NO:179) | 2.700 |
| 19 | 175 | YILVTALGL (SEQ ID NO:160) | 6.000 | 164 | DVKEVDPAG (SEQ ID NO:180) | 1.800 |
| 20 | 127 | EPVTKAEML (SEQ ID NO:161) | 6.000 | 167 | EVDPAGHSY (SEQ ID NO:181) | 1.800 |

BIMAS: http://bimas.dcrt.nih.gov/molbio/hla_bind/

Table 6. Class I (9 mer) potential MAGE-A9 epitopes - BIMAS RESULTS (continued).

| | HLA-A3101 | | |
|---|---|---|---|
| RANK | POS. | SEQUENCE | SCORE |
| 1 | 250 | VQENYLEYR (SEQ ID NO:182) | 4.000 |
| 2 | 144 | RYFPVIFGK (SEQ ID NO:183) | 3.240 |
| 3 | 287 | NYLVMLNAR (SEQ ID NO:184) | 2.400 |
| 4 | 203 | IVLGVILTK (SEQ ID NO:185) | 1.600 |
| 5 | 225 | SVMGVYVGK (SEQ ID NO:186) | 0.600 |
| 6 | 235 | HMFYGEPRK (SEQ ID NO:187) | 0.600 |
| 7 | 114 | ELVHFLLHK (SEQ ID NO:188) | 0.480 |
| 8 | 158 | QVIFGTDVK (SEQ ID NO:189) | 0.400 |
| 9 | 284 | KVINYLVML (SEQ ID NO:190) | 0.240 |
| 10 | 199 | ALLIIVLGV (SEQ ID NO:191) | 0.160 |
| 11 | 111 | KVAELVHFL (SEQ ID NO:192) | 0.120 |
| 12 | 219 | VIWEALSVM (SEQ ID NO:193) | 0.120 |
| 13 | 258 | RQVPGSDPA (SEQ ID NO:194) | 0.120 |
| 14 | 109 | KLKVAELVH (SEQ ID NO:195) | 0.120 |
| 15 | 124 | RVKEPVTKA (SEQ ID NO:196) | 0.120 |
| 16 | 102 | FMFQEALKL (SEQ ID NO:197) | 0.120 |
| 17 | 200 | LLIIVLGVI (SEQ ID NO:198) | 0.080 |
| 18 | 175 | YILVTALGL (SEQ ID NO:199) | 0.080 |
| 19 | 267 | HYEFLWGSK (SEQ ID NO:200) | 0.060 |
| 20 | 70 | SVYYTLWSQ (SEQ ID NO:201) | 0.060 |

BIMAS: http://bimas.dcrt.nih.gov/molbio/hla_bind/

Table 7. Class I (9 mer) potential MAGE-A9 epitopes - SYFPEITHI RESULTS.

| | HLA-A01 | | | HLA-A0201 | | |
|---|---|---|---|---|---|---|
| RANK | POS. | SEQUENCE | SCORE | POS. | SEQUENCE | SCORE |
| 1 | 167 | E V **D** P A G H S **Y** (SEQ ID NO:202) | 26 | 107 | A **L** K L K V A E **L** (SEQ ID NO:222) | 30 |
| 2 | 246 | T Q **D** W V Q E N **Y** (SEQ ID NO:203) | 25 | 199 | A **L** L I I V L G **V** (SEQ ID NO:223) | 30 |
| 3 | 134 | M L **E** S V I K N **Y** (SEQ ID NO:204) | 24 | 200 | L **L** I I V L G V **I** (SEQ ID NO:224) | 26 |
| 4 | 65 | A S **S** S I S V Y **Y** (SEQ ID NO:205) | 20 | 111 | K **V** A E L V H F **L** (SEQ ID NO:225) | 25 |
| 5 | 280 | T S **Y** E K V I N **Y** (SEQ ID NO:206) | 20 | 201 | L **I** I V L G V I **L** (SEQ ID NO:226) | 25 |
| 6 | 249 | W V **Q** E N Y L E **Y** (SEQ ID NO:207) | 19 | 175 | Y **I** L V T A L G **L** (SEQ ID NO:227) | 24 |
| 7 | 115 | L V **H** F L L H K **Y** (SEQ ID NO:208) | 18 | 223 | A **L** S V M G V Y **V** (SEQ ID NO:228) | 24 |
| 8 | 137 | S V **I** K N Y K R **Y** (SEQ ID NO:209) | 17 | 284 | K **V** I N Y L V M **L** (SEQ ID NO:229) | 24 |
| 9 | 41 | S S **D** S K E E E **V** (SEQ ID NO:210) | 16 | 307 | V **L** G E E Q E G **V** (SEQ ID NO:230) | 24 |
| 10 | 64 | G A **S** S S I S V **Y** (SEQ ID NO:211) | 16 | 102 | F **M** F Q E A L K **L** (SEQ ID NO:231) | 23 |
| 11 | 94 | S V **D** P A Q L E **F** (SEQ ID NO:212) | 16 | 197 | K **A** A L L I I V **L** (SEQ ID NO:232) | 22 |
| 12 | 162 | G T **D** V K E V D **P** (SEQ ID NO:213) | 16 | 187 | S **M** L G D G H S **M** (SEQ ID NO:233) | 21 |
| 13 | 222 | E A **L** S V M G V **Y** (SEQ ID NO:214) | 16 | 302 | S **L** Y E E V L G **E** (SEQ ID NO:234) | 21 |
| 14 | 230 | Y V **G** K E H M F **Y** (SEQ ID NO:215) | 16 | 130 | T **K** A E M L E S **V** (SEQ ID NO:235) | 20 |
| 15 | 260 | V P **G** S D P A H **Y** (SEQ ID NO:216) | 16 | 202 | I **I** V L G V I L **T** (SEQ ID NO:236) | 20 |
| 16 | 262 | G S **D** P A H Y E **F** (SEQ ID NO:217) | 16 | 219 | V **I** W E A L S V **M** (SEQ ID NO:237) | 20 |
| 17 | 274 | S K **A** H A E T S **Y** (SEQ ID NO:218) | 16 | 270 | F **L** W G S K A H **A** (SEQ ID NO:238) | 20 |
| 18 | 292 | L N **A** R E P I C **Y** (SEQ ID NO:219) | 16 | 194 | S **M** P K A A L L **I** (SEQ ID NO:239) | 19 |
| 19 | 296 | E P **I** C Y P S L **Y** (SEQ ID NO:220) | 16 | 290 | V **M** L N A R E P **I** (SEQ ID NO:240) | 19 |
| 20 | 238 | Y G **E** P R K L L **T** (SEQ ID NO:221) | 14 | 15 | D **L** E A Q G E D **L** (SEQ ID NO:241) | 18 |

SYFEITHI: http://www.syfpeithi.de/Scripts/MHCServer.dll/EpitopePrediction.htm

Table 7. Class I (9 mer) potential MAGE-A9 epitopes - SYFPEITHI RESULTS (continued).

| | HLA-A03 | | | HLA-A1101 | | |
|---|---|---|---|---|---|---|
| RANK | POS. | SEQUENCE | SCORE | POS. | SEQUENCE | SCORE |
| 1 | 203 | I **V** L G V I L T **K** (SEQ ID NO:242) | 34 | 225 | S V M G V Y V G **K** (SEQ ID NO:262) | 28 |
| 2 | 158 | Q **V** I F G T D V **K** (SEQ ID NO:243) | 31 | 203 | I V L G V I L T **K** (SEQ ID NO:263) | 27 |
| 3 | 225 | S **V** M G V Y V G **K** (SEQ ID NO:244) | 28 | 158 | Q V I F G T D V **K** (SEQ ID NO:264) | 22 |
| 4 | 118 | F **L** L H K Y R V **K** (SEQ ID NO:245) | 27 | 37 | E T T S S S D S **K** (SEQ ID NO:265) | 21 |
| 5 | 109 | K **L** K V A E L V **H** (SEQ ID NO:246) | 25 | 114 | E L V H F L L H **K** (SEQ ID NO:266) | 21 |
| 6 | 167 | E **V** D P A G H S **Y** (SEQ ID NO:247) | 24 | 94 | S V D P A Q L E **F** (SEQ ID NO:267) | 19 |
| 7 | 114 | E **L** V H F L L H **K** (SEQ ID NO:248) | 23 | 65 | A S S S I S V Y **Y** (SEQ ID NO:268) | 18 |
| 8 | 284 | K **V** I N Y L V M **L** (SEQ ID NO:249) | 22 | 93 | S S V D P A Q L **E** (SEQ ID NO:269) | 18 |
| 9 | 94 | S **V** D P A Q L E **F** (SEQ ID NO:250) | 21 | 118 | F L L H K Y R V **K** (SEQ ID NO:270) | 18 |
| 10 | 148 | V **I** F G K A S E **F** (SEQ ID NO:251) | 21 | 136 | E S V I K N Y K **R** (SEQ ID NO:271) | 18 |
| 11 | 218 | E **V** I W E A L S **V** (SEQ ID NO:252) | 21 | 162 | G T D V K E V D **P** (SEQ ID NO:272) | 18 |
| 12 | 22 | D **L** G L M G A Q **E** (SEQ ID NO:253) | 20 | 132 | A E M L E S V I **K** (SEQ ID NO:273) | 17 |
| 13 | 48 | E **V** S A A G S S **S** (SEQ ID NO:254) | 20 | 153 | A S E F M Q V I **F** (SEQ ID NO:274) | 17 |
| 14 | 137 | S **V** I K N Y K R **Y** (SEQ ID NO:255) | 20 | 235 | H M F Y G E P R **K** (SEQ ID NO:275) | 17 |
| 15 | 147 | P **V** I F G K A S **E** (SEQ ID NO:256) | 20 | 301 | P S L Y E E V L **G** (SEQ ID NO:276) | 17 |
| 16 | 177 | L **V** T A L G L S **C** (SEQ ID NO:257) | 20 | 40 | S S S D S K E E **E** (SEQ ID NO:277) | 16 |
| 17 | 200 | L **L** I I V L G V **I** (SEQ ID NO:258) | 20 | 129 | V T K A E M L E **S** (SEQ ID NO:278) | 16 |
| 18 | 223 | A **L** S V M G V Y **V** (SEQ ID NO:259) | 20 | 183 | L S C D S M L G **D** (SEQ ID NO:279) | 16 |
| 19 | 249 | W **V** Q E N Y L E **Y** (SEQ ID NO:260) | 20 | 194 | S M P K A A L L **I** (SEQ ID NO:280) | 16 |
| 20 | 107 | A **L** K L K V A E **L** (SEQ ID NO:261) | 19 | 218 | E V I W E A L S **V** (SEQ ID NO:281) | 16 |

SYFEITHI: http://www.syfpeithi.de/Scripts/MHCServer.dll/EpitopePrediction.htm

Table 7. Class I (9 mer) potential MAGE-A9 epitopes - SYFPEITHI RESULTS (continued).

| | HLA-A24 | | | HLA-A6801 | | |
|---|---|---|---|---|---|---|
| RANK | POS. | SEQUENCE | SCORE | POS. | SEQUENCE | SCORE |
| 1 | 237 | F **Y** G E P R K L **L** (SEQ ID NO:282) | 24 | 114 | E **L** V H F L L H **K** (SEQ ID NO:302) | 21 |
| 2 | 229 | V **Y** V G K E H M **F** (SEQ ID NO:283) | 23 | 37 | E **T** T S S S D S **K** (SEQ ID NO:303) | 20 |
| 3 | 281 | S **Y** E K V I N Y **L** (SEQ ID NO:284) | 23 | 203 | I **V** L G V I L T **K** (SEQ ID NO:304) | 20 |
| 4 | 71 | V **Y** Y T L W S Q **F** (SEQ ID NO:285) | 22 | 225 | S **V** M G V Y V G **K** (SEQ ID NO:305) | 20 |
| 5 | 141 | N **Y** K R Y F P V **I** (SEQ ID NO:286) | 20 | 136 | E **S** V I K N Y K **R** (SEQ ID NO:306) | 19 |
| 6 | 236 | M **F** Y G E P R K **L** (SEQ ID NO:287) | 19 | 158 | Q **V** I F G T D V **K** (SEQ ID NO:307) | 17 |
| 7 | 112 | V **A** E L V H F L **L** (SEQ ID NO:288) | 17 | 101 | E **F** M F Q E A L **K** (SEQ ID NO:308) | 16 |
| 8 | 168 | V **D** P A G H S Y **I** (SEQ ID NO:289) | 15 | 198 | A **A** L L I I V L **G** (SEQ ID NO:309) | 16 |
| 9 | 174 | S **Y** I L V T A L **G** (SEQ ID NO:290) | 15 | 279 | E **T** S Y E K V I **N** (SEQ ID NO:310) | 16 |
| 10 | 194 | S **M** P K A A L L **I** (SEQ ID NO:291) | 15 | 284 | K **V** I N Y L V M **L** (SEQ ID NO:311) | 16 |
| 11 | 290 | V **M** L N A R E P **I** (SEQ ID NO:292) | 15 | 124 | R **V** K E P V T K **A** (SEQ ID NO:312) | 15 |
| 12 | 111 | K **V** A E L V H F **L** (SEQ ID NO:293) | 14 | 204 | V **L** G V I L T K **D** (SEQ ID NO:313) | 15 |
| 13 | 144 | R **Y** F P V I F G **K** (SEQ ID NO:294) | 14 | 106 | E **A** L K L K V A **E** (SEQ ID NO:314) | 14 |
| 14 | 193 | H **S** M P K A A L **L** (SEQ ID NO:295) | 14 | 197 | K **A** A L L I I V **L** (SEQ ID NO:315) | 14 |
| 15 | 253 | N **Y** L E Y R Q V **P** (SEQ ID NO:296) | 14 | 202 | I **I** V L G V I L **T** (SEQ ID NO:316) | 14 |
| 16 | 263 | S **D** P A H Y E F **L** (SEQ ID NO:297) | 14 | 214 | C **A** P E E V I W **E** (SEQ ID NO:317) | 14 |
| 17 | 67 | S **S** I S V Y Y T **L** (SEQ ID NO:298) | 13 | 234 | E **H** M F Y G E P **R** (SEQ ID NO:318) | 14 |
| 18 | 148 | V **I** F G K A S E **F** (SEQ ID NO:299) | 13 | 287 | N **Y** L V M L N A **R** (SEQ ID NO:319) | 14 |
| 19 | 152 | K **A** S E F M Q V **I** (SEQ ID NO:300) | 13 | 22 | D **L** G L M G A Q **E** (SEQ ID NO:320) | 13 |
| 20 | 200 | L **L** I I V L G V **I** (SEQ ID NO:301) | 13 | 133 | E **M** L E S V I K **N** (SEQ ID NO:321) | 13 |

SYFEITHI: http://www.syfpeithi.de/Scripts/MHCServer.dll/EpitopePrediction.htm

Table 8. Class II (15 mer) potential MAGE-A9 epitopes - SYFPEITHI RESULTS.

| | HLA-DRB1*0101 | | | HLA-DRB1*0301 | | |
|---|---|---|---|---|---|---|
| RANK | POS. | SEQUENCE | SCORE | POS. | SEQUENCE | SCORE |
| 1 | 265 | **P** A H **Y** E **F** L W **G** S K A H A E (SEQ ID NO:322) | 36 | 97 | **P** A Q **L** E **F** M F **Q** E A L K L K (SEQ ID NO:342) | 25 |
| 2 | 99 | **Q** L E **F** M **F** Q E **A** L K L K V A (SEQ ID NO:323) | 35 | 135 | **L** E S **V** I **K** N Y **K** R Y F P V I (SEQ ID NO:343) | 25 |
| 3 | 172 | **G** H S **Y** I **L** V T **A** L G L S C D (SEQ ID NO:324) | 35 | 226 | **V** M G **V** Y **V** G K **E** H M F Y G E (SEQ ID NO:344) | 25 |
| 4 | 286 | **I** N Y **L** V **M** L N **A** R E P I C Y (SEQ ID NO:325) | 35 | 158 | **Q** V I **F** G **T** D V **K** E V D P A G (SEQ ID NO:345) | 24 |
| 5 | 254 | **Y** L E **Y** R **Q** V P **G** S D P A H Y (SEQ ID NO:326) | 34 | 234 | **E** H M **F** Y **G** E P **R** K L L T Q D (SEQ ID NO:346) | 24 |
| 6 | 143 | **K** R Y **F** P **V** I F **G** K A S E F M (SEQ ID NO:327) | 33 | 185 | **C** D S **M** L **G** D G **H** S M P K A A (SEQ ID NO:347) | 23 |
| 7 | 198 | **A** A L **L** I **I** V L **G** V I L T K D (SEQ ID NO:328) | 33 | 131 | **K** A E **M** L **E** S V **I** K N Y K R Y (SEQ ID NO:348) | 22 |
| 8 | 20 | **G** E D **L** G **L** M G **A** Q E P T G E (SEQ ID NO:329) | 32 | 180 | **A** L G **L** S **C** D S **M** L G D G H S (SEQ ID NO:349) | 22 |
| 9 | 154 | **S** E F **M** Q **V** I F **G** T D V K E V (SEQ ID NO:330) | 31 | 206 | **G** V I **L** T **K** D N **C** A P E E V I (SEQ ID NO:350) | 22 |
| 10 | 218 | **E** V I **W** E **A** L S **V** M G V Y V G (SEQ ID NO:331) | 28 | 242 | **R** K L **L** T **Q** D W **V** Q E N Y L E (SEQ ID NO:351) | 22 |
| 11 | 105 | **Q** E A **L** K **L** K V **A** E L V H F L (SEQ ID NO:332) | 26 | 107 | **A** L K **L** K **V** A E **L** V H F L L H (SEQ ID NO:352) | 21 |
| 12 | 279 | **E** T S **Y** E **K** V I **N** Y L V M L N (SEQ ID NO:333) | 26 | 112 | **V** A E **L** V **H** F L **L** H K Y R V K (SEQ ID NO:353) | 21 |
| 13 | 297 | **P** I C **Y** P **S** L Y **E** E V L G E E (SEQ ID NO:334) | 26 | 198 | **A** A L **L** I **I** V L **G** V I L T K D (SEQ ID NO:354) | 21 |
| 14 | 139 | **I** K N **Y** K **R** Y F **P** V I F G K A (SEQ ID NO:335) | 25 | 109 | **K** L K **V** A **E** L V **H** F L L H K Y (SEQ ID NO:355) | 20 |
| 15 | 142 | **Y** K R **Y** F **P** V I **F** G K A S E F (SEQ ID NO:336) | 25 | 116 | **V** H F **L** L **H** K Y **R** V K E P V T (SEQ ID NO:356) | 20 |
| 16 | 145 | **Y** F P **V** I **F** G K **A** S E F M Q V (SEQ ID NO:337) | 25 | 145 | **Y** F P **V** I **F** G K **A** S E F M Q V (SEQ ID NO:357) | 20 |
| 17 | 197 | **K** A A **L** L **I** I V **L** G V I L T K (SEQ ID NO:338) | 25 | 233 | **K** E H **M** F **Y** G E **P** R K L L T Q (SEQ ID NO:358) | 20 |
| 18 | 233 | **K** E H **M** F **Y** G E **P** R K L L T Q (SEQ ID NO:339) | 25 | 9 | **H** C K **P** D **E** D L **E** A Q G E D L (SEQ ID NO:359) | 19 |
| 19 | 283 | **E** K V **I** N **Y** L V **M** L N A R E P (SEQ ID NO:340) | 25 | 68 | **S** I S **V** Y **Y** T L **W** S Q F D E G (SEQ ID NO:360) | 19 |
| 20 | 23 | **L** G L **M** G **A** Q E **P** T G E E E E (SEQ ID NO:341) | 24 | 99 | **Q** L E **F** M **F** Q E **A** L K L K V A (SEQ ID NO:361) | 19 |

SYFEITHI: http://www.syfpeithi.de/Scripts/MHCServer.dll/EpitopePrediction.htm

Table 8. Class II (15 mer) potential MAGE-A9 epitopes - SYFPEITHI RESULTS (continued).

| | HLA-DRB1*0401 | | | HLA-DRB1*0701 | | |
|---|---|---|---|---|---|---|
| RANK | POS. | SEQUENCE | SCORE | POS. | SEQUENCE | SCORE |
| 1 | 234 | E H M F Y G E P R K L L T Q D (SEQ ID NO:362) | 28 | 178 | V T A L G L S C D S M L G D G (SEQ ID NO:382) | 28 |
| 2 | 279 | E T S Y E K V I N Y L V M L N (SEQ ID NO:363) | 28 | 204 | V L G V I L T K D N C A P E E (SEQ ID NO:383) | 26 |
| 3 | 109 | K L K V A E L V H F L L H K Y (SEQ ID NO:364) | 26 | 101 | E F M F Q E A L K L K V A E L (SEQ ID NO:384) | 24 |
| 4 | 165 | V K E V D P A G H S Y I L V T (SEQ ID NO:365) | 26 | 146 | F P V I F G K A S E F M Q V I (SEQ ID NO:385) | 24 |
| 5 | 202 | I I V L G V I L T K D N C A P (SEQ ID NO:366) | 26 | 157 | M Q V I F G T D V K E V D P A (SEQ ID NO:386) | 24 |
| 6 | 69 | I S V Y Y T L W S Q F D E G S (SEQ ID NO:367) | 22 | 279 | E T S Y E K V I N Y L V M L N (SEQ ID NO:387) | 24 |
| 7 | 73 | Y T L W S Q F D E G S S S Q E (SEQ ID NO:368) | 22 | 297 | P I C Y P S L Y E E V L G E E (SEQ ID NO:388) | 24 |
| 8 | 101 | E F M F Q E A L K L K V A E L (SEQ ID NO:369) | 22 | 46 | E E E V S A A G S S S P P Q S (SEQ ID NO:389) | 22 |
| 9 | 142 | Y K R Y F P V I F G K A S E F (SEQ ID NO:370) | 22 | 60 | S P Q G G A S S S I S V Y Y T (SEQ ID NO:390) | 22 |
| 10 | 143 | K R Y F P V I F G K A S E F M (SEQ ID NO:371) | 22 | 68 | S I S V Y Y T L W S Q F D E G (SEQ ID NO:391) | 22 |
| 11 | 147 | P V I F G K A S E F M Q V I F (SEQ ID NO:372) | 22 | 92 | S S S V D P A Q L E F M F Q E (SEQ ID NO:392) | 22 |
| 12 | 172 | G H S Y I L V T A L G L S C D (SEQ ID NO:373) | 22 | 147 | P V I F G K A S E F M Q V I F (SEQ ID NO:393) | 22 |
| 13 | 218 | E V I W E A L S V M G V Y V G (SEQ ID NO:374) | 22 | 154 | S E F M Q V I F G T D V K E V (SEQ ID NO:394) | 22 |
| 14 | 265 | P A H Y E F L W G S K A H A E (SEQ ID NO:375) | 22 | 165 | V K E V D P A G H S Y I L V T (SEQ ID NO:395) | 22 |
| 15 | 297 | P I C Y P S L Y E E V L G E E (SEQ ID NO:376) | 22 | 192 | G H S M P K A A L L I I V L G (SEQ ID NO:396) | 22 |
| 16 | 301 | P S L Y E E V L G E E Q E G V (SEQ ID NO:377) | 22 | 197 | K A A L L I I V L G V I L T K (SEQ ID NO:397) | 22 |
| 17 | 46 | E E E V S A A G S S S P P Q S (SEQ ID NO:378) | 20 | 202 | I I V L G V I L T K D N C A P (SEQ ID NO:398) | 22 |
| 18 | 66 | S S S I S V Y Y T L W S Q F D (SEQ ID NO:379) | 20 | 217 | E E V I W E A L S V M G V Y V (SEQ ID NO:399) | 22 |
| 19 | 92 | S S S V D P A Q L E F M F Q E (SEQ ID NO:380) | 20 | 22 | D L G L M G A Q E P T G E E E (SEQ ID NO:400) | 20 |
| 20 | 97 | P A Q L E F M F Q E A L K L K (SEQ ID NO:381) | 20 | 38 | T T S S S D S K E E E V S A A (SEQ ID NO:401) | 20 |

SYFEITHI: http://www.syfpeithi.de/Scripts/MHCServer.dll/EpitopePrediction.htm

Table 8. Class II (15 mer) potential MAGE-A9 epitopes - SYFPEITHI RESULTS (continued).

| | HLA-DRB1*0701 | | | HLA-DRB1*1501 | | |
|---|---|---|---|---|---|---|
| RANK | POS. | SEQUENCE | SCORE | POS. | SEQUENCE | SCORE |
| 1 | 142 | **Y** K R **Y** F **P** V I **F** G K A S E F (SEQ ID NO:402) | 24 | 66 | **S** S S **I** S V **Y** Y T L W S Q F D (SEQ ID NO:422) | 34 |
| 2 | 267 | **H** Y E **F** L **W** G S **K** A H A E T S (SEQ ID NO:403) | 24 | 282 | **Y** E K **V** I N **Y** L V M L N A R E (SEQ ID NO:423) | 30 |
| 3 | 109 | **K** L K **V** A **E** L V **H** F L L H K Y (SEQ ID NO:404) | 23 | 224 | **L** S V **M** G V **Y** V G K E H M F Y (SEQ ID NO:424) | 28 |
| 4 | 143 | **K** R Y **F** P **V** I F **G** K A S E F M (SEQ ID NO:405) | 23 | 117 | **H** F L **L** H K **Y** R V K E P V T K (SEQ ID NO:425) | 24 |
| 5 | 265 | **P** A H **Y** E **F** L W **G** S K A H A E (SEQ ID NO:406) | 23 | 136 | **E** S V **I** K N **Y** K R Y F P V I F (SEQ ID NO:426) | 24 |
| 6 | 254 | **Y** L E **Y** R **Q** V P **G** S D P A H Y (SEQ ID NO:407) | 22 | 174 | **S** Y I **L** V T **A** L G L S C D S M (SEQ ID NO:427) | 24 |
| 7 | 158 | **Q** V I **F** G **T** D V **K** E V D P A G (SEQ ID NO:408) | 21 | 186 | **D** S M **L** G D **G** H S M P K A A L (SEQ ID NO:428) | 24 |
| 8 | 113 | **A** E L **V** H **F** L L **H** K Y R V K E (SEQ ID NO:409) | 20 | 198 | **A** A L **L** I I **V** L G V I L T K D (SEQ ID NO:429) | 24 |
| 9 | 132 | **A** E M **L** E **S** V I **K** N Y K R Y F (SEQ ID NO:410) | 20 | 215 | **A** P E **E** V I **W** E A L S V M G V (SEQ ID NO:430) | 24 |
| 10 | 136 | **E** S V **I** K **N** Y K **R** Y F P V I F (SEQ ID NO:411) | 20 | 217 | **E** E V **I** W E **A** L S V M G V Y V (SEQ ID NO:431) | 24 |
| 11 | 185 | **C** D S **M** L **G** D G **H** S M P K A A (SEQ ID NO:412) | 20 | 99 | **Q** L E **F** M F **Q** E A L K L K V A (SEQ ID NO:432) | 22 |
| 12 | 221 | **W** E A **L** S **V** M G **V** Y V G K E H (SEQ ID NO:413) | 20 | 109 | **K** L K **V** A E **L** V H F L L H K Y (SEQ ID NO:433) | 20 |
| 13 | 283 | **E** K V **I** N **Y** L V **M** L N A R E P (SEQ ID NO:414) | 20 | 112 | **V** A E **L** V H **F** L L H K Y R V K (SEQ ID NO:434) | 20 |
| 14 | 101 | **E** F M **F** Q **E** A L **K** L K V A E L (SEQ ID NO:415) | 19 | 126 | **K** E P **V** T K **A** E M L E S V I K (SEQ ID NO:435) | 20 |
| 15 | 234 | **E** H M **F** Y **G** E P **R** K L L T Q D (SEQ ID NO:416) | 19 | 139 | **I** K N **Y** K R **Y** F P V I F G K A (SEQ ID NO:436) | 20 |
| 16 | 235 | **H** M F **Y** G **E** P R **K** L L T Q D W (SEQ ID NO:417) | 19 | 140 | **K** N Y **K** R Y **F** P V I F G K A S (SEQ ID NO:437) | 20 |
| 17 | 285 | **V** I N **Y** L **V** M L **N** A R E P I C (SEQ ID NO:418) | 19 | 169 | **D** P A **G** H S **Y** I L V T A L G L (SEQ ID NO:438) | 20 |
| 18 | 286 | **I** N Y **L** V **M** L N **A** R E P I C Y (SEQ ID NO:419) | 19 | 192 | **G** H S **M** P K **A** A L L I I V L G (SEQ ID NO:439) | 20 |
| 19 | 20 | **G** E D **L** G **L** M G **A** Q E P T G E (SEQ ID NO:420) | 18 | 199 | **A** L L **I** I V **L** G V I L T K D N (SEQ ID NO:440) | 20 |
| 20 | 227 | **M** G V **Y** V **G** K E **H** M F Y G E P (SEQ ID NO:421) | 18 | 200 | **L** L I **I** V L **G** V I L T K D N C (SEQ ID NO:441) | 20 |

SYFEITHI: http://www.syfpeithi.de/Scripts/MHCServer.dll/EpitopePrediction.htm

Computer searches for epitopes bearing HLA Class I or Class II supermotifs or motifs are performed as follows. All translated MAGE-A9 protein sequences are analyzed using a text string search software program to identify potential peptide sequences containing appropriate HLA binding motifs; such programs are readily produced in accordance with information in the art in view of known motif/supermotif disclosures. Identified A2-, A3-, and DR-supermotif sequences are scored using polynomial algorithms to predict their capacity to bind to specific HLA-Class I (Tables 5, 6 and 7) or Class II molecules (Table 8). The method of derivation of specific algorithm coefficients has been described in Gulukota et al., J. Mol. Biol. 267:1258-126, 1997.

Protein sequences from MAGE-A9 are scanned using motif identification software, to identify 8-, 9-, 10- and 11-mer sequences containing the HLA-A2-supermotif main anchor specificity. Typically, these sequences are then scored using the protocol described above and the peptides corresponding to the positive-scoring sequences are synthesized and tested for their capacity to bind purified HLA-A*0201 molecules *in vitro* (HLA-A*0201 is considered a prototype A2 supertype molecule).

These peptides are then tested for the capacity to bind to additional A2-supertype molecules (A*0202, A*0203, A*0206, and A*6802). Peptides that bind to at least three of the five A2-supertype alleles tested are typically deemed A2-supertype cross-reactive binders. Preferred peptides bind at an affinity equal to or less than 500 nM to three or more HLA-A2 supertype molecules.

The MAGE-A9 protein sequence(s) scanned above is (are) also examined for the presence of peptides with the HLA-A3-supermotif primary anchors. Peptides corresponding to the HLA A3 supermotif-bearing sequences are then synthesized and tested for binding to HLA-A*0301 and HLA-A*1101 molecules, the molecules encoded by the two most prevalent A3-supertype alleles. The peptides that bind at least one of the two alleles with binding affinities of of less than 500 nM, often less than 200 nM, are then tested for binding cross-reactivity to the other common A3-supertype alleles (e.g., A*3101, A*3301, and A*6801) to identify those that can bind at least three of the five HLA-A3-supertype molecules tested.

The MAGE-A9 protein(s) scanned above is (are) also analyzed for the presence of 8-, 9-, 10-, or 11-mer peptides with the HLA-B7-supermotif. Corresponding peptides are synthesized and tested for binding to HLA-B*0702, the molecule encoded by the most common B7-supertype allele (i.e., the prototype B7 supertype allele). Peptides binding B*0702 with $IC_{50}$ of less than 500 nM are identified using standard methods. These peptides are then tested for binding to other common B7-supertype molecules (e.g., B*3501, B*5101, B*5301, and B*5401). Peptides capable of binding to three or more of the five B7-supertype alleles tested are thereby identified.

To further increase population coverage, HLA-A1 and -A24 epitopes can also be incorporated into vaccine compositions. An analysis of the MAGE-A9 protein can also be performed to identify HLA-A1- and A24-motif-containing sequences.

High affinity and/or cross-reactive binding epitopes that bear other motif and/or supermotifs are identified using analogous methodology.

EXAMPLE 8: Confirmation of immunogenicity

Cross-reactive candidate CTL A2-supermotif-bearing peptides that are identified as described herein are selected to confirm in vitro immunogenicity. Confirmation is performed using the following methodology: The 0.221A2.1 cell line, produced by transferring the HLA-A2.1 gene into the HLA-A, -B, -C null mutant human B-lymphoblastoid cell line 721.221, is used as the peptide-loaded target to measure activity of HLA-A2.1-restricted CTL. This cell line is grown in RPMI-1640 medium supplemented with antibiotics, sodium pyruvate, nonessential amino acids and 10% (v/v) heat inactivated FCS. Cells that express an antigen of interest, or transfectants comprising the gene encoding the antigen of interest, can be used as target cells to confirm the ability of peptide-specific CTLs to recognize endogenous antigen.

For generating dendritic cells (DC), PBMCs are thawed in RPMI with 30 $\mu$g/ml DNAse, washed twice and resuspended in complete medium (RPMI-1640 plus 5% AB human serum, non-essential amino acids, sodium pyruvate, L-glutamine and penicillin/streptomycin). The monocytes are purified by plating $10x10^6$ PBMC/well in a 6-well plate. After 2 hours at 37° C., the non-adherent cells are removed by gently shaking the plates and aspirating the supernatants. The wells are washed a total of three times with 3 ml RPMI to remove most of the non-adherent and loosely adherent cells. Three ml of complete medium containing 50 ng/ml of GM-CSF and 1,000 U/ml of IL-4 are then added to each well. TNF-alpha is added to the DCs on day 6 at 75 ng/ml and the cells are used for CTL induction cultures on day 7.

Induction of CTL with DC and Peptide: CD8+ T-cells are isolated by positive selection with Dynal immunomagnetic beads (Dynabeads™. M-450) and the detacha-bead™ reagent. Typically about $200\text{-}250x10^6$ PBMC are processed to obtain $24x10^6$ $CD8^+$ T-cells (enough for a 48-well plate culture). Briefly, the PBMCs are thawed in RPMI with 30 $\mu$g/ml DNAse, washed once with PBS containing 1% human AB serum and resuspended in PBS/1% AB serum at a concentration of $20x10^6$ cells/ml. The magnetic beads are washed 3 times with PBS/AB serum, added to the cells (140 $\mu$l beads/$20x10^6$ cells) and incubated for 1 hour at 4° C. with continuous mixing. The beads and cells are washed 4x with PBS/AB serum to remove the nonadherent cells and resuspended at $100x10^6$ cells/ml (based on the original cell number) in PBS/AB serum containing 100 μl/ml detacha-bead.RTM. reagent and 30 μg/ml DNAse. The mixture is incubated for 1 hour at room temperature with continuous mixing. The beads are washed again with PBS/AB/DNAse to collect the CD8+ T-cells. The DC are collected and centrifuged at 1300 rpm for 5-7 minutes, washed once with PBS with 1% BSA, counted and pulsed with 40 μg/ml of peptide at a cell concentration of 1-2x$10^6$/ml in the presence of 3 μg/ml $\beta_2$-microglobulin for 4 hours at 20° C. The DC are then irradiated (4,200 rads), washed 1 time with medium and counted again.

Setting up induction cultures: 0.25 ml cytokine-generated DC (at 1x$10^5$ cells/ml) are co-cultured with 0.25 ml of CD8+ T-cells (at 2x$10^6$ cell/ml) in each well of a 48-well plate in the presence of 10 ng/ml of IL-7. Recombinant human IL-10 is added the next day at a final concentration of 10 ng/ml and rhuman IL-2 is added 48 hours later at 10 IU/ml.

Re-stimulation of the induction cultures with peptide-pulsed adherent cells: Seven and fourteen days after the primary induction, the cells are re-stimulated with peptide-pulsed adherent cells. The PBMCs are thawed and washed twice with RPMI and DNAse. The cells are resuspended at 5x$10^6$ cells/ml and irradiated at about 4200 rads. The PBMCs are plated at 2x$10^6$ in 0.5 ml complete medium per well and incubated for 2 hours at 37° C. The plates are washed twice with RPMI by tapping the plate gently to remove the nonadherent cells and the adherent cells pulsed with 101 g/ml of peptide in the presence of 3 μg/ml $\beta_2$ microglobulin in 0.25 ml RPMI/5% AB per well for 2 hours at 37° C. Peptide solution from each well is aspirated and the wells are washed once with RPMI. Most of the media is aspirated from the induction cultures (CD8+ cells) and brought to 0.5 ml with fresh media. The cells are then transferred to the wells containing the peptide-pulsed adherent cells. Twenty four hours later recombinant human IL-10 is added at a final concentration of 10 ng/ml and recombinant human IL-2 is added the next day and again 2-3 days later at 50 IU/ml. Seven days later, the cultures are assayed for CTL activity in a $^{51}$Cr release assay. In some experiments the cultures are assayed for peptide-specific recognition in the *in situ* IFN-gamma ELISA at the time of the second restimulation followed by assay of endogenous recognition 7 days later. After expansion, activity is measured in both assays for a side-by-side comparison.

Seven days after the second restimulation, cytotoxicity is determined in a standard (5 hr) $^{51}$Cr release assay by assaying individual wells at a single E:T. Peptide-pulsed targets are prepared by incubating the cells with 10 μg/ml peptide overnight at 37° C.

Adherent target cells are removed from culture flasks with trypsin-EDTA. Target cells are labeled with 200 μCi of $^{51}$Cr sodium chromate (Dupont, Wilmington, Del.) for 1 hour at 37° C. Labeled target cells are resuspended at $10^6$ per ml and diluted 1:10 with K562 cells at a concentration of 3.3x$10^6$/mm (an NK-sensitive erythroblastoma cell line used to reduce non-specific lysis). Target cells (100 μl) and effectors (100 μl) are plated in 96 well round-bottom plates and incubated for 5 hours at 37° C. At that time, 100 μl of supernatant are collected from each well and percent lysis is determined according to the formula: [(cpm of the test sample-cpm of the spontaneous $^{51}$Cr release sample)/(cpm of the maximal $^{51}$Cr release sample-cpm of the spontaneous $^{51}$Cr release sample)]x100.

Maximum and spontaneous release are determined by incubating the labeled targets with 1% Triton X-100 and media alone, respectively. A positive culture is defined as one in which the specific lysis (sample-background) is 10% or higher in the case of individual wells and is 15% or more at the two highest E:T ratios when expanded cultures are assayed.

For *in situ* measurement of human IFN-gamma, Immulon™ 2 plates are coated with mouse anti-human IFN-gamma monoclonal antibody (4 μg/ml 0.1 M $NaHCO_3$, pH8.2) overnight at 4° C. The plates are washed with $Ca^{2+}$, $Mg^{2+}$-free PBS/0.05% Tween 20 and blocked with PBS/10% FCS for two hours, after which the CTLs (100 μl/well) and targets (100 μl/well) are added to each well, leaving empty wells for the standards and blanks (which received media only). The target cells, either peptide-pulsed or endogenous targets, are used at a concentration of 1x$10^6$ cells/ml. The plates are incubated for 48 hours at 37° C. with 5% $CO_2$.

Recombinant human IFN-gamma is added to the standard wells starting at 400 pg or 1200 pg/100 microliter/well and the plate incubated for two hours at 37° C. The plates are washed and 100 μl of biotinylated mouse anti-human IFN-gamma monoclonal antibody (2 microgram/ml in PBS/3% FCS/0.05% Tween 20) are added and incubated for 2 hours at room temperature. After washing again, 100 microliter HRP-streptavidin (1:4000) are added and the plates incubated for one hour at room temperature. The plates are then washed 6x with wash buffer, 100 microliter/well developing solution (TMB 1:1) are added, and the plates allowed to develop for 5-15 minutes. The reaction is stopped with 50 microliter/well 1 M $H_3PO_4$ and read at OD450. A culture is considered positive if it measured at least 50 pg of IFN-gamma/well above background and is twice the background level of expression.

Those cultures that demonstrate specific lytic activity against peptide-pulsed targets and/or tumor targets are expanded over a two week period with anti-CD3. Briefly, 5x$10^4$ CD8+ cells are added to a T25 flask containing the following: 1x$10^6$ irradiated (4,200 rad) PBMC (autologous or allogeneic) per ml, 2x$10^5$ irradiated (8,000 rad) EBV-transformed cells per ml, and OKT3 (anti-CD3) at 30 ng per ml in RPMI-1640 containing 10% (v/v) human AB serum, non-essential amino acids, sodium pyruvate, 2 5 μM 2-mercaptoethanol, L-glutamine and penicillin/streptomycin. Recombinant human IL2 is added 24 hours later at a final concentration of 200 IU/ml and every three days thereafter with fresh media at 50 IU/ml. The cells are split if the cell concentration exceeds 1x$10^6$/ml and the cultures are assayed between days 13 and 15 at E:T ratios of 30, 10, 3 and 1:1 in the $^{51}$Cr release assay or at 1x$10^6$/ml in the in situ IFN-gamma assay using the same targets as before the expansion.

Cultures are expanded in the absence of anti-CD3+ as follows. Those cultures that demonstrate specific lytic activity against peptide and endogenous targets are selected and $5x10^4$ $CD8^+$ cells are added to a T25 flask containing the following: $1x10^6$ autologous PBMC per ml which have been peptide-pulsed with 10 μg/ml peptide for two hours at 37° C. and irradiated (4,200 rad); $2x10^5$ irradiated (8,000 rad) EBV-transformed cells per ml RPMI-1640 containing 10% (v/v) human AB serum, non-essential AA, sodium pyruvate, 25 mM 2-ME, L-glutamine and gentamicin.

A2-supermotif cross-reactive binding peptides are tested in the cellular assay for the ability to induce peptide-specific CTL in normal individuals. In this analysis, a peptide is typically considered to be an epitope if it induces peptide-specific CTLs in at least individuals, and preferably, also recognizes the endogenously expressed peptide.

Immunogenicity can also be confirmed using PBMCs isolated from patients bearing a tumor that expresses MAGE-A9. Briefly, PBMCs are isolated from patients, re-stimulated with peptide-pulsed monocytes and assayed for the ability to recognize peptide-pulsed target cells as well as transfected cells endogenously expressing the antigen.

HLA-A3 supermotif-bearing cross-reactive binding peptides are also evaluated for immunogenicity using methodology analogous for that used to evaluate the immunogenicity of the HLA-A2 supermotif peptides.

Immunogenicity screening of the B7-supertype cross-reactive binding peptides identified as set forth herein are confirmed in a manner analogous to the confirmation of A2- and A3-supermotif-bearing peptides.

Peptides bearing other supermotifs/motifs, e.g., HLA-A1, HLA-A24 etc. are also confirmed using a similar methodology.

EXAMPLE 9 : Implementation of the extended supermotif to improve the binding capacity of native epitopes by creating analogs

HLA motifs and supermotifs (comprising primary and/or secondary residues) are useful in the identification and preparation of highly cross-reactive native peptides, as demonstrated herein. Moreover, the definition of HLA motifs and supermotifs also allows one to engineer highly cross-reactive epitopes by identifying residues within a native peptide sequence which can be analoged to confer upon the peptide certain characteristics, e.g. greater cross-reactivity within the group of HLA molecules that comprise a supertype, and/or greater binding affinity for some or all of those HLA molecules. Examples of analoging peptides to exhibit modulated binding affinity are set forth in this example.

For analoging at primary anchor residues, peptide engineering strategies are implemented to further increase the cross-reactivity of the epitopes. For example, the main anchors of A2-supermotif-bearing peptides are altered, for example, to introduce a preferred L, I, V, or M at position 2, and I or V at the C-terminus.

To analyze the cross-reactivity of the analog peptides, each engineered analog is initially tested for binding to the prototype A2 supertype allele A*0201, then, if A*0201 binding capacity is maintained, for A2-supertype cross-reactivity.

Alternatively, a peptide is confirmed as binding one or all supertype members and then analoged to modulate binding affinity to any one (or more) of the supertype members to add population coverage.

The selection of analogs for immunogenicity in a cellular screening analysis is typically further restricted by the capacity of the parent wild type (WT) peptide to bind at least weakly, i.e., bind at an $IC_{50}$ of 5000 nM or less, to three of more A2 supertype alleles. The rationale for this requirement is that the WT peptides must be present endogenously in sufficient quantity to be biologically relevant. Analoged peptides have been shown to have increased immunogenicity and cross-reactivity by T cells specific for the parent epitope.

In the cellular screening of these peptide analogs, it is important to confirm that analog-specific CTLs are also able to recognize the wild-type peptide and, when possible, target cells that endogenously express the epitope.

For analoging of HLA-A3 and B7 supermotif bearing peptides, analogs of HLA-A3 supermotif-bearing epitopes are generated using strategies similar to those employed in analoging HLA-A2 supermotif-bearing peptides (as described in Example 8). For example, peptides binding to 3/5 of the A3-supertype molecules are engineered at primary anchor residues to possess a preferred residue (V, S, M, or A) at position 2.

The analog peptides are then tested for the ability to bind A*03 and A*11 (prototype A3 supertype alleles). Those peptides that demonstrate less than 500 nM binding capacity are then confirmed as having A3-supertype cross-reactivity.

Similarly to the A2- and A3-motif bearing peptides, peptides binding 3 or more B7-supertype alleles can be improved, where possible, to achieve increased cross-reactive binding or greater binding affinity or binding half life. B7 supermotif-bearing peptides are, for example, engineered to possess a preferred residue (V, I, L, or F) at the C-terminal primary anchor position.

EXAMPLE 1 0 : Analoging at primary anchor residues of other motif and/or supermotif-bearing epitopes is performed in a like manner.

The analog peptides are then be confirmed for immunogenicity, typically in a cellular screening assay. Again, it is generally important to demonstrate that analog-specific CTLs are also able to recognize the wild-type peptide and, when possible, targets that endogenously express the epitope.

Moreover, HLA supermotifs are of value in engineering highly cross-reactive peptides and/or peptides that bind HLA molecules with increased affinity by identifying particular residues at secondary anchor positions that are associated with such properties. For example, the binding capacity of a B7 supermotif-bearing peptide with an F residue at position 1 is analyzed. The peptide is then analoged to, for example, substitute L for F at position 1. The analoged peptide is evaluated for increased binding affinity, binding half life and/or increased cross-reactivity. Such a procedure identifies analoged peptides with enhanced properties.

Engineered analogs with sufficiently improved binding capacity or cross-reactivity can also be tested for immunogenicity in HLA-B7-transgenic mice, following for example, IFA immunization or lipopeptide immunization. Analoged peptides are additionally tested for the ability to stimulate a recall response using PBMC from patients with MAGE-A9-expressing tumors.

Another form of peptide analoging, unrelated to anchor positions, involves the substitution of a cysteine with $\alpha$-amino butyric acid. Due to its chemical nature, cysteine has the propensity to form disulfide bridges and sufficiently alter the peptide structurally so as to reduce binding capacity. Substitution of $\alpha$-amino butyric acid for cysteine not only alleviates this problem, but has been shown to improve binding and crossbinding capabilities in some instances.

Thus, by the use of single amino acid substitutions, the binding properties and/or cross-reactivity of peptide ligands for HLA supertype molecules can be modulated.

EXAMPLE 11: Identification and confirmation of MAGE-A9-derived sequences with HLA-DR binding motifs

Peptide epitopes bearing an HLA class II supermotif or motif are identified and confirmed as outlined below using methodology similar to that described for HLA Class I peptides.

To identify MAGE-A9-derived, HLA class II HTL epitopes, a MAGE-A9 antigen is analyzed for the presence of sequences bearing an HLA-DR-motif or supermotif. Specifically, 15-mer sequences are selected comprising a DR-supermotif, comprising a 9-mer core, and three-residue N- and C-terminal flanking regions (15 amino acids total).

Protocols for predicting peptide binding to DR molecules have been developed (Southwood et al., J. Immunol. 160:3363-3373, 1998). These protocols, specific for individual DR molecules, allow the scoring, and ranking, of 9-mer core regions. Each protocol not only scores peptide sequences for the presence of DR-supermotif primary anchors (i.e., at position 1 and position 6) within a 9-mer core, but additionally evaluates sequences for the presence of secondary anchors. Using allele-specific selection tables (see, e.g., Southwood et al., ibid.), it has been found that these protocols efficiently select peptide sequences with a high probability of binding a particular DR molecule. Additionally, it has been found that performing these protocols in tandem, specifically those for DR1, DR4w4, and DR7, can efficiently select DR cross-reactive peptides.

The MAGE-A9-derived peptides identified above are tested for their binding capacity for various common HLA-DR molecules. All peptides are initially tested for binding to the DR molecules in the primary panel: DR1, DR4w4, and DR7. Peptides binding at least two of these three DR molecules are then tested for binding to DR2w2 $\beta$1, DR2w2 $\beta$2, DR6w19, and DR9 molecules in secondary assays. Finally, peptides binding at least two of the four secondary panel DR molecules, and thus cumulatively at least four of seven different DR molecules, are screened for binding to DR4w15, DR5w11, and DR8w2 molecules in tertiary assays. Peptides binding at least seven of the ten DR molecules comprising the primary, secondary, and tertiary screening assays are considered cross-reactive DR binders. MAGE-A9-derived peptides found to bind common HLA-DR alleles are of particular interest.

Because HLA-DR3 is an allele that is prevalent in Caucasian, Black, and Hispanic populations, DR3 binding capacity is a relevant criterion in the selection of HTL epitopes. Thus, peptides shown to be candidates may also be assayed for their DR3 binding capacity. However, in view of the binding specificity of the DR3 motif, peptides binding only to DR3 can also be considered as candidates for inclusion in a vaccine formulation.

To efficiently identify peptides that bind DR3, target MAGE-A9 antigens are analyzed for sequences carrying one of the two DR3-specific binding motifs reported by Geluk et al. (J. Immunol. 152:5742-5748, 1994). The corresponding peptides are then synthesized and confirmed as having the ability to bind DR3 with an affinity of 1 $\mu$M or better, i.e., less than 1 $\mu$M. Peptides are found that meet this binding criterion and qualify as HLA class II high affinity binders.

Similarly to the case of HLA class I motif-bearing peptides, the class II motif-bearing peptides are analoged to improve affinity or cross-reactivity. For example, aspartic acid at position 4 of the 9-mer core sequence is an optimal residue for DR3 binding, and substitution for that residue often improves DR 3 binding.

Example 12 : Immunogenicity of MAGE-A9-derived HTL epitopes Immunogenicity of HTL epitopes are confirmed in a manner analogous to the determination of immunogenicity of CTL epitopes, by assessing the ability to stimulate HTL responses and/or by using appropriate transgenic mouse models. Immunogenicity is determined by screening for: 1.) *in vitro* primary induction using normal PBMC or 2.) recall responses from patients who have MAGE-A9-expressing tumors.

Example 13 : Calculation of Phenotypic Frequencies of HLA-Supertypes in Various Ethnic Backgrounds to Determine Breadth of Population Coverage

In order to analyze population coverage, gene frequencies of HLA alleles are determined. Gene frequencies for each HLA allele are calculated from antigen or allele frequencies utilizing the binomial distribution formulae gf-1-(SQRT (1-af)) (see, e.g., Sidney et al., Human Immunol. 45:79-93, 1996). To obtain overall phenotypic frequencies, cumulative gene frequencies are calculated, and the cumulative antigen frequencies derived by the use of the inverse formula [af=1-(1-Cgf)$^2$].

Where frequency data is not available at the level of DNA typing, correspondence to the serologically defined antigen frequencies is assumed. To obtain total potential supertype population coverage no linkage disequilibrium is assumed, and only alleles confirmed to belong to each of the supertypes are included (minimal estimates). Estimates of total potential coverage achieved by inter-loci combinations are made by adding to the A coverage the proportion of the non-A covered population that could be expected to be covered by the B alleles considered (e.g., total=A+B*(1-A)). Confirmed members of the A3-like supertype are A3, A11, A31, A*3301, and A*6801. Although the A3-like supertype may also include A34, A66, and A*7401, these alleles were not included in overall frequency calculations. Likewise, confirmed members of the A2-like supertype family are A*0201, A*0202, A*0203, A*0204, A*0205, A*0206, A*0207, A*6802, and A*6901. Finally, the B7-like supertype-confirmed alleles are: B7, B*3501-03, B51, B*5301, B*5401, B*5501-2, B*5601, B*6701, and B*7801 (potentially also B*1401, B*3504-06, B*4201, and B*5602).

Population coverage achieved by combining the A2-, A3- and B7-supertypes is approximately 86% in five major ethnic groups. Coverage may be extended by including peptides bearing the A1 and A24 motifs. On average, A1 is present in 12% and A24 in 29% of the population across five different major ethnic groups (Caucasian, North American Black, Chinese, Japanese, and Hispanic). Together, these alleles are represented with an average frequency of 39% in these same ethnic populations. The total coverage across the major ethnicities when A1 and A24 are combined with the coverage of the A2-, A3- and B7-supertype alleles is >95%. An analogous approach can be used to estimate population coverage achieved with combinations of class II motif-bearing epitopes.

Immunogenicity studies in humans have shown that highly cross-reactive binding peptides are almost always recognized as epitopes. The use of highly cross-reactive binding peptides is an important selection criterion in identifying candidate epitopes for inclusion in a vaccine that is immunogenic in a diverse population.

With a sufficient number of epitopes (as disclosed herein and from the art), an average population coverage is predicted to be greater than 95% in each of five major ethnic populations. The Monte Carlo simulation analysis, which is known in the art, can be used to estimate what percentage of the individuals in a population comprised of the Caucasian, North American Black, Japanese, Chinese, and Hispanic ethnic groups would recognize the vaccine epitopes described herein. A preferred percentage is 90%. A more preferred percentage is 95%.

Example 14 : CTL Recognition of Endogenously Processed Antigens after Priming

Effector cells isolated from transgenic mice that are immunized with peptide epitopes, for example HLA-A2 supermotif-bearing epitopes, are re-stimulated in vitro using peptide-coated stimulator cells. Six days later, effector cells are assayed for cytotoxicity and the cell lines that contain peptide-specific cytotoxic activity are further re-stimulated. An additional six days later, these cell lines are tested for cytotoxic activity on $^{51}$Cr labeled Jurkat-A2.1/K$^b$ target cells in the absence or presence of peptide, and also tested on $^{51}$Cr labeled target cells bearing the endogenously synthesized antigen, i.e. cells that are stably transfected with MAGE-A9 expression vectors.

The results demonstrate that CTL lines obtained from animals primed with peptide epitope recognize endogenously synthesized MAGE-A9 antigen. The choice of transgenic mouse model to be used for such an analysis depends upon the epitope(s) that are being evaluated. In addition to HLA-A*0201/K$^b$ transgenic mice, several other transgenic mouse models including mice with human A11, which may also be used to evaluate A3 epitopes, and B7 alleles have been characterized and others (e.g., transgenic mice for HLA-A 1 and A24) are being developed. HLA-DR1 and HLA-DR3 mouse models have also been developed, which may be used to evaluate HTL epitopes.

EXAMPLE 15 : Activity of CTL-HTL Conjugated Epitopes in Transgenic Mice

This example illustrates the induction of CTLs and HTLs in transgenic mice, by use of a MAGE-A9-derived CTL and HTL peptide vaccine compositions. The vaccine composition used herein comprise peptides to be administered to a patient with a MAGE-A9-expressing tumor. The peptide composition can comprise multiple CTL and/or HTL epitopes. The epitopes are identified using methodology as described herein. This example also illustrates that enhanced immunogenicity can be achieved by inclusion of one or more HTL epitopes in a CTL vaccine composition; such a peptide composition can comprise an HTL epitope conjugated to a CTL epitope. The CTL epitope can be one that binds to multiple HLA family members at an affinity of 500 nM or less, or analogs of that epitope. The peptides may be lipidated, if desired.

Immunization of transgenic mice is performed according to methods known in the art. For example, A2/$K^b$ mice, which are transgenic for the human HLA A2.1 allele and are used to confirm the immunogenicity of HLA-A*0201 motif- or HLA-A2 supermotif-bearing epitopes, and are primed subcutaneously (base of the tail) with a 0.1 ml of peptide in Incomplete Freund's Adjuvant, or if the peptide composition is a lipidated CTL/HTL conjugate, in DMSO/saline, or if the peptide composition is a polypeptide, in PBS or Incomplete Freund's Adjuvant. Seven days after priming, splenocytes obtained from these animals are restimulated with syngenic irradiated LPS-activated lymphoblasts coated with peptide.

One week after priming, spleen cells ($30x10^6$ cells/flask) are co-cultured at 37° C. with syngeneic, irradiated (3000 rads), peptide coated lymphoblasts ($10x10^6$ cells/flask) in 10 ml of culture medium/T25 flask. After six days, effector cells are harvested and assayed for cytotoxic activity. Target cells (1.0 to $1.5x10^6$) are incubated at 37° C. in the presence of 200 µl of $^{51}Cr$. After 60 minutes, cells are washed three times and resuspended in R10 medium. Peptide is added where required at a concentration of 1 µg/ml. For the assay, $10^4$ 51Cr-labeled target cells are added to different concentrations of effector cells (final volume of 200 µl) in U-bottom 96-well plates. After a six hour incubation period at 37° C, a 0.1 ml aliquot of supernatant is removed from each well and radioactivity is determined in a Micromedic automatic gamma counter. The percent specific lysis is determined by the formula: percent specific release=100x(experimental release-spontaneous release)/(maximum release-spontaneous release). To facilitate comparison between separate CTL assays run under the same conditions, % $^{51}Cr$ release data is expressed as lytic units/$10^6$ cells. One lytic unit is arbitrarily defined as the number of effector cells required to achieve 30% lysis of 10,000 target cells in a six hour $^{51}Cr$ release assay. To obtain specific lytic units/$10^6$, the lytic units/$10^6$ obtained in the absence of peptide is subtracted from the lytic units/$10^6$ obtained in the presence of peptide. For example, if 30% $^{51}Cr$ release is obtained at the effector (E): target (T) ratio of 50:1 (i.e., $5x10^5$ effector cells for 10,000 targets) in the absence of peptide and 5:1 (i.e., $5x10^4$ effector cells for 10,000 targets) in the presence of peptide, the specific lytic units would be: $[(1/50{,}000)-(1/500{,}000)]x10^6=18$ LU.

The results are analyzed to assess the magnitude of the CTL responses of animals injected with the immunogenic CTL/HTL conjugate vaccine preparation and are compared to the magnitude of the CTL response achieved using, for example, CTL epitopes as outlined above in Example 8. Analyses similar to this may be performed to confirm the immunogenicity of peptide conjugates containing multiple CTL epitopes and/or multiple HTL epitopes. In accordance with these procedures, it is found that a CTL response is induced, and concomitantly that an HTL response is induced upon administration of such compositions.

EXAMPLE 16 : Selection of CTL and HTL epitopes for inclusion in a MAGE-A9-specific vaccine.

This example illustrates a procedure for selecting peptide epitopes for vaccine compositions of the invention. The peptides in the composition can be in the form of a nucleic acid sequence, either single or one or more sequences (i.e., minigene) that encodes peptide(s), or can be single and/or polyepitopic peptides.

Epitopes are selected which, upon administration, mimic immune responses that are correlated with MAGE-A9 clearance. The number of epitopes used depends on observations of patients who spontaneously dear MAGE-A9. For example, if it has been observed that patients who spontaneously dear MAGE-A9-expressing cells generate an immune response to at least three (3) epitopes from MAGE-A9 antigen, then at least three epitopes should be included for HLA class I. A similar rationale is used to determine HLA class II epitopes.

Epitopes are often selected that have a binding affinity of an $IC_{50}$ of 500 nM or less for an HLA class I molecule, or for class II, an $IC_{50}$ of 1000 nM or less; or HLA Class I peptides with high binding scores from the BIMAS web site, at URL bimas.dcrt.nih.gov/ (Table 6).

In order to achieve broad coverage of the vaccine through out a diverse population, sufficient supermotif bearing peptides, or a sufficient array of allele-specific motif bearing peptides, are selected to give broad population coverage. In one embodiment, epitopes are selected to provide at least 80% population coverage. A Monte Carlo analysis, a statistical evaluation known in the art, can be employed to assess breadth, or redundancy, of population coverage.

When creating polyepitopic compositions, or a minigene that encodes same, it is typically desirable to generate the smallest peptide possible that encompasses the epitopes of interest. The principles employed are similar, if not the same, as those employed when selecting a peptide comprising nested epitopes. For example, a protein sequence for the vaccine composition is selected because it has maximal number of epitopes contained within the sequence, i.e., it has a high concentration of epitopes. Epitopes may be nested or overlapping (i.e., frame shifted relative to one another). For example, with overlapping epitopes, two 9-mer epitopes and one 10-mer epitope can be present in a 10 amino acid peptide. Each epitope can be exposed and bound by an HLA molecule upon administration of such a peptide. A multi-epitopic, peptide can be generated synthetically, recombinantly, or via cleavage from the native source. Alternatively, an analog can be made of this native sequence, whereby one or more of the epitopes comprise substitutions that alter the cross-reactivity and/or binding affinity properties of the polyepitopic peptide. Such a vaccine composition is administered for therapeutic or prophylactic purposes. This embodiment provides for the possibility that an as yet undiscovered aspect of immune system processing will apply to the native nested sequence and thereby facilitate the production of therapeutic or prophylactic immune response-inducing vaccine compositions. Additionally such an embodiment provides for the possibility of motif-bearing epitopes for an HLA makeup that is presently unknown. Furthermore, this embodiment (absent the creating of any analogs) directs the immune response to multiple peptide sequences that are actually present in MAGE-A9, thus avoiding the need to evaluate any junctional epitopes. Lastly, the embodiment provides an economy of scale when producing nucleic acid vaccine compositions. Related to this embodiment, computer programs can be derived in accordance with principles in the art, which identify in a target sequence, the greatest number of epitopes per sequence length.

EXAMPLE 17 : Construction of "minigene" multi-epitope DNA plasmids The minigene DNA plasmid of this example contains a consensus Kozak sequence and a consensus murine kappa Ig-light chain signal sequence followed by CTL and/or HTL epitopes selected in accordance with principles disclosed herein. The sequence encodes an open reading frame fused to the Myc and His antibody epitope tag coded for by the pcDNA 3.1 Myc-His vector.

Overlapping oligonucleotides that can, for example, average about 70 nucleotides in length with 15 nucleotide overlaps, are synthesized and HPLC-purified. The oligonucleotides encode the selected peptide epitopes as well as appropriate linker nucleotides, Kozak sequence, and signal sequence. The final multiepitope minigene is assembled by extending the overlapping oligonucleotides in three sets of reactions using PCR. A Perkin/Elmer 9600 PCR machine is used and a total of 30 cycles are performed using the following conditions: 95° C. for 15 sec, annealing temperature (5° below the lowest calculated Tm of each primer pair) for 30 sec, and 72° C. for 1 min.

For example, a minigene is prepared as follows. For a first PCR reaction, 5 $\mu$g of each of two oligonucleotides are annealed and extended: In an example using eight oligonucleotides, i.e., four pairs of primers, oligonucleotides 1+2, 3+4, 5+6, and 7+8 are combined in 100 $\mu$l reactions containing Pfu polymerase buffer (1x=10 mM KCL, 10 mM $(NH4)_2SO_4$, 20 mM Tris-chloride, pH 8.75, 2 mM $MgSO_4$, 0.1% Triton X-100, 100 $\mu$g/ml BSA), 0.25 mM each dNTP, and 2.5 U of Pfu polymerase. The full-length dimer products are gel-purified, and two reactions containing the product of 1+2 and 3+4, and the product of 5+6 and 7+8 are mixed, annealed, and extended for 10 cycles. Half of the two reactions are then mixed, and 5 cycles of annealing and extension carried out before flanking primers are added to amplify the full length product. The full-length product is gel-purified and cloned into pCR-blunt (Invitrogen) and individual clones are screened by sequencing.

EXAMPLE 18 : The plasmid construct and the degree to which it induces immunogenicity

The degree to which a plasmid construct, for example a plasmid constructed in accordance with the previous Example, is able to induce immunogenicity is confirmed *in vitro* by determining epitope presentation by APC following transduction or transfection of the APC with an epitope-expressing nucleic acid construct. Such a study determines "antigenicity" and allows the use of human APC. The assay determines the ability of the epitope to be presented by the APC in a context that is recognized by a T cell by quantifying the density of epitope-HLA class I complexes on the cell surface. Quantitation can be performed by directly measuring the amount of peptide eluted from the APC (see, e.g., Sijts et al., J. Immunol. 156:683-692, 1996; Demotz et al., Nature 342:682-684, 1989); or the number of peptide-HLA class I complexes can be estimated by measuring the amount of lysis or lymphokine release induced by diseased or transfected target cells, and then determining the concentration of peptide necessary to obtain equivalent levels of lysis or lymphokine release (see, e.g., Kageyama et al., J. Immunol. 154:567-576, 1995).

Alternatively, immunogenicity is confirmed through *in vivo* injections into mice and subsequent *in vitro* assessment of CTL and HTL activity, which are analyzed using cytotoxicity and proliferation assays, respectively, as detailed e.g., in Alexander et al., Immunity 1:751-761, 1994.

For example, to confirm the capacity of a DNA minigene construct containing at least one HLA-A2 supermotif peptide to induce CTLs *in vivo,* HLA-A2.1/$K^b$ transgenic mice are immunized intramuscularly with 100 $\mu$g of naked cDNA. As a means of comparing the level of CTLs induced by cDNA immunization, a control group of animals is also immunized with an actual peptide composition that comprises multiple epitopes synthesized as a single polypeptide as they would be encoded by the minigene.

Splenocytes from immunized animals are stimulated twice with each of the respective compositions (peptide epitopes encoded in the minigene or the polyepitopic peptide), then assayed for peptide-specific cytotoxic activity in a $^{51}$Cr release assay. The results indicate the magnitude of the CTL response directed against the A2-restricted epitope, thus indicating the *in vivo* immunogenicity of the minigene vaccine and polyepitopic vaccine.

It is, therefore, found that the minigene elicits immune responses directed toward the HLA-A2 supermotif peptide epitopes as does the polyepitopic peptide vaccine. A similar analysis is also performed using other HLA-A3 and HLA-B7 transgenic mouse models to assess CTL induction by HLA-A3 and HLA-B7 motif or supermotif epitopes, whereby it is also found that the minigene elicits appropriate immune responses directed toward the provided epitopes.

DNA minigenes, constructed as described in the previous Example, can also be confirmed as a vaccine in combination with a boosting agent using a prime boost protocol. The boosting agent can consist of recombinant protein or recombinant vaccinia, for example, expressing a minigene or DNA encoding the complete protein of interest.

For example, the efficacy of the DNA minigene used in a prime boost protocol is initially evaluated in transgenic mice. In this example, A2.1/K.sup.b transgenic mice are immunized IM with 100 μg of a DNA minigene encoding the immunogenic peptides including at least one HLA-A2 supermotif-bearing peptide. After an incubation period (ranging from 3-9 weeks), the mice are boosted IP with $10^7$ pfu/mouse of a recombinant vaccinia virus expressing the same sequence encoded by the DNA minigene. Control mice are immunized with 100 μg of DNA or recombinant vaccinia without the minigene sequence, or with DNA encoding the minigene, but without the vaccinia boost. After an additional incubation period of two weeks, splenocytes from the mice are immediately assayed for peptide-specific activity in an ELISPOT assay.

EXAMPLE 19 : Peptide compositions for prophylactic uses

For example, a peptide-based composition is provided as a single polypeptide that encompasses multiple epitopes. The vaccine is typically administered in a physiological solution that comprises an adjuvant, such as Incomplete Freunds Adjuvant. The dose of peptide for the initial immunization is from about 1 to about 50,000 μg, generally 100-5,000 μg, for a 70 kg patient. The initial administration of vaccine is followed by booster dosages at 4 weeks followed by evaluation of the magnitude of the immune response in the patient, by techniques that determine the presence of epitope-specific CTL populations in a PBMC sample. Additional booster doses are administered as required. The composition is found to be both safe and efficacious as a prophylaxis against MAGE-A9-associated disease.

Alternatively, a composition typically comprising transfecting agents is used for the administration of a nucleic acid-based vaccine in accordance with methodologies known in the art and disclosed herein.

EXAMPLE 20 : Polyepitopic vaccine compositions derived from native MAGE-A9 sequences

A native MAGE-A9 polyprotein sequence is analyzed, preferably using computer algorithms defined for each class I and/or class II supermotif or motif, to identify "relatively short" regions of the polyprotein that comprise multiple epitopes. The "relatively short" regions are preferably less in length than an entire native antigen. This relatively short sequence that contains multiple distinct or overlapping, "nested" epitopes can be used to generate a minigene construct. The construct is engineered to express the peptide, which corresponds to the native protein sequence. The "relatively short" peptide is generally less than 250 amino acids in length, often less than 100 amino acids in length, preferably less than 75 amino acids in length, and more preferably less than 50 amino acids in length. The protein sequence of the vaccine composition is selected because it has maximal number of epitopes contained within the sequence, i.e., it has a high concentration of epitopes. As noted herein, epitope motifs may be nested or overlapping (i.e., frame shifted relative to one another). For example, with overlapping epitopes, two 9-mer epitopes and one 10-mer epitope can be present in a 10 amino acid peptide. Such a vaccine composition is administered for therapeutic or prophylactic purposes.

The vaccine composition will include, for example, multiple CTL epitopes from MAGE-A9 antigen and at least one HTL epitope. This polyepitopic native sequence is administered either as a peptide or as a nucleic acid sequence which encodes the peptide. Alternatively, an analog can be made of this native sequence, whereby one or more of the epitopes comprise substitutions that alter the cross-reactivity and/or binding affinity properties of the polyepitopic peptide.

The embodiment of this example provides for the possibility that an as yet undiscovered aspect of immune system processing will apply to the native nested sequence and thereby facilitate the production of therapeutic or prophylactic immune response-inducing vaccine compositions. Additionally, such an embodiment provides for the possibility of motif-bearing epitopes for an HLA makeup(s) that is presently unknown. Furthermore, this embodiment (excluding an analoged embodiment) directs the immune response to multiple peptide sequences that are actually present in native MAGE-A9, thus avoiding the need to evaluate any junctional epitopes. Lastly, the embodiment provides an economy of scale when producing peptide or nucleic acid vaccine compositions.

Related to this embodiment, computer programs are available in the art which can be used to identify in a target sequence, the greatest number of epitopes per sequence length.

EXAMPLE 21 : Polyepitopic vaccine compositions from multiple antigens

The MAGE-A9 peptide epitopes of the present invention are used in conjunction with epitopes from other target tumor-associated antigens, to create a vaccine composition that is useful for the prevention or treatment of cancer that expresses MAGE-A9 and such other antigens. For example, a vaccine composition can be provided as a single polypeptide that incorporates multiple epitopes from MAGE-A9 as well as tumor-associated antigens that are often expressed with a target cancer associated with MAGE-A9 expression, or can be administered as a composition comprising a cocktail of one or more discrete epitopes. Alternatively, the vaccine can be administered as a minigene construct or as dendritic cells which have been loaded with the peptide epitopes *in vitro*.

EXAMPLE 22 : Use of peptides to evaluate an immune response

Peptides of the invention may be used to analyze an immune response for the presence of specific antibodies, CTL or HTL directed to MAGE-A9. Such an analysis can be performed in a manner described by Ogg et al., Science 279:2103-2106, 1998. In this Example, peptides in accordance with the invention are used as a reagent for diagnostic or prognostic purposes, not as an immunogen.

In this example highly sensitive human leukocyte antigen tetrameric complexes ("tetramers") are used for a cross-sectional analysis of, for example, MAGE-A9 HLA-A*0201-specific CTL frequencies from HLA A*0201-positive individuals at different stages of disease or following immunization comprising a MAGE-A9 peptide containing an A*0201 motif. Tetrameric complexes are synthesized according to methods known in the art. Briefly, purified HLA heavy chain (A*0201 in this example) and β2-microglobulin are synthesized by means of a prokaryotic expression system. The heavy chain is modified by deletion of the transmembrane-cytosolic tail and COOH-terminal addition of a sequence containing a BirA enzymatic biotinylation site. The heavy chain, β2-microglobulin, and peptide are refolded by dilution. The 45-kD refolded product is isolated by fast protein liquid chromatography and then biotinylated by BirA in the presence of biotin (Sigma, St. Louis, Mo.), adenosine 5' triphosphate and magnesium. Streptavidin-phycoerythrin conjugate is added in a 1:4 molar ratio, and the tetrameric product is concentrated to 1 mg/ml. The resulting product is referred to as tetramer-phycoerythrin.

For the analysis of patient blood samples, approximately one million PBMCs are centrifuged at 300 g for 5 minutes and resuspended in 50 μl of cold phosphate-buffered saline. Tri-color analysis is performed with the tetramer-phycoerythrin, along with anti-CD8-Tricolor, and anti-CD38. The PBMCs are incubated with tetramer and antibodies on ice for 30 to 60 min and then washed twice before formaldehyde fixation. Gates are applied to contain >99.98% of control samples. Controls for the tetramers include both A*0201-negative individuals and A*0201-positive non-diseased donors. The percentage of cells stained with the tetramer is then determined by flow cytometry. The results indicate the number of cells in the PBMC sample that contain epitope-restricted CTLs, thereby readily indicating the extent of immune response to the MAGE-A9 epitope, and thus the status of exposure to MAGE-A9, or exposure to a vaccine that elicits a protective or therapeutic response.

EXAMPLE 23 : Use of peptide epitopes to evaluate recall responses

The peptide epitopes of the invention are used as reagents to evaluate T cell responses, such as acute or recall responses, in patients. Such an analysis may be performed on patients who have recovered from MAGE-A9-associated disease or who have been vaccinated with a MAGE-A9 vaccine.

For example, the class I restricted CTL response of persons who have been vaccinated may be analyzed. The vaccine may be any MAGE-A9 vaccine. PBMC are collected from vaccinated individuals and HLA typed. Appropriate peptide epitopes of the invention that, optimally, bear supermotifs to provide cross-reactivity with multiple HLA supertype family members, are then used for analysis of samples derived from individuals who bear that HLA type.

PBMC from vaccinated individuals are separated on Ficoll-Histopaque density gradients (Sigma Chemical Co., St. Louis, Mo.), washed three times in HBSS (GIBCO Laboratories), resuspended in RPMI-1640 (GIBCO Laboratories) supplemented with L-glutamine (2 mM), penicillin (50 U/ml), streptomycin (50 μg/ml), and Hepes (10 mM) containing 10% heat-inactivated human AB serum (complete RPMI) and plated using microculture formats. A synthetic peptide comprising an epitope of the invention is added at 10 μg/ml to each well and HBV core 128-140 epitope is added at 1 μg/ml to each well as a source of T cell help during the first week of stimulation.

In the microculture format, $4x10^5$ PBMC are stimulated with peptide in 8 replicate cultures in 96-well round bottom plate in 100 μl/well of complete RPMI. On days 3 and 10, 100 μl of complete RPMI and 20 U/ml final concentration of rIL-2 are added to each well. On day 7 the cultures are transferred into a 96-well flat-bottom plate and restimulated with peptide, rIL-2 and $10^5$ irradiated (3,000 rad) autologous feeder cells. The cultures are tested for cytotoxic activity on day 14. A positive CTL response requires two or more of the eight replicate cultures to display greater than 10% specific $^{51}Cr$ release, based on comparison with non-diseased control subjects.

Cytotoxicity assays are performed in the following manner. Target cells consist of either allogeneic HLA-matched or autologous EBV-transformed B lymphoblastoid cell line that are incubated overnight with the synthetic peptide epitope of the invention at 10 μM, and labeled with 100 μCi of $^{51}Cr$ (Amersham Corp., Arlington Heights, III.) for 1 hour after which they are washed four times with HBSS.

Cytolytic activity is determined in a standard 4-h, split well $^{51}Cr$ release assay using U-bottomed 96 well plates containing 3,000 targets/well. Stimulated PBMC are tested at effector/target (E/T) ratios of 20-50:1 on day 14. Percent cytotoxicity is determined from the formula: 100x[(experimental release-spontaneous release)/maximum release-spontaneous release)]. Maximum release is determined by lysis of targets by detergent (2% Triton X-100; Sigma Chemical Co., St. Louis, Mo.). Spontaneous release is <25% of maximum release for all experiments.

The results of such an analysis indicate the extent to which HLA-restricted CTL populations have been stimulated by previous exposure to MAGE-A9 or a MAGE-A9 vaccine.

Similarly, Class II restricted HTL responses may also be analyzed. Purified PBMC are cultured in a 96-well flat bottom plate at a density of $1.5x10^5$ cells/well and are stimulated with 10 μg/ml synthetic peptide of the invention, whole MAGE-A9 antigen, or PHA. Cells are routinely plated in replicates of 4-6 wells for each condition. After seven days of culture, the medium is removed and replaced with fresh medium containing 10 U/ml IL-2. Two days later, 1 μCi $^3$H-thymidine is added to each well and incubation is continued for an additional 18 hours. Cellular DNA is then harvested on glass fiber mats and analyzed for $^3$H-thymidine incorporation. Antigen-specific T cell proliferation is calculated as the ratio of $^3$H-thymidine incorporation in the presence of antigen divided by the $^3$H-thymidine incorporation in the absence of antigen.

EXAMPLE 24 : Induction of specific CTL response in humans

A human clinical trial for an immunogenic composition comprising CTL and HTL epitopes of the invention is set up as an IND Phase I, dose escalation study and carried out as a randomized, double-blind, placebo-controlled trial. Such a trial is designed, for example, as follows:

A total of about 27 individuals are enrolled and divided into 3 groups:

Group I: 3 subjects are injected with placebo and 6 subjects are injected with 5 μg of peptide composition;

Group II: 3 subjects are injected with placebo and 6 subjects are injected with 50 μg peptide composition;

Group III: 3 subjects are injected with placebo and 6 subjects are injected with 500 μg of peptide composition.

After 4 weeks following the first injection, all subjects receive a booster inoculation at the same dosage.

The endpoints measured in this study relate to the safety and tolerability of the peptide composition as well as its immunogenicity. Cellular immune responses to the peptide composition are an index of the intrinsic activity of this the peptide composition, and can therefore be viewed as a measure of biological efficacy. The following summarize the clinical and laboratory data that relate to safety and efficacy endpoints.

Safety: The incidence of adverse events is monitored in the placebo and drug treatment group and assessed in terms of degree and reversibility.

Evaluation of Vaccine Efficacy: For evaluation of vaccine efficacy, subjects are bled before and after injection. Peripheral blood mononuclear cells are isolated from fresh heparinized blood by Ficoll-Hypaque density gradient centrifugation, aliquoted in freezing media and stored frozen. Samples are assayed for CTL and HTL activity.

The vaccine is found to be both safe and efficacious.

EXAMPLE 25 : Phase II trials in patients expressing MAGE-A9

Phase II trials are performed to study the effect of administering the CTL-HTL peptide compositions to patients having cancer that expresses MAGE-A9. The main objectives of the trial are to determine an effective dose and regimen for inducing CTLs in cancer patients that express MAGE-A9, to establish the safety of inducing a CTL and HTL response in these patients, and to see to what extent activation of CTLs improves the clinical picture of these patients, as manifested, e.g., by the reduction and/or shrinking of lesions. Such a study is designed, for example, as follows:

The studies are performed in multiple centers. The trial design is an open-label, uncontrolled, dose escalation protocol wherein the peptide composition is administered as a single dose followed six weeks later by a single booster shot of the same dose. The dosages are 50, 500 and 5,000 micrograms per injection. Drug-associated adverse effects (severity and reversibility) are recorded.

There are three patient groupings. The first group is injected with 50 micrograms of the peptide composition and the second and third groups with 500 and 5,000 micrograms of peptide composition, respectively. The patients within each group range in age from 21-65 and represent diverse ethnic backgrounds. All of them have a tumor that expresses MAGE-A9.

Clinical manifestations or antigen-specific T-cell responses are monitored to assess the effects of administering the peptide compositions. The vaccine composition is found to be both safe and efficacious in the treatment of MAGE-A9-associated disease.

EXAMPLE 26 : Administration of vaccine compositions using dendritic cells (DC)

Vaccines comprising peptide epitopes of the invention can be administered using APCs, or "professional" APCs such as DC. In this example, peptide-pulsed DC are administered to a patient to stimulate a CTL response *in vivo*. In this method, dendritic cells are isolated, expanded, and pulsed with a vaccine comprising peptide CTL and HTL epitopes of the invention. The dendritic cells are infused back into the patient to elicit CTL and HTL responses *in vivo*. The induced CTL and HTL then destroy or facilitate destruction, respectively, of the target cells that bear the MAGE-A9 protein from which the epitopes in the vaccine are derived.

For example, a cocktail of epitope-comprising peptides is administered *ex vivo* to PBMC, or isolated DC therefrom. A pharmaceutical to facilitate harvesting of DC can be used, such as Progenipoietin™ (Monsanto, St. Louis, Mo.) or GM-CSF/IL4. After pulsing the DC with peptides, and prior to reinfusion into patients, the DC are washed to remove unbound peptides.

As appreciated clinically, and readily determined by one of skill based on clinical outcomes, the number of DC reinfused into the patient can vary. Although 2-50x$10^6$ DC per patient are typically administered, larger number of DC, such as $10^7$ or $10^8$ can also be provided. Such cell populations typically contain between 50-90% DC.

In some embodiments, peptide-loaded PBMC are injected into patients without purification of the DC. For example, PBMC generated after treatment with an agent such as Progenipoietin™ are injected into patients without purification of the DC. The total number of PBMC that are administered often ranges from $10^8$ to $10^{10}$. Generally, the cell doses injected into patients is based on the percentage of DC in the blood of each patient, as determined, for example, by immunofluorescence analysis with specific anti-DC antibodies. Thus, for example, if Progenipoietin™ mobilizes 2% DC in the peripheral blood of a given patient, and that patient is to receive 5x$10^6$ DC, then the patient will be injected with a total of 2.5x$10^8$ peptide-loaded PBMC. The percent DC mobilized by an agent such as Progenipoietin™ is typically estimated to be between 2-10%, but can vary as appreciated by one of skill in the art.

Alternatively, *ex vivo* CTL or HTL responses to MAGE-A9 antigens can be induced by incubating, in tissue culture, the patient's, or genetically compatible, CTL or HTL precursor cells together with a source of APC, such as DC, and immunogenic peptides. After an appropriate incubation time (typically about 7-28 days), in which the precursor cells are activated and expanded into effector cells, the cells are infused into the patient, where they will destroy (CTL) or facilitate destruction (HTL) of their specific target cells, i.e., tumor cells.

EXAMPLE 27 : An alternative method of identifying and confirming motif-bearing peptides

Another method of identifying and confirming motif-bearing peptides is to elute them from cells bearing defined MHC molecules. For example, EBV transformed B cell lines used for tissue typing have been extensively characterized to determine which HLA molecules they express. In certain cases these cells express only a single type of HLA molecule. These cells can be transfected with nucleic acids that express the antigen of interest, e.g. MAGE-A9. Peptides produced by endogenous antigen processing of peptides produced as a result of transfection will then bind to HLA molecules within the cell and be transported and displayed on the cell's surface. Peptides are then eluted from the HLA molecules by exposure to mild acid conditions and their amino acid sequence determined, e.g., by mass spectral analysis. Because the majority of peptides that bind a particular HLA molecule are motif-bearing, this is an alternative modality for obtaining the motif-bearing peptides correlated with the particular HLA molecule expressed on the cell.

Alternatively, cell lines that do not express endogenous HLA molecules can be transfected with an expression construct encoding a single HLA allele. These cells can then be used as described, i.e., they can then be transfected with nucleic acids that encode MAGE-A9 to isolate peptides corresponding to MAGE-A9 that have been presented on the cell surface. Peptides obtained from such an analysis will bear motif(s) that correspond to binding to the single HLA allele that is expressed in the cell.

As appreciated by one in the art, one can perform a similar analysis on a cell bearing more than one HLA allele and subsequently determine peptides specific for each HLA allele expressed. Moreover, one of skill would also recognize that means other than transfection, such as loading with a protein antigen, can be used to provide a source of antigen to the cell.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

**Claims**

1. Medicament comprising a MAGE-A9 gene product for the prevention or treatment of a MAGE-A9-associated bladder cancer in a subject.

2. The medicament of claim 1, wherein said MAGE-A9 gene product is a MAGE-A9-encoding polynucleic acid selected from the group consisting of genomic DNA, cDNA, mRNA, variants thereof and fragments thereof.

3. The medicament of claim 2, wherein said MAGE-A9-encoding polynucleic acid comprises the nucleic acid sequence of SEQ ID NO:1 or a fragment thereof.

4. The medicament of claim 1, wherein said MAGE-A9 gene product is selected from the group consisting of a MAGE-A9 polypeptide, a MAGE-A9 polypeptide variant and a MAGE-A9 polypeptide fragment.

5. The medicament of claim 4, wherein said MAGE-A9 polypeptide comprises the amino acid sequence of SEQ ID NO:2 or a fragment thereof.

6. The medicament of claim 1, wherein said subject is human.

7. The medicament of claim 1, wherein said medicament is a vaccine.

8. The medicament of claim 7, wherein said vaccine comprises antigen presenting cells transformed *ex vivo* with said MAGE-A9-encoding polynucleic acid or pulsed *ex vivo* with said MAGE-A9 polypeptide.

**9.** A MAGE-A9 gene product for its use in the prevention or treatment of a MAGE-A9-associated bladder cancer in a subject.

**10.** The MAGE-A9 product of claim 9, wherein said MAGE-A9 gene product is a MAGE-A9-encoding polynucleic acid selected from the group consisting of genomic DNA, cDNA, mRNA, variants thereof and fragments thereof.

**11.** The MAGE-A9 product of claim 10, wherein said MAGE-A9-encoding polynucleic acid comprises the nucleic acid sequence of SEQ ID NO:1 or a fragment thereof.

**12.** The MAGE-A9 product of claim 9, wherein said MAGE-A9 gene product is selected from the group consisting of MAGE-A9 polypeptide, a MAGE-A9 polypeptide variant and a MAGE-A9 polypeptide fragment.

**13.** The MAGE-A9 product of claim 12, wherein said MAGE-A9 polypeptide comprises the amino acid sequence of SEQ ID NO:2 or a fragment thereof.

**14.** The MAGE-A9 product of claim 9, wherein said subject is human.

**15.** The use of any one of claims 3, 5, 11 or 13, wherein said MAGE-A9-encoding polynucleic acid has at least 90% identity with the polynucleic acid sequence of SEQ ID NO:1, or wherein said MAGE-A9 polypeptide has at least 75% identity with the amino acid sequence of SEQ ID NO:2.

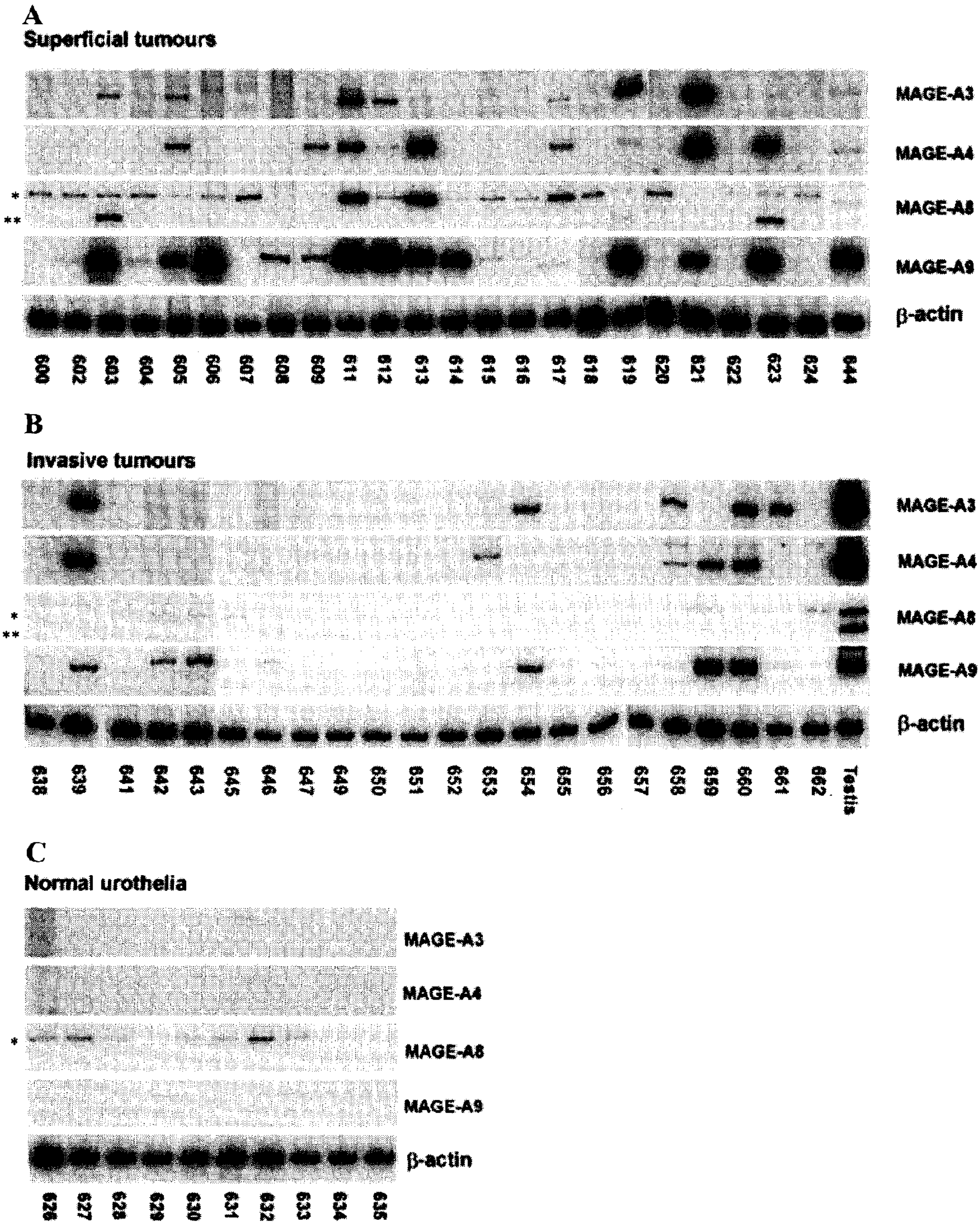
A
Superficial tumours
MAGE-A3
MAGE-A4
*
**
MAGE-A8
MAGE-A9
β-actin
600 602 603 604 605 606 607 608 609 611 612 613 614 615 616 617 618 619 620 621 622 623 624 644
B
Invasive tumours
MAGE-A3
MAGE-A4
*
**
MAGE-A8
MAGE-A9
β-actin
638 639 641 642 643 645 646 647 649 650 651 652 653 654 655 656 657 658 659 660 661 662 Testis
C
Normal urothelia
MAGE-A3
MAGE-A4
*
MAGE-A8
MAGE-A9
β-actin
626 627 628 629 630 631 632 633 634 635

Fig. 1

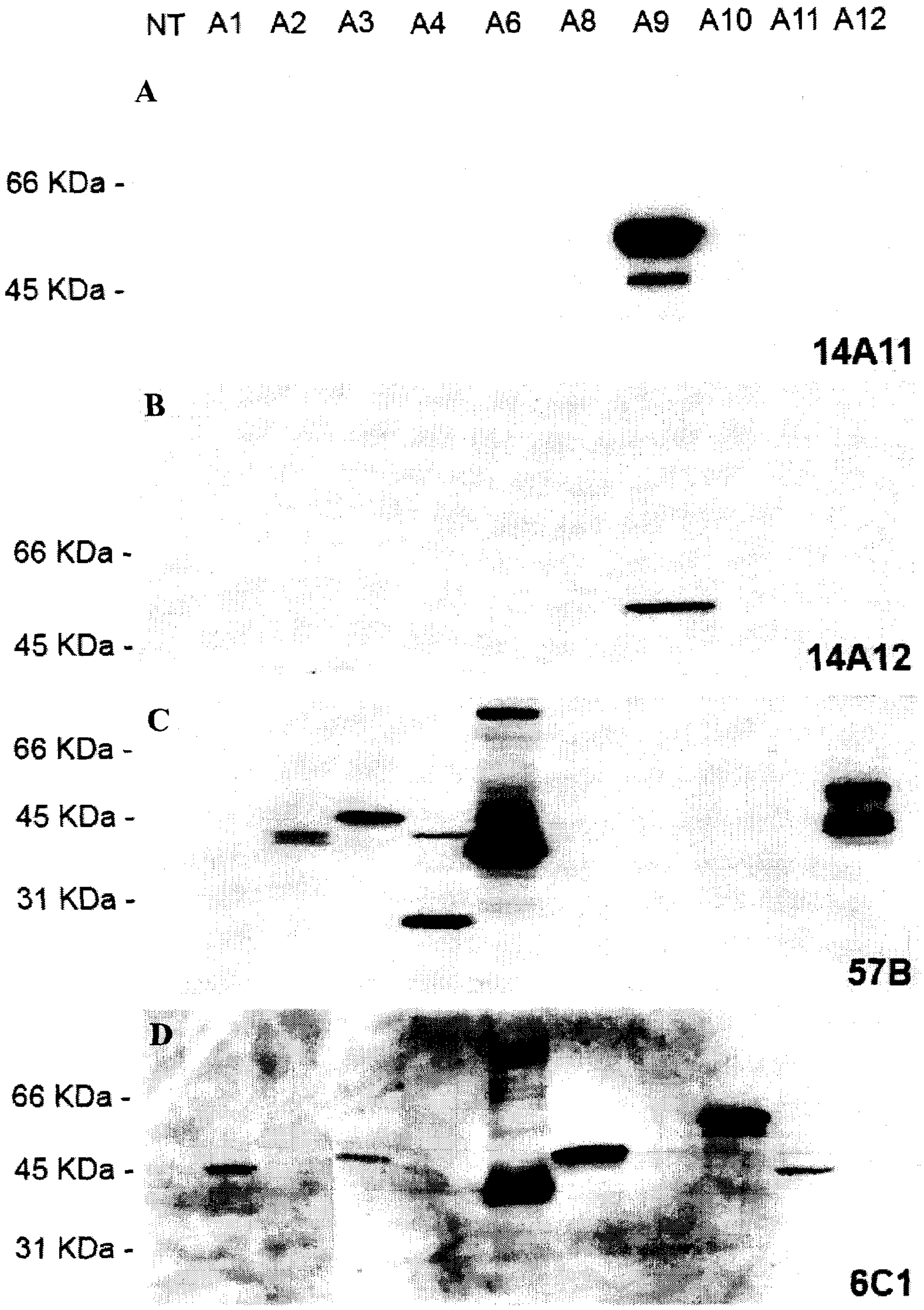
NT A1 A2 A3 A4 A6 A8 A9 A10 A11 A12
A
66 KDa -
45 KDa -
14A11
B
66 KDa -
45 KDa -
14A12
C
66 KDa -
45 KDa -
31 KDa -
57B
D
66 KDa -
45 KDa -
31 KDa -
6C1

Fig. 2

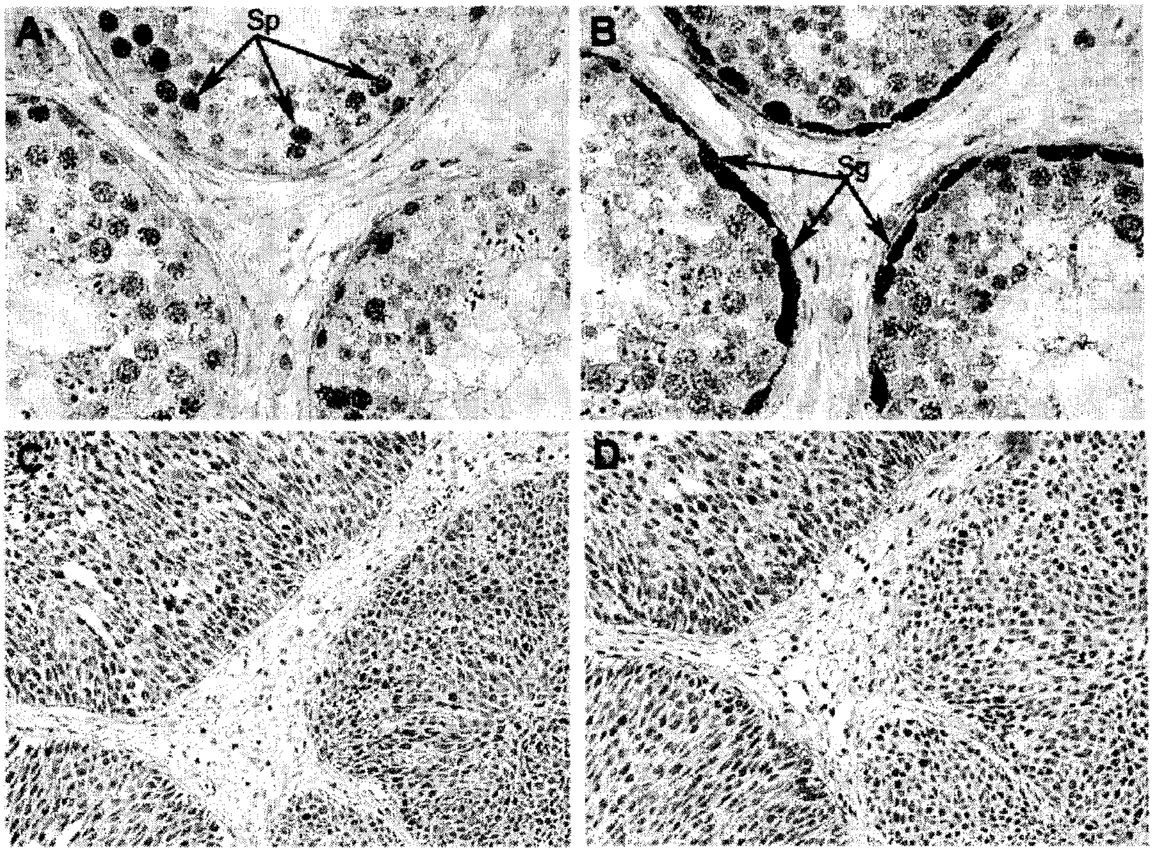


Fig. 3

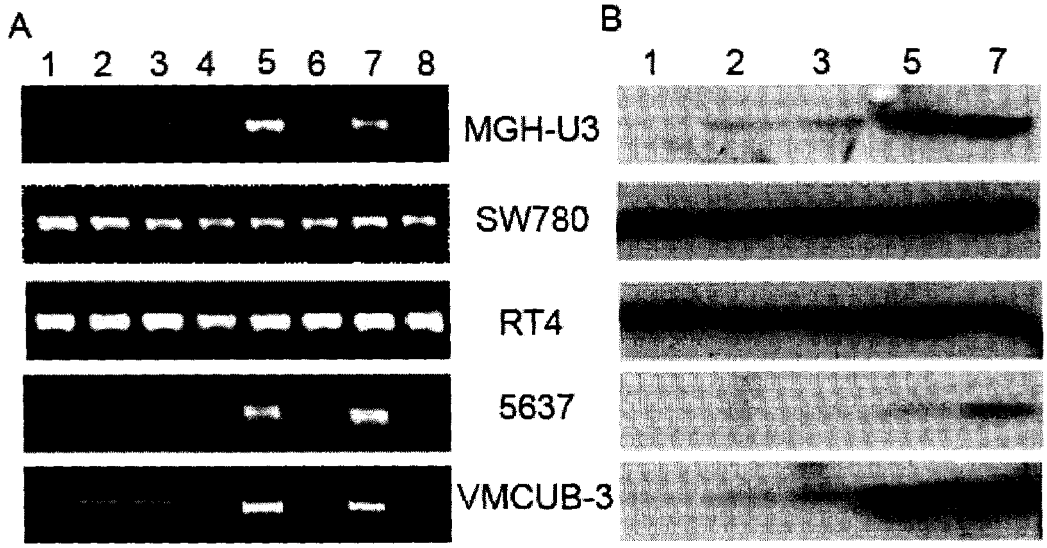


Fig. 4

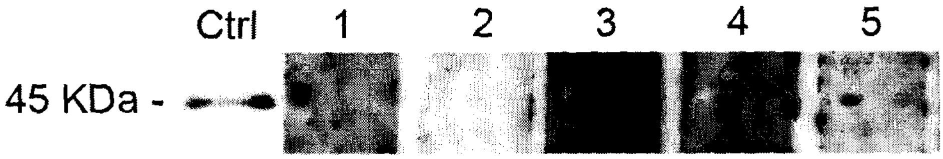


Fig. 5

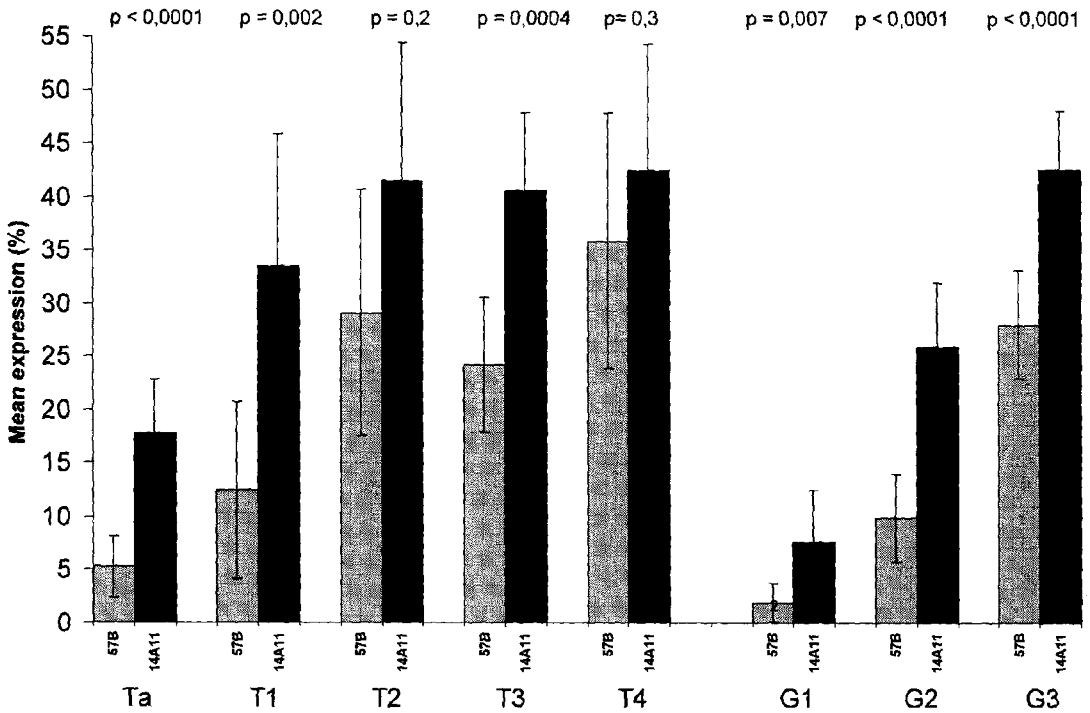
p < 0,0001
p = 0,002
p = 0,2
p = 0,0004
p≈ 0,3
p = 0,007
p < 0,0001
p < 0,0001
Mean expression (%)
55
50
45
40
35
30
25
20
15
10
5
0
57B
14A11
Ta
T1
T2
T3
T4
G1
G2
G3

Fig. 6

## A ) MAGE-A4 (mAb 57b)

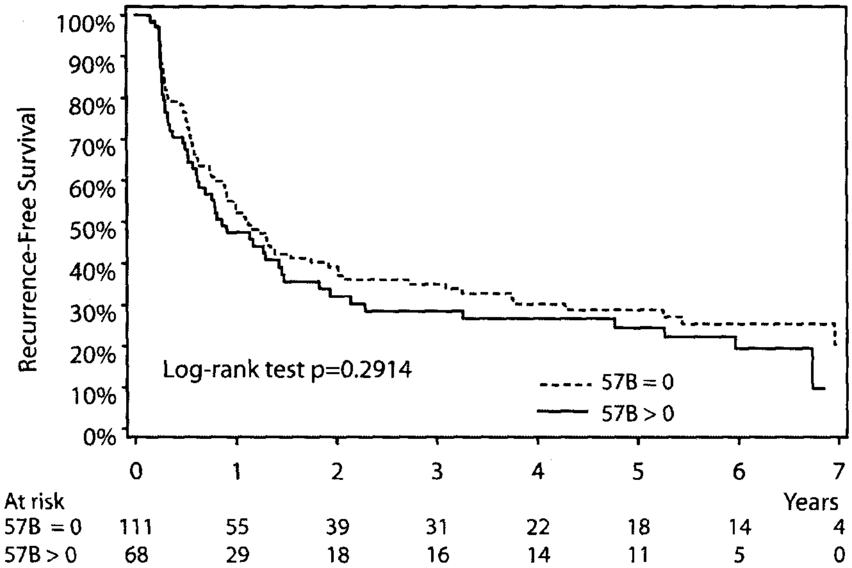


## B) MAGE-A9 (mAb 14A11)

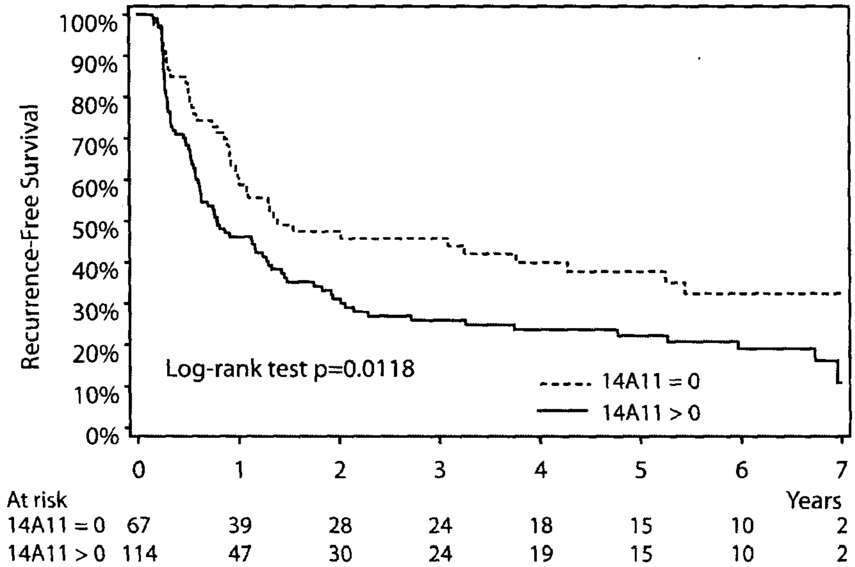


Fig. 7

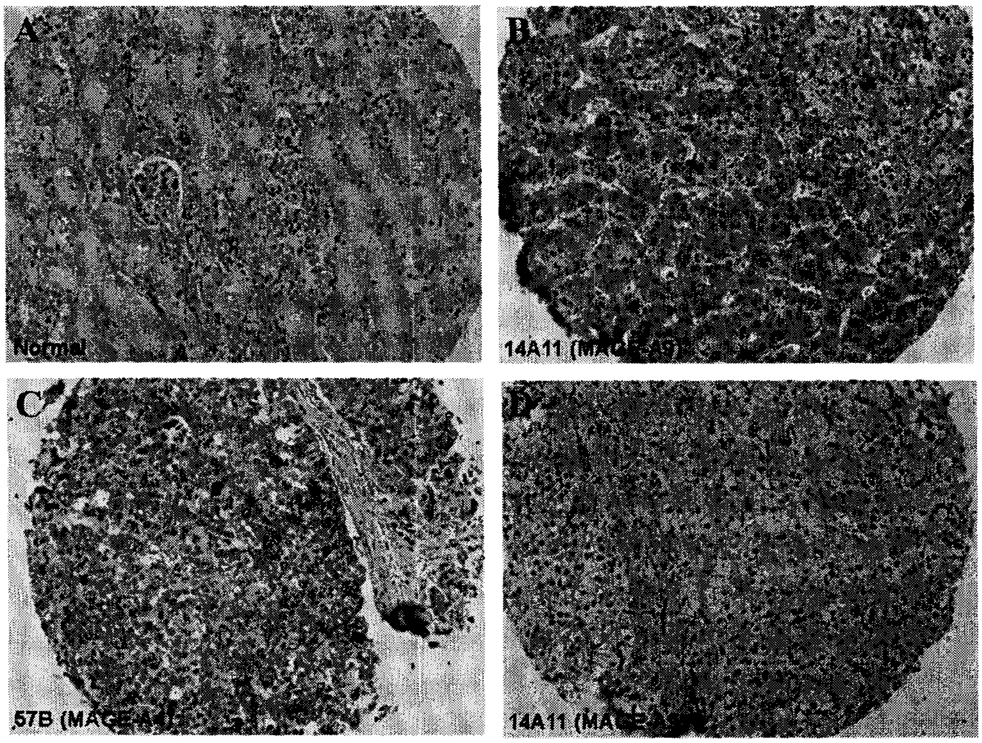


Fig. 8

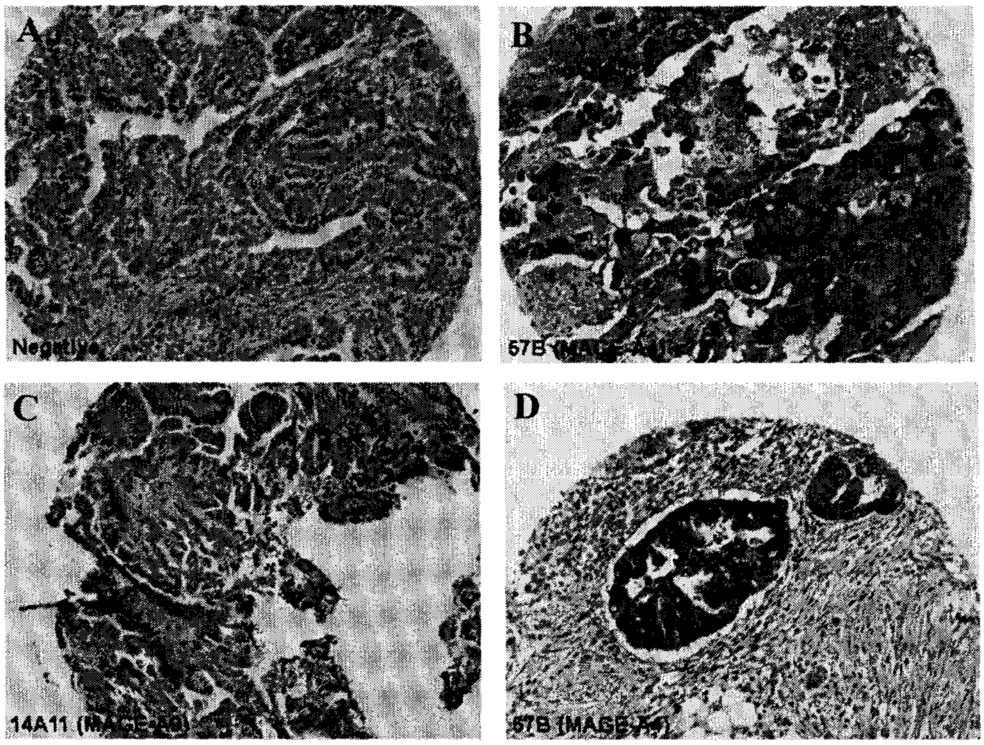


Fig. 9

Europäisches Patentamt
European Patent Office
Office européen des brevets

# EUROPEAN SEARCH REPORT

Application Number
EP 10 18 9300

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/52163 A (LUDWIG INST CANCER RES [US]) 8 September 2000 (2000-09-08)<br>* sequences 19, 23 *<br>* figure 1 *<br>* claims 1-17 *<br>* page 29 *<br>* pages 41-43 *<br>----- | 1-15 | INV.<br>C12P21/08<br>A61K31/7088<br>A61K39/00<br>A61K39/395<br>A61K48/00<br>A61P35/00<br>C07K16/18<br>C12N5/12<br>C12Q1/02<br>C12Q1/68<br>G01N33/53<br>G01N33/574 |
| X | WO 99/40188 A (SMITHKLINE BEECHAM BIOLOG [BE]; CABEZON SILVA TERESA [BE]; COHEN JOSEP) 12 August 1999 (1999-08-12)<br>* page 1 - page 2 *<br>* claims 1-20 *<br>----- | 1-15 | |
| A | US 2003/108890 A1 (BARANOVA ANNA VJACHESLAVOVNA [US] ET AL) 12 June 2003 (2003-06-12)<br>----- | 1-15 | |
| T | PICARD VALERIE ET AL: "MAGE-A9 mRNA and protein expression in bladder cancer", INTERNATIONAL JOURNAL OF CANCER, vol. 120, no. 10, May 2007 (2007-05), pages 2170-2177, XP002509737, ISSN: 0020-7136<br>* the whole document *<br>----- | 1-15 | TECHNICAL FIELDS SEARCHED (IPC)<br>C07K<br>G01N<br>A61K |

The present search report has been drawn up for all claims

| Place of search | Date of completion of the search | Examiner |
|---|---|---|
| Munich | 24 January 2011 | Bayer, Martin |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 10 18 9300

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2011

| Patent document cited in search report | Publication date | Patent family member(s) | Publication date |
|---|---|---|---|
| WO 0052163 A | 08-09-2000 | AU 3389500 A | 21-09-2000 |
| | | CA 2366059 A1 | 08-09-2000 |
| | | EP 1194542 A1 | 10-04-2002 |
| | | JP 2003512814 T | 08-04-2003 |
| | | NZ 513739 A | 28-09-2001 |
| WO 9940188 A | 12-08-1999 | AT 462788 T | 15-04-2010 |
| | | AT 315088 T | 15-02-2006 |
| | | AU 737337 B2 | 16-08-2001 |
| | | BR 9907691 A | 14-11-2000 |
| | | CA 2319309 A1 | 12-08-1999 |
| | | CA 2584482 A1 | 12-08-1999 |
| | | CN 1295616 A | 16-05-2001 |
| | | CZ 20002869 A3 | 17-01-2001 |
| | | CZ 298347 B6 | 05-09-2007 |
| | | DE 69929310 T2 | 03-08-2006 |
| | | DK 1659178 T3 | 12-07-2010 |
| | | DK 1053325 T3 | 22-05-2006 |
| | | EP 1584685 A2 | 12-10-2005 |
| | | EP 1659178 A2 | 24-05-2006 |
| | | EP 1659179 A2 | 24-05-2006 |
| | | EP 1053325 A2 | 22-11-2000 |
| | | ES 2342416 T3 | 06-07-2010 |
| | | ES 2255248 T3 | 16-06-2006 |
| | | HK 1033838 A1 | 03-11-2006 |
| | | HU 0102639 A2 | 28-11-2001 |
| | | IL 137442 A | 13-04-2008 |
| | | JP 2002502604 T | 29-01-2002 |
| | | JP 2009201510 A | 10-09-2009 |
| | | KR 20060067977 A | 20-06-2006 |
| | | NO 20003958 A | 04-10-2000 |
| | | NZ 506086 A | 31-01-2003 |
| | | PL 342852 A1 | 16-07-2001 |
| | | PT 1659178 E | 28-06-2010 |
| | | PT 1053325 E | 31-05-2006 |
| | | SI 1053325 T1 | 30-06-2006 |
| | | SI 1659178 T1 | 30-07-2010 |
| | | TR 200002284 T2 | 21-11-2000 |
| | | TW 238853 B | 01-09-2005 |
| | | US 2010209444 A1 | 19-08-2010 |
| | | US 2010204458 A1 | 12-08-2010 |
| US 2003108890 A1 | 12-06-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **De Plaen et al.** *Immunogenetics,* 1994, vol. 40, 360-9
- **Landry C. et al.** *Int J Cancer,* 2000, vol. 86, 835-41
- **Patard et al.** *Int J Cancer,* 1995, vol. 64, 60-4
- **Bar-Haim et al.** *Br J Cancer,* 2004, vol. 91, 398-407
- **De Plaen E. et al.** *Immunogenetics,* 1994, vol. 40, 360-9
- **Eichmüller et al.** *Int J Cancer,* 2003, vol. 104, 482-7
- **Lin et al.** *Clin Cancer Res,* 2004, vol. 10, 5708-16
- **Oehlrich et al.** *Int J Cancer,* 2005, vol. 117, 256-64
- **Landry C et al.** *Int J Cancer,* 2000, vol. 86, 835-41
- **Gulukota et al.** *J. Mol. Biol.,* 1997, vol. 267, 1258-126
- **Southwood et al.** *J. Immunol.,* 1998, vol. 160, 3363-3373
- **Geluk et al.** *J. Immunol.,* 1994, vol. 152, 5742-5748
- **Sidney et al.** *Human Immunol.,* 1996, vol. 45, 79-93
- **Sijts et al.** *J. Immunol.,* 1996, vol. 156, 683-692
- **Demotz et al.** *Nature,* 1989, vol. 342, 682-684
- **Kageyama et al.** *J. Immunol.,* 1995, vol. 154, 567-576
- **Alexander et al.** *Immunity,* 1994, vol. 1, 751-761
- **Ogg et al.** *Science,* 1998, vol. 279, 2103-2106

SEQUENCE LISTING

```
<110>  UNIVERSITE LAVAL
       BERGERON, ALAIN
       FRADET, YVES
       LARUE, HELENE
       PICARD, VALERIE

<120>  CANCER ANTIGEN MAGE-A9 AND USES THEREOF

<130>  6013-180PCT

<150>  US 60/735,859
<151>  2005-11-14

<160>  441

<170>  PatentIn version 3.3

<210>  1
<211>  1824
<212>  DNA
<213>  Homo sapiens


<220>
<221>  CDS
<222>  (281)..(1228)

<400>  1
ctgggggtca gagagaaggg agaggcctcc ttctgagggg cggcttgata ccggtggagg      60

agctccagga agcaggcagg ccttggtctg agacagtgtc ctcaggtcgc agagcagagg     120

agacccaggc agtgtcagca gtgaaggttc tcgggacagg ctaaccagga ggacaggagc     180

cccaagaggc cccagagcag cactgacgaa gacctgcctg tgggtctcca tcgcccagct     240

cctgcccacg ctcctgactg ctgccctgac cagagtcatc atg tct ctc gag cag      295
                                            Met Ser Leu Glu Gln
                                            1               5

agg agt ccg cac tgc aag cct gat gaa gac ctt gaa gcc caa gga gag      343
Arg Ser Pro His Cys Lys Pro Asp Glu Asp Leu Glu Ala Gln Gly Glu
                10                  15                  20

gac ttg ggc ctg atg ggt gca cag gaa ccc aca ggc gag gag gag gag      391
Asp Leu Gly Leu Met Gly Ala Gln Glu Pro Thr Gly Glu Glu Glu Glu
            25                  30                  35

act acc tcc tcc tct gac agc aag gag gag gag gtg tct gct gct ggg      439
Thr Thr Ser Ser Ser Asp Ser Lys Glu Glu Glu Val Ser Ala Ala Gly
        40                  45                  50

tca tca agt cct ccc cag agt cct cag gga ggc gct tcc tcc tcc att      487
Ser Ser Ser Pro Pro Gln Ser Pro Gln Gly Gly Ala Ser Ser Ser Ile
    55                  60                  65

tcc gtc tac tac act tta tgg agc caa ttc gat gag ggc tcc agc agt      535
Ser Val Tyr Tyr Thr Leu Trp Ser Gln Phe Asp Glu Gly Ser Ser Ser
70                  75                  80                  85

caa gaa gag gaa gag cca agc tcc tcg gtc gac cca gct cag ctg gag      583
Gln Glu Glu Glu Glu Pro Ser Ser Ser Val Asp Pro Ala Gln Leu Glu
                90                  95                  100

ttc atg ttc caa gaa gca ctg aaa ttg aag gtg gct gag ttg gtt cat      631
Phe Met Phe Gln Glu Ala Leu Lys Leu Lys Val Ala Glu Leu Val His
```

```
                105                 110                 115

ttc ctg ctc cac aaa tat cga gtc aag gag ccg gtc aca aag gca gaa      679
Phe Leu Leu His Lys Tyr Arg Val Lys Glu Pro Val Thr Lys Ala Glu
        120                 125                 130

atg ctg gag agc gtc atc aaa aat tac aag cgc tac ttt cct gtg atc      727
Met Leu Glu Ser Val Ile Lys Asn Tyr Lys Arg Tyr Phe Pro Val Ile
    135                 140                 145

ttc ggc aaa gcc tcc gag ttc atg cag gtg atc ttt ggc act gat gtg      775
Phe Gly Lys Ala Ser Glu Phe Met Gln Val Ile Phe Gly Thr Asp Val
150                 155                 160                 165

aag gag gtg gac ccc gcc ggc cac tcc tac atc ctt gtc act gct ctt      823
Lys Glu Val Asp Pro Ala Gly His Ser Tyr Ile Leu Val Thr Ala Leu
                170                 175                 180

ggc ctc tcg tgc gat agc atg ctg ggt gat ggt cat agc atg ccc aag      871
Gly Leu Ser Cys Asp Ser Met Leu Gly Asp Gly His Ser Met Pro Lys
            185                 190                 195

gcc gcc ctc ctg atc att gtc ctg ggt gtg atc cta acc aaa gac aac      919
Ala Ala Leu Leu Ile Ile Val Leu Gly Val Ile Leu Thr Lys Asp Asn
        200                 205                 210

tgc gcc cct gaa gag gtt atc tgg gaa gcg ttg agt gtg atg ggg gtg      967
Cys Ala Pro Glu Glu Val Ile Trp Glu Ala Leu Ser Val Met Gly Val
    215                 220                 225

tat gtt ggg aag gag cac atg ttc tac ggg gag ccc agg aag ctg ctc     1015
Tyr Val Gly Lys Glu His Met Phe Tyr Gly Glu Pro Arg Lys Leu Leu
230                 235                 240                 245

acc caa gat tgg gtg cag gaa aac tac ctg gag tac cgg cag gtg ccc     1063
Thr Gln Asp Trp Val Gln Glu Asn Tyr Leu Glu Tyr Arg Gln Val Pro
                250                 255                 260

ggc agt gat cct gcg cac tac gag ttc ctg tgg ggt tcc aag gcc cac     1111
Gly Ser Asp Pro Ala His Tyr Glu Phe Leu Trp Gly Ser Lys Ala His
            265                 270                 275

gct gaa acc agc tat gag aag gtc ata aat tat ttg gtc atg ctc aat     1159
Ala Glu Thr Ser Tyr Glu Lys Val Ile Asn Tyr Leu Val Met Leu Asn
        280                 285                 290

gca aga gag ccc atc tgc tac cca tcc ctt tat gaa gag gtt ttg gga     1207
Ala Arg Glu Pro Ile Cys Tyr Pro Ser Leu Tyr Glu Glu Val Leu Gly
    295                 300                 305

gag gag caa gag gga gtc tga gcaccagccg cagccggggc caaagtttgt        1258
Glu Glu Gln Glu Gly Val
310                 315

ggggtcaggg ccccatccag cagctgccct gccccatgtg acatgaggcc cattcttcgc   1318

tctgtgtttg aagagagcaa tcagtgttct cagtggcagt gggtggaagt gagcacactg   1378

tatgtcatct ctgggttcct tgtctattgg gtgatttgga gatttatcct tgctcccttt   1438

tggaattgtt caaatgttct tttaatggtc agtttaatga acttcaccat cgaagttaat   1498

gaatgacagt agtcacacat attgctgttt atgttattta ggagtaagat tcttgctttt   1558

gagtcacatg gggaaatccc tgttattttg tgaattggga caagataaca tagcagagga   1618

attaataatt tttttgaaac ttgaacttag cagcaaaata gagctcataa agaaatagtg   1678
```

```
aaatgaaaat gtagttaatt cttgccttat acctctttct ctctcctgta aaattaaaac   1738

atatacatgt atacctggat ttgcttggct tctttgagca tgtaagagaa ataaaaattg   1798

aaagaataaa aaaaaaaaaa aaaaaa                                        1824


<210>  2
<211>  315
<212>  PRT
<213>  Homo sapiens

<400>  2

Met Ser Leu Glu Gln Arg Ser Pro His Cys Lys Pro Asp Glu Asp Leu
1               5                   10                  15

Glu Ala Gln Gly Glu Asp Leu Gly Leu Met Gly Ala Gln Glu Pro Thr
            20                  25                  30

Gly Glu Glu Glu Glu Thr Thr Ser Ser Ser Asp Ser Lys Glu Glu Glu
        35                  40                  45

Val Ser Ala Ala Gly Ser Ser Ser Pro Pro Gln Ser Pro Gln Gly Gly
    50                  55                  60

Ala Ser Ser Ser Ile Ser Val Tyr Tyr Thr Leu Trp Ser Gln Phe Asp
65                  70                  75                  80

Glu Gly Ser Ser Ser Gln Glu Glu Glu Glu Pro Ser Ser Ser Val Asp
                85                  90                  95

Pro Ala Gln Leu Glu Phe Met Phe Gln Glu Ala Leu Lys Leu Lys Val
            100                 105                 110

Ala Glu Leu Val His Phe Leu Leu His Lys Tyr Arg Val Lys Glu Pro
        115                 120                 125

Val Thr Lys Ala Glu Met Leu Glu Ser Val Ile Lys Asn Tyr Lys Arg
    130                 135                 140

Tyr Phe Pro Val Ile Phe Gly Lys Ala Ser Glu Phe Met Gln Val Ile
145                 150                 155                 160

Phe Gly Thr Asp Val Lys Glu Val Asp Pro Ala Gly His Ser Tyr Ile
                165                 170                 175

Leu Val Thr Ala Leu Gly Leu Ser Cys Asp Ser Met Leu Gly Asp Gly
            180                 185                 190

His Ser Met Pro Lys Ala Ala Leu Leu Ile Ile Val Leu Gly Val Ile
        195                 200                 205

Leu Thr Lys Asp Asn Cys Ala Pro Glu Glu Val Ile Trp Glu Ala Leu
    210                 215                 220
```

Ser Val Met Gly Val Tyr Val Gly Lys Glu His Met Phe Tyr Gly Glu
225 230 235 240

Pro Arg Lys Leu Leu Thr Gln Asp Trp Val Gln Glu Asn Tyr Leu Glu
245 250 255

Tyr Arg Gln Val Pro Gly Ser Asp Pro Ala His Tyr Glu Phe Leu Trp
260 265 270

Gly Ser Lys Ala His Ala Glu Thr Ser Tyr Glu Lys Val Ile Asn Tyr
275 280 285

Leu Val Met Leu Asn Ala Arg Glu Pro Ile Cys Tyr Pro Ser Leu Tyr
290 295 300

Glu Glu Val Leu Gly Glu Glu Gln Glu Gly Val
305 310 315

<210> 3
<211> 20
<212> DNA
<213> Artificial

<220>
<223> beta-actin primer

<400> 3
tcatcaccat tggcaatgag 20

<210> 4
<211> 19
<212> DNA
<213> Artificial

<220>
<223> beta-actin primer

<400> 4
gatgtccacg tcacacttc 19

<210> 5
<211> 19
<212> DNA
<213> Artificial

<220>
<223> MAGE-A3 primer

<400> 5
tggaggacca gaggccccc 19

<210> 6
<211> 22
<212> DNA
<213> Artificial

<220>

<223> MAGE-A3 primer

<400> 6
ggacgattat caggaggcct gc 22

<210> 7
<211> 18
<212> DNA
<213> Artificial

<220>
<223> MAGE-A4 primer

<400> 7
gagcagacag gccaaccg 18

<210> 8
<211> 18
<212> DNA
<213> Artificial

<220>
<223> MAGE-A4 primer

<400> 8
aaggactctg cgtcaggc 18

<210> 9
<211> 22
<212> DNA
<213> Artificial

<220>
<223> MAGE-A8 primer

<400> 9
ccccagagaa gcactgaaga ag 22

<210> 10
<211> 17
<212> DNA
<213> Artificial

<220>
<223> MAGE-A8 primer

<400> 10
ggtgagctgg gtccggg 17

<210> 11
<211> 19
<212> DNA
<213> Artificial

<220>
<223> MAGE-A9 primer

<400> 11
ccccagagca gcactgacg 19

<210> 12
<211> 19

<212> DNA
<213> Artificial

<220>
<223> MAGE-A9 primer

<400> 12
cagctgagct gggtcgacc 19

<210> 13
<211> 9
<212> PRT
<213> Artificial

<220>
<223> fragment of SEQ ID NO:2

<400> 13

Glu Val Asp Pro Ala Gly His Ser Tyr
1 5

<210> 14
<211> 9
<212> PRT
<213> Artificial

<220>
<223> fragment of SEQ ID NO:2

<400> 14

Gly Ser Asp Pro Ala His Tyr Glu Phe
1 5

<210> 15
<211> 9
<212> PRT
<213> Artificial

<220>
<223> fragment of SEQ ID NO:2

<400> 15

Ser Val Asp Pro Ala Gln Leu Glu Phe
1 5

<210> 16
<211> 9
<212> PRT
<213> Artificial

<220>
<223> fragment of SEQ ID NO:2

<400> 16

Ser Ser Asp Ser Lys Glu Glu Glu Val
1 5

<210> 17
<211> 9

<212> PRT
<213> Artificial

<220>
<223> fragment of SEQ ID NO:2

<400> 17

Glu Glu Glu Thr Thr Ser Ser Ser Asp
1               5

<210> 18
<211> 9
<212> PRT
<213> Artificial

<220>
<223> fragment of SEQ ID NO:2

<400> 18

Gln Phe Asp Glu Gly Ser Ser Ser Gln
1               5

<210> 19
<211> 9
<212> PRT
<213> Artificial

<220>
<223> fragment of SEQ ID NO:2

<400> 19

Ala Gln Glu Pro Thr Gly Glu Glu Glu
1               5

<210> 20
<211> 9
<212> PRT
<213> Artificial

<220>
<223> fragment of SEQ ID NO:2

<400> 20

Ala Leu Lys Leu Lys Val Ala Glu Leu
1               5

<210> 21
<211> 9
<212> PRT
<213> Artificial

<220>
<223> fragment of SEQ ID NO:2

<400> 21

Ala Leu Leu Ile Ile Val Leu Gly Val
1               5

<210> 22
<211> 9
<212> PRT
<213> Artificial

<220>
<223> fragment of SEQ ID NO:2

<400> 22

Ala Leu Ser Val Met Gly Val Tyr Val
1 5

<210> 23
<211> 9
<212> PRT
<213> Artificial

<220>
<223> fragment of SEQ ID NO:2

<400> 23

Ser Met Pro Lys Ala Ala Leu Leu Ile
1 5

<210> 24
<211> 9
<212> PRT
<213> Artificial

<220>
<223> fragment of SEQ ID NO:2

<400> 24

Lys Val Ile Asn Tyr Leu Val Met Leu
1 5

<210> 25
<211> 9
<212> PRT
<213> Artificial

<220>
<223> fragment of SEQ ID NO:2

<400> 25

Val Ile Trp Glu Ala Leu Ser Val Met
1 5

<210> 26
<211> 9
<212> PRT
<213> Artificial

<220>
<223> fragment of SEQ ID NO:2

<400> 26

Leu Leu Ile Ile Val Leu Gly Val Ile
1 5

<210> 27
<211> 9
<212> PRT
<213> Artificial

<220>
<223> fragment of SEQ ID NO:2

<400> 27

Ser Leu Tyr Glu Glu Val Leu Gly Glu
1 5

<210> 28
<211> 9
<212> PRT
<213> Artificial

<220>
<223> fragment of SEQ ID NO:2

<400> 28

Glu Thr Thr Ser Ser Ser Asp Ser Lys
1 5

<210> 29
<211> 9
<212> PRT
<213> Artificial

<220>
<223> fragment of SEQ ID NO:2

<400> 29

Ala His Ala Glu Thr Ser Tyr Glu Lys
1 5

<210> 30
<211> 9
<212> PRT
<213> Artificial

<220>
<223> fragment of SEQ ID NO:2

<400> 30

Phe Leu Leu His Lys Tyr Arg Val Lys
1 5

<210> 31
<211> 9
<212> PRT
<213> Artificial

<220>
<223> fragment of SEQ ID NO:2

<400> 31

Ile Val Leu Gly Val Ile Leu Thr Lys
1 5

<210> 32
<211> 9
<212> PRT
<213> Artificial

<220>
<223> fragment of SEQ ID NO:2

<400> 32

Glu Leu Val His Phe Leu Leu His Lys
1 5

<210> 33
<211> 9
<212> PRT
<213> Artificial

<220>
<223> fragment of SEQ ID NO:2

<400> 33

Ser Val Tyr Tyr Thr Leu Trp Ser Gln
1 5

<210> 34
<211> 9
<212> PRT
<213> Artificial

<220>
<223> fragment of SEQ ID NO:2

<400> 34

Ala His Ala Glu Thr Ser Tyr Glu Lys
1 5

<210> 35
<211> 9
<212> PRT
<213> Artificial

<220>
<223> fragment of SEQ ID NO:2

<400> 35

Ile Val Leu Gly Val Ile Leu Thr Lys
1 5

<210> 36
<211> 9
<212> PRT
<213> Artificial

<220>
<223> fragment of SEQ ID NO:2

<400> 36

Glu Thr Thr Ser Ser Ser Asp Ser Lys
1 5

<210> 37
<211> 9
<212> PRT
<213> Artificial

<220>
<223> fragment of SEQ ID NO:2

<400> 37

Ser Val Met Gly Val Tyr Val Gly Lys
1 5

<210> 38
<211> 9
<212> PRT
<213> Artificial

<220>
<223> fragment of SEQ ID NO:2

<400> 38

Ser Leu Tyr Glu Glu Val Leu Gly Glu
1 5

<210> 39
<211> 9
<212> PRT
<213> Artificial

<220>
<223> fragment of SEQ ID NO:2

<400> 39

Glu Leu Val His Phe Leu Leu His Lys
1 5

<210> 40
<211> 9
<212> PRT
<213> Artificial

<220>
<223> fragment of SEQ ID NO:2

<400> 40

Ser Val Tyr Tyr Thr Leu Trp Ser Gln
1 5

<210> 41
<211> 9
<212> PRT
<213> Artificial

<220>

<223> fragment of SEQ ID NO:2

<400> 41

Ser Tyr Glu Lys Val Ile Asn Tyr Leu
1 5

<210> 42
<211> 9
<212> PRT
<213> Artificial

<220>
<223> fragment of SEQ ID NO:2

<400> 42

Val Tyr Tyr Thr Leu Trp Ser Gln Phe
1 5

<210> 43
<211> 9
<212> PRT
<213> Artificial

<220>
<223> fragment of SEQ ID NO:2

<400> 43

Cys Tyr Pro Ser Leu Tyr Glu Glu Val
1 5

<210> 44
<211> 9
<212> PRT
<213> Artificial

<220>
<223> fragment of SEQ ID NO:2

<400> 44

Val Tyr Val Gly Lys Glu His Met Phe
1 5

<210> 45
<211> 9
<212> PRT
<213> Artificial

<220>
<223> fragment of SEQ ID NO:2

<400> 45

Asn Tyr Lys Arg Tyr Phe Pro Val Ile
1 5

<210> 46
<211> 9
<212> PRT
<213> Artificial

<220>
<223> fragment of SEQ ID NO:2

<400> 46

Phe Tyr Gly Glu Pro Arg Lys Leu Leu
1 5

<210> 47
<211> 9
<212> PRT
<213> Artificial

<220>
<223> fragment of SEQ ID NO:2

<400> 47

Met Phe Tyr Gly Glu Pro Arg Lys Leu
1 5

<210> 48
<211> 9
<212> PRT
<213> Artificial

<220>
<223> fragment of SEQ ID NO:2

<400> 48

Ile Val Leu Gly Val Ile Leu Thr Lys
1 5

<210> 49
<211> 9
<212> PRT
<213> Artificial

<220>
<223> fragment of SEQ ID NO:2

<400> 49

Glu Thr Thr Ser Ser Ser Asp Ser Lys
1 5

<210> 50
<211> 9
<212> PRT
<213> Artificial

<220>
<223> fragment of SEQ ID NO:2

<400> 50

Ser Val Met Gly Val Tyr Val Gly Lys
1 5

<210> 51
<211> 9

<212> PRT
<213> Artificial

<220>
<223> fragment of SEQ ID NO:2

<400> 51

Ala His Ala Glu Thr Ser Tyr Glu Lys
1 5

<210> 52
<211> 9
<212> PRT
<213> Artificial

<220>
<223> fragment of SEQ ID NO:2

<400> 52

Glu Leu Val His Phe Leu Leu His Lys
1 5

<210> 53
<211> 9
<212> PRT
<213> Artificial

<220>
<223> fragment of SEQ ID NO:2

<400> 53

Ser Val Tyr Tyr Thr Leu Trp Ser Gln
1 5

<210> 54
<211> 9
<212> PRT
<213> Artificial

<220>
<223> fragment of SEQ ID NO:2

<400> 54

His Met Phe Tyr Gly Glu Pro Arg Lys
1 5

<210> 55
<211> 9
<212> PRT
<213> Artificial

<220>
<223> fragment of SEQ ID NO:2

<400> 55

Ser Val Tyr Tyr Thr Leu Trp Ser Gln
1 5

<210> 56
<211> 9
<212> PRT
<213> Artificial

<220>
<223> fragment of SEQ ID NO:2

<400> 56

Ala His Ala Glu Thr Ser Tyr Glu Lys
1 5

<210> 57
<211> 9
<212> PRT
<213> Artificial

<220>
<223> fragment of SEQ ID NO:2

<400> 57

Glu Met Leu Glu Ser Val Ile Lys Asn
1 5

<210> 58
<211> 9
<212> PRT
<213> Artificial

<220>
<223> fragment of SEQ ID NO:2

<400> 58

Ser Leu Tyr Glu Glu Val Leu Gly Glu
1 5

<210> 59
<211> 9
<212> PRT
<213> Artificial

<220>
<223> fragment of SEQ ID NO:2

<400> 59

Arg Tyr Phe Pro Val Ile Phe Gly Lys
1 5

<210> 60
<211> 9
<212> PRT
<213> Artificial

<220>
<223> fragment of SEQ ID NO:2

<400> 60

Glu Ser Val Ile Lys Asn Tyr Lys Arg
1 5

<210> 61
<211> 9
<212> PRT
<213> Artificial

<220>
<223> fragment of SEQ ID NO:2

<400> 61

His Met Phe Tyr Gly Glu Pro Arg Lys
1               5

<210> 62
<211> 9
<212> PRT
<213> Artificial

<220>
<223> fragment of SEQ ID NO:2

<400> 62

Glu Val Asp Pro Ala Gly His Ser Tyr
1               5

<210> 63
<211> 9
<212> PRT
<213> Artificial

<220>
<223> fragment of SEQ ID NO:2

<400> 63

Ser Val Asp Pro Ala Gln Leu Glu Phe
1               5

<210> 64
<211> 9
<212> PRT
<213> Artificial

<220>
<223> fragment of SEQ ID NO:2

<400> 64

Gly Ser Asp Pro Ala His Tyr Glu Phe
1               5

<210> 65
<211> 9
<212> PRT
<213> Artificial

<220>
<223> fragment of SEQ ID NO:2

<400> 65

Met Leu Glu Ser Val Ile Lys Asn Tyr
1               5

<210> 66
<211> 9
<212> PRT
<213> Artificial

<220>
<223> fragment of SEQ ID NO:2

<400> 66

Ala Ser Glu Phe Met Gln Val Ile Phe
1               5

<210> 67
<211> 9
<212> PRT
<213> Artificial

<220>
<223> fragment of SEQ ID NO:2

<400> 67

Leu Gly Asp Gly His Ser Met Pro Lys
1               5

<210> 68
<211> 9
<212> PRT
<213> Artificial

<220>
<223> fragment of SEQ ID NO:2

<400> 68

Tyr Gly Glu Pro Arg Lys Leu Leu Thr
1               5

<210> 69
<211> 9
<212> PRT
<213> Artificial

<220>
<223> fragment of SEQ ID NO:2

<400> 69

Val Ala Glu Leu Val His Phe Leu Leu
1               5

<210> 70
<211> 9
<212> PRT
<213> Artificial

<220>
<223> fragment of SEQ ID NO:2

<400> 70

Thr Gln Asp Trp Val Gln Glu Asn Tyr
1               5

<210> 71
<211> 9
<212> PRT
<213> Artificial

<220>
<223> fragment of SEQ ID NO:2

<400> 71

Thr Ser Tyr Glu Lys Val Ile Asn Tyr
1               5

<210> 72
<211> 9
<212> PRT
<213> Artificial

<220>
<223> fragment of SEQ ID NO:2

<400> 72

Trp Val Gln Glu Asn Tyr Leu Glu Tyr
1               5

<210> 73
<211> 9
<212> PRT
<213> Artificial

<220>
<223> fragment of SEQ ID NO:2

<400> 73

Ser Leu Glu Gln Arg Ser Pro His Cys
1               5

<210> 74
<211> 9
<212> PRT
<213> Artificial

<220>
<223> fragment of SEQ ID NO:2

<400> 74

Tyr Leu Glu Tyr Arg Gln Val Pro Gly
1               5

<210> 75
<211> 9
<212> PRT
<213> Artificial

<220>

<223> fragment of SEQ ID NO:2

<400> 75

Ala Ser Ser Ser Ile Ser Val Tyr Tyr
1 5

<210> 76
<211> 9
<212> PRT
<213> Artificial

<220>
<223> fragment of SEQ ID NO:2

<400> 76

Val Gln Glu Asn Tyr Leu Glu Tyr Arg
1 5

<210> 77
<211> 9
<212> PRT
<213> Artificial

<220>
<223> fragment of SEQ ID NO:2

<400> 77

Gln Gly Glu Asp Leu Gly Leu Met Gly
1 5

<210> 78
<211> 9
<212> PRT
<213> Artificial

<220>
<223> fragment of SEQ ID NO:2

<400> 78

Ile Val Leu Gly Val Ile Leu Thr Lys
1 5

<210> 79
<211> 9
<212> PRT
<213> Artificial

<220>
<223> fragment of SEQ ID NO:2

<400> 79

Glu Leu Val His Phe Leu Leu His Lys
1 5

<210> 80
<211> 9
<212> PRT
<213> Artificial

<220>
<223> fragment of SEQ ID NO:2

<400> 80

His Tyr Glu Phe Leu Trp Gly Ser Lys
1               5

<210> 81
<211> 9
<212> PRT
<213> Artificial

<220>
<223> fragment of SEQ ID NO:2

<400> 81

His Ala Glu Thr Ser Tyr Glu Lys Val
1               5

<210> 82
<211> 9
<212> PRT
<213> Artificial

<220>
<223> fragment of SEQ ID NO:2

<400> 82

Ala Leu Leu Ile Ile Val Leu Gly Val
1               5

<210> 83
<211> 9
<212> PRT
<213> Artificial

<220>
<223> fragment of SEQ ID NO:2

<400> 83

Ala Leu Ser Val Met Gly Val Tyr Val
1               5

<210> 84
<211> 9
<212> PRT
<213> Artificial

<220>
<223> fragment of SEQ ID NO:2

<400> 84

Lys Val Ala Glu Leu Val His Phe Leu
1               5

<210> 85
<211> 9

<212> PRT
<213> Artificial

<220>
<223> fragment of SEQ ID NO:2

<400> 85

Phe Met Phe Gln Glu Ala Leu Lys Leu
1 5

<210> 86
<211> 9
<212> PRT
<213> Artificial

<220>
<223> fragment of SEQ ID NO:2

<400> 86

Val Leu Gly Glu Glu Gln Glu Gly Val
1 5

<210> 87
<211> 9
<212> PRT
<213> Artificial

<220>
<223> fragment of SEQ ID NO:2

<400> 87

Phe Leu Trp Gly Ser Lys Ala His Ala
1 5

<210> 88
<211> 9
<212> PRT
<213> Artificial

<220>
<223> fragment of SEQ ID NO:2

<400> 88

Tyr Ile Leu Val Thr Ala Leu Gly Leu
1 5

<210> 89
<211> 9
<212> PRT
<213> Artificial

<220>
<223> fragment of SEQ ID NO:2

<400> 89

Met Gln Val Ile Phe Gly Thr Asp Val
1 5

<210> 90
<211> 9
<212> PRT
<213> Artificial

<220>
<223> fragment of SEQ ID NO:2

<400> 90

Lys Asn Tyr Lys Arg Tyr Phe Pro Val
1               5

<210> 91
<211> 9
<212> PRT
<213> Artificial

<220>
<223> fragment of SEQ ID NO:2

<400> 91

Val Ile Trp Glu Ala Leu Ser Val Met
1               5

<210> 92
<211> 9
<212> PRT
<213> Artificial

<220>
<223> fragment of SEQ ID NO:2

<400> 92

Val Met Leu Asn Ala Arg Glu Pro Ile
1               5

<210> 93
<211> 9
<212> PRT
<213> Artificial

<220>
<223> fragment of SEQ ID NO:2

<400> 93

Lys Val Ile Asn Tyr Leu Val Met Leu
1               5

<210> 94
<211> 9
<212> PRT
<213> Artificial

<220>
<223> fragment of SEQ ID NO:2

<400> 94

Trp Glu Ala Leu Ser Val Met Gly Val
1               5

<210> 95
<211> 9
<212> PRT
<213> Artificial

<220>
<223> fragment of SEQ ID NO:2

<400> 95

Gly Leu Met Gly Ala Gln Glu Pro Thr
1 5

<210> 96
<211> 9
<212> PRT
<213> Artificial

<220>
<223> fragment of SEQ ID NO:2

<400> 96

Ser Met Leu Gly Asp Gly His Ser Met
1 5

<210> 97
<211> 9
<212> PRT
<213> Artificial

<220>
<223> fragment of SEQ ID NO:2

<400> 97

Ser Met Pro Lys Ala Ala Leu Leu Ile
1 5

<210> 98
<211> 9
<212> PRT
<213> Artificial

<220>
<223> fragment of SEQ ID NO:2

<400> 98

Lys Ala Ser Glu Phe Met Gln Val Ile
1 5

<210> 99
<211> 9
<212> PRT
<213> Artificial

<220>
<223> fragment of SEQ ID NO:2

<400> 99

Leu Glu Phe Met Phe Gln Glu Ala Leu
1               5

<210> 100
<211> 9
<212> PRT
<213> Artificial

<220>
<223> fragment of SEQ ID NO:2

<400> 100

Val Ile Leu Thr Lys Asp Asn Cys Ala
1               5

<210> 101
<211> 9
<212> PRT
<213> Artificial

<220>
<223> fragment of SEQ ID NO:2

<400> 101

Ile Ile Val Leu Gly Val Ile Leu Thr
1               5

<210> 102
<211> 9
<212> PRT
<213> Artificial

<220>
<223> fragment of SEQ ID NO:2

<400> 102

His Met Phe Tyr Gly Glu Pro Arg Lys
1               5

<210> 103
<211> 9
<212> PRT
<213> Artificial

<220>
<223> fragment of SEQ ID NO:2

<400> 103

Glu Leu Val His Phe Leu Leu His Lys
1               5

<210> 104
<211> 9
<212> PRT
<213> Artificial

<220>
<223> fragment of SEQ ID NO:2

<400> 104

Ile Val Leu Gly Val Ile Leu Thr Lys
1 5

<210> 105
<211> 9
<212> PRT
<213> Artificial

<220>
<223> fragment of SEQ ID NO:2

<400> 105

Ser Val Met Gly Val Tyr Val Gly Lys
1 5

<210> 106
<211> 9
<212> PRT
<213> Artificial

<220>
<223> fragment of SEQ ID NO:2

<400> 106

Phe Met Phe Gln Glu Ala Leu Lys Leu
1 5

<210> 107
<211> 9
<212> PRT
<213> Artificial

<220>
<223> fragment of SEQ ID NO:2

<400> 107

Met Leu Glu Ser Val Ile Lys Asn Tyr
1 5

<210> 108
<211> 9
<212> PRT
<213> Artificial

<220>
<223> fragment of SEQ ID NO:2

<400> 108

Gln Val Ile Phe Gly Thr Asp Val Lys
1 5

<210> 109
<211> 9
<212> PRT
<213> Artificial

<220>

<223> fragment of SEQ ID NO:2

<400> 109

Phe Leu Leu His Lys Tyr Arg Val Lys
1 5

<210> 110
<211> 9
<212> PRT
<213> Artificial

<220>
<223> fragment of SEQ ID NO:2

<400> 110

Ala Leu Leu Ile Ile Val Leu Gly Val
1 5

<210> 111
<211> 9
<212> PRT
<213> Artificial

<220>
<223> fragment of SEQ ID NO:2

<400> 111

Ala Leu Lys Leu Lys Val Ala Glu Leu
1 5

<210> 112
<211> 9
<212> PRT
<213> Artificial

<220>
<223> fragment of SEQ ID NO:2

<400> 112

Val Ile Phe Gly Lys Ala Ser Glu Phe
1 5

<210> 113
<211> 9
<212> PRT
<213> Artificial

<220>
<223> fragment of SEQ ID NO:2

<400> 113

Phe Leu Trp Gly Ser Lys Ala His Ala
1 5

<210> 114
<211> 9
<212> PRT
<213> Artificial

<220>
<223> fragment of SEQ ID NO:2

<400> 114

Lys Val Ile Asn Tyr Leu Val Met Leu
1 5

<210> 115
<211> 9
<212> PRT
<213> Artificial

<220>
<223> fragment of SEQ ID NO:2

<400> 115

Lys Leu Lys Val Ala Glu Leu Val His
1 5

<210> 116
<211> 9
<212> PRT
<213> Artificial

<220>
<223> fragment of SEQ ID NO:2

<400> 116

Trp Val Gln Glu Asn Tyr Leu Glu Tyr
1 5

<210> 117
<211> 9
<212> PRT
<213> Artificial

<220>
<223> fragment of SEQ ID NO:2

<400> 117

Ser Met Pro Lys Ala Ala Leu Leu Ile
1 5

<210> 118
<211> 9
<212> PRT
<213> Artificial

<220>
<223> fragment of SEQ ID NO:2

<400> 118

Arg Tyr Phe Pro Val Ile Phe Gly Lys
1 5

<210> 119
<211> 9

<212> PRT
<213> Artificial

<220>
<223> fragment of SEQ ID NO:2

<400> 119

Val Met Leu Asn Ala Arg Glu Pro Ile
1               5

<210> 120
<211> 9
<212> PRT
<213> Artificial

<220>
<223> fragment of SEQ ID NO:2

<400> 120

Ser Leu Tyr Glu Glu Val Leu Gly Glu
1               5

<210> 121
<211> 9
<212> PRT
<213> Artificial

<220>
<223> fragment of SEQ ID NO:2

<400> 121

Thr Ser Tyr Glu Lys Val Ile Asn Tyr
1               5

<210> 122
<211> 9
<212> PRT
<213> Artificial

<220>
<223> fragment of SEQ ID NO:2

<400> 122

Arg Tyr Phe Pro Val Ile Phe Gly Lys
1               5

<210> 123
<211> 9
<212> PRT
<213> Artificial

<220>
<223> fragment of SEQ ID NO:2

<400> 123

Ile Val Leu Gly Val Ile Leu Thr Lys
1               5

<210> 124
<211> 9
<212> PRT
<213> Artificial

<220>
<223> fragment of SEQ ID NO:2

<400> 124

Ser Val Met Gly Val Tyr Val Gly Lys
1 5

<210> 125
<211> 9
<212> PRT
<213> Artificial

<220>
<223> fragment of SEQ ID NO:2

<400> 125

Gln Val Ile Phe Gly Thr Asp Val Lys
1 5

<210> 126
<211> 9
<212> PRT
<213> Artificial

<220>
<223> fragment of SEQ ID NO:2

<400> 126

His Met Phe Tyr Gly Glu Pro Arg Lys
1 5

<210> 127
<211> 9
<212> PRT
<213> Artificial

<220>
<223> fragment of SEQ ID NO:2

<400> 127

His Tyr Glu Phe Leu Trp Gly Ser Lys
1 5

<210> 128
<211> 9
<212> PRT
<213> Artificial

<220>
<223> fragment of SEQ ID NO:2

<400> 128

Glu Leu Val His Phe Leu Leu His Lys
1 5

<210> 129
<211> 9
<212> PRT
<213> Artificial

<220>
<223> fragment of SEQ ID NO:2

<400> 129

Glu Thr Thr Ser Ser Ser Asp Ser Lys
1 5

<210> 130
<211> 9
<212> PRT
<213> Artificial

<220>
<223> fragment of SEQ ID NO:2

<400> 130

Val Gln Glu Asn Tyr Leu Glu Tyr Arg
1 5

<210> 131
<211> 9
<212> PRT
<213> Artificial

<220>
<223> fragment of SEQ ID NO:2

<400> 131

Gly Val Tyr Val Gly Lys Glu His Met
1 5

<210> 132
<211> 9
<212> PRT
<213> Artificial

<220>
<223> fragment of SEQ ID NO:2

<400> 132

Asn Tyr Leu Val Met Leu Asn Ala Arg
1 5

<210> 133
<211> 9
<212> PRT
<213> Artificial

<220>
<223> fragment of SEQ ID NO:2

<400> 133

Ala Glu Met Leu Glu Ser Val Ile Lys
1               5

<210>  134
<211>  9
<212>  PRT
<213>  Artificial

<220>
<223>  fragment of SEQ ID NO:2

<400>  134

Glu Phe Met Phe Gln Glu Ala Leu Lys
1               5

<210>  135
<211>  9
<212>  PRT
<213>  Artificial

<220>
<223>  fragment of SEQ ID NO:2

<400>  135

Met Phe Gln Glu Ala Leu Lys Leu Lys
1               5

<210>  136
<211>  9
<212>  PRT
<213>  Artificial

<220>
<223>  fragment of SEQ ID NO:2

<400>  136

Lys Val Ile Asn Tyr Leu Val Met Leu
1               5

<210>  137
<211>  9
<212>  PRT
<213>  Artificial

<220>
<223>  fragment of SEQ ID NO:2

<400>  137

Phe Leu Leu His Lys Tyr Arg Val Lys
1               5

<210>  138
<211>  9
<212>  PRT
<213>  Artificial

<220>
<223>  fragment of SEQ ID NO:2

<400> 138

Lys Val Ala Glu Leu Val His Phe Leu
1 5

<210> 139
<211> 9
<212> PRT
<213> Artificial

<220>
<223> fragment of SEQ ID NO:2

<400> 139

Leu Glu Gln Arg Ser Pro His Cys Lys
1 5

<210> 140
<211> 9
<212> PRT
<213> Artificial

<220>
<223> fragment of SEQ ID NO:2

<400> 140

Arg Val Lys Glu Pro Val Thr Lys Ala
1 5

<210> 141
<211> 9
<212> PRT
<213> Artificial

<220>
<223> fragment of SEQ ID NO:2

<400> 141

Leu Glu Ser Val Ile Lys Asn Tyr Lys
1 5

<210> 142
<211> 9
<212> PRT
<213> Artificial

<220>
<223> fragment of SEQ ID NO:2

<400> 142

Ser Tyr Glu Lys Val Ile Asn Tyr Leu
1 5

<210> 143
<211> 9
<212> PRT
<213> Artificial

<220>

<223> fragment of SEQ ID NO:2

<400> 143

Phe Tyr Gly Glu Pro Arg Lys Leu Leu
1 5

<210> 144
<211> 9
<212> PRT
<213> Artificial

<220>
<223> fragment of SEQ ID NO:2

<400> 144

Val Tyr Val Gly Lys Glu His Met Phe
1 5

<210> 145
<211> 9
<212> PRT
<213> Artificial

<220>
<223> fragment of SEQ ID NO:2

<400> 145

Val Tyr Tyr Thr Leu Trp Ser Gln Phe
1 5

<210> 146
<211> 9
<212> PRT
<213> Artificial

<220>
<223> fragment of SEQ ID NO:2

<400> 146

Asn Tyr Lys Arg Tyr Phe Pro Val Ile
1 5

<210> 147
<211> 9
<212> PRT
<213> Artificial

<220>
<223> fragment of SEQ ID NO:2

<400> 147

Met Phe Tyr Gly Glu Pro Arg Lys Leu
1 5

<210> 148
<211> 9
<212> PRT
<213> Artificial

```
<220>
<223>  fragment of SEQ ID NO:2

<400>  148

Lys Val Ile Asn Tyr Leu Val Met Leu
1               5


<210>  149
<211>  9
<212>  PRT
<213>  Artificial

<220>
<223>  fragment of SEQ ID NO:2

<400>  149

Lys Val Ala Glu Leu Val His Phe Leu
1               5


<210>  150
<211>  9
<212>  PRT
<213>  Artificial

<220>
<223>  fragment of SEQ ID NO:2

<400>  150

Lys Tyr Arg Val Lys Glu Pro Val Thr
1               5


<210>  151
<211>  9
<212>  PRT
<213>  Artificial

<220>
<223>  fragment of SEQ ID NO:2

<400>  151

Cys Tyr Pro Ser Leu Tyr Glu Glu Val
1               5


<210>  152
<211>  9
<212>  PRT
<213>  Artificial

<220>
<223>  fragment of SEQ ID NO:2

<400>  152

Lys Ala Ala Leu Leu Ile Ile Val Leu
1               5


<210>  153
<211>  9
```

<212> PRT
<213> Artificial

<220>
<223> fragment of SEQ ID NO:2

<400> 153

Val Ala Glu Leu Val His Phe Leu Leu
1 5

<210> 154
<211> 9
<212> PRT
<213> Artificial

<220>
<223> fragment of SEQ ID NO:2

<400> 154

Ser Ser Ile Ser Val Tyr Tyr Thr Leu
1 5

<210> 155
<211> 9
<212> PRT
<213> Artificial

<220>
<223> fragment of SEQ ID NO:2

<400> 155

Leu Ile Ile Val Leu Gly Val Ile Leu
1 5

<210> 156
<211> 9
<212> PRT
<213> Artificial

<220>
<223> fragment of SEQ ID NO:2

<400> 156

His Ser Met Pro Lys Ala Ala Leu Leu
1 5

<210> 157
<211> 9
<212> PRT
<213> Artificial

<220>
<223> fragment of SEQ ID NO:2

<400> 157

Glu Ala Gln Gly Glu Asp Leu Gly Leu
1 5

<210> 158
<211> 9
<212> PRT
<213> Artificial

<220>
<223> fragment of SEQ ID NO:2

<400> 158

Leu Gly Leu Ser Cys Asp Ser Met Leu
1 5

<210> 159
<211> 9
<212> PRT
<213> Artificial

<220>
<223> fragment of SEQ ID NO:2

<400> 159

Asp Leu Glu Ala Gln Gly Glu Asp Leu
1 5

<210> 160
<211> 9
<212> PRT
<213> Artificial

<220>
<223> fragment of SEQ ID NO:2

<400> 160

Tyr Ile Leu Val Thr Ala Leu Gly Leu
1 5

<210> 161
<211> 9
<212> PRT
<213> Artificial

<220>
<223> fragment of SEQ ID NO:2

<400> 161

Glu Pro Val Thr Lys Ala Glu Met Leu
1 5

<210> 162
<211> 9
<212> PRT
<213> Artificial

<220>
<223> fragment of SEQ ID NO:2

<400> 162

Ser Val Met Gly Val Tyr Val Gly Lys
1 5

<210> 163
<211> 9
<212> PRT
<213> Artificial

<220>
<223> fragment of SEQ ID NO:2

<400> 163

Ile Val Leu Gly Val Ile Leu Thr Lys
1 5

<210> 164
<211> 9
<212> PRT
<213> Artificial

<220>
<223> fragment of SEQ ID NO:2

<400> 164

Gln Val Ile Phe Gly Thr Asp Val Lys
1 5

<210> 165
<211> 9
<212> PRT
<213> Artificial

<220>
<223> fragment of SEQ ID NO:2

<400> 165

Glu Ser Val Ile Lys Asn Tyr Lys Arg
1 5

<210> 166
<211> 9
<212> PRT
<213> Artificial

<220>
<223> fragment of SEQ ID NO:2

<400> 166

Glu Thr Thr Ser Ser Ser Asp Ser Lys
1 5

<210> 167
<211> 9
<212> PRT
<213> Artificial

<220>
<223> fragment of SEQ ID NO:2

<400> 167

Glu Leu Val His Phe Leu Leu His Lys
1 5

<210> 168
<211> 9
<212> PRT
<213> Artificial

<220>
<223> fragment of SEQ ID NO:2

<400> 168

Glu Val Ile Trp Glu Ala Leu Ser Val
1 5

<210> 169
<211> 9
<212> PRT
<213> Artificial

<220>
<223> fragment of SEQ ID NO:2

<400> 169

His Met Phe Tyr Gly Glu Pro Arg Lys
1 5

<210> 170
<211> 9
<212> PRT
<213> Artificial

<220>
<223> fragment of SEQ ID NO:2

<400> 170

Lys Val Ala Glu Leu Val His Phe Leu
1 5

<210> 171
<211> 9
<212> PRT
<213> Artificial

<220>
<223> fragment of SEQ ID NO:2

<400> 171

Lys Val Ile Asn Tyr Leu Val Met Leu
1 5

<210> 172
<211> 9
<212> PRT
<213> Artificial

<220>
<223> fragment of SEQ ID NO:2

<400> 172

Gly Val Tyr Val Gly Lys Glu His Met
1 5

<210> 173
<211> 9
<212> PRT
<213> Artificial

<220>
<223> fragment of SEQ ID NO:2

<400> 173

Gln Val Pro Gly Ser Asp Pro Ala His
1 5

<210> 174
<211> 9
<212> PRT
<213> Artificial

<220>
<223> fragment of SEQ ID NO:2

<400> 174

Phe Leu Leu His Lys Tyr Arg Val Lys
1 5

<210> 175
<211> 9
<212> PRT
<213> Artificial

<220>
<223> fragment of SEQ ID NO:2

<400> 175

Val Gln Glu Asn Tyr Leu Glu Tyr Arg
1 5

<210> 176
<211> 9
<212> PRT
<213> Artificial

<220>
<223> fragment of SEQ ID NO:2

<400> 176

Arg Val Lys Glu Pro Val Thr Lys Ala
1 5

<210> 177
<211> 9
<212> PRT
<213> Artificial

<220>

<223> fragment of SEQ ID NO:2

<400> 177

Glu Val Leu Gly Glu Glu Gln Glu Gly
1 5

<210> 178
<211> 9
<212> PRT
<213> Artificial

<220>
<223> fragment of SEQ ID NO:2

<400> 178

Glu His Met Phe Tyr Gly Glu Pro Arg
1 5

<210> 179
<211> 9
<212> PRT
<213> Artificial

<220>
<223> fragment of SEQ ID NO:2

<400> 179

Glu Phe Met Phe Gln Glu Ala Leu Lys
1 5

<210> 180
<211> 9
<212> PRT
<213> Artificial

<220>
<223> fragment of SEQ ID NO:2

<400> 180

Asp Val Lys Glu Val Asp Pro Ala Gly
1 5

<210> 181
<211> 9
<212> PRT
<213> Artificial

<220>
<223> fragment of SEQ ID NO:2

<400> 181

Glu Val Asp Pro Ala Gly His Ser Tyr
1 5

<210> 182
<211> 9
<212> PRT
<213> Artificial

<220>
<223> fragment of SEQ ID NO:2

<400> 182

Val Gln Glu Asn Tyr Leu Glu Tyr Arg
1 5

<210> 183
<211> 9
<212> PRT
<213> Artificial

<220>
<223> fragment of SEQ ID NO:2

<400> 183

Arg Tyr Phe Pro Val Ile Phe Gly Lys
1 5

<210> 184
<211> 9
<212> PRT
<213> Artificial

<220>
<223> fragment of SEQ ID NO:2

<400> 184

Asn Tyr Leu Val Met Leu Asn Ala Arg
1 5

<210> 185
<211> 9
<212> PRT
<213> Artificial

<220>
<223> fragment of SEQ ID NO:2

<400> 185

Ile Val Leu Gly Val Ile Leu Thr Lys
1 5

<210> 186
<211> 9
<212> PRT
<213> Artificial

<220>
<223> fragment of SEQ ID NO:2

<400> 186

Ser Val Met Gly Val Tyr Val Gly Lys
1 5

<210> 187
<211> 9

<212> PRT
<213> Artificial

<220>
<223> fragment of SEQ ID NO:2

<400> 187

His Met Phe Tyr Gly Glu Pro Arg Lys
1 5

<210> 188
<211> 9
<212> PRT
<213> Artificial

<220>
<223> fragment of SEQ ID NO:2

<400> 188

Glu Leu Val His Phe Leu Leu His Lys
1 5

<210> 189
<211> 9
<212> PRT
<213> Artificial

<220>
<223> fragment of SEQ ID NO:2

<400> 189

Gln Val Ile Phe Gly Thr Asp Val Lys
1 5

<210> 190
<211> 9
<212> PRT
<213> Artificial

<220>
<223> fragment of SEQ ID NO:2

<400> 190

Lys Val Ile Asn Tyr Leu Val Met Leu
1 5

<210> 191
<211> 9
<212> PRT
<213> Artificial

<220>
<223> fragment of SEQ ID NO:2

<400> 191

Ala Leu Leu Ile Ile Val Leu Gly Val
1 5

<210> 192
<211> 9
<212> PRT
<213> Artificial

<220>
<223> fragment of SEQ ID NO:2

<400> 192

Lys Val Ala Glu Leu Val His Phe Leu
1 5

<210> 193
<211> 9
<212> PRT
<213> Artificial

<220>
<223> fragment of SEQ ID NO:2

<400> 193

Val Ile Trp Glu Ala Leu Ser Val Met
1 5

<210> 194
<211> 9
<212> PRT
<213> Artificial

<220>
<223> fragment of SEQ ID NO:2

<400> 194

Arg Gln Val Pro Gly Ser Asp Pro Ala
1 5

<210> 195
<211> 9
<212> PRT
<213> Artificial

<220>
<223> fragment of SEQ ID NO:2

<400> 195

Lys Leu Lys Val Ala Glu Leu Val His
1 5

<210> 196
<211> 9
<212> PRT
<213> Artificial

<220>
<223> fragment of SEQ ID NO:2

<400> 196

Arg Val Lys Glu Pro Val Thr Lys Ala
1 5

<210> 197
<211> 9
<212> PRT
<213> Artificial

<220>
<223> fragment of SEQ ID NO:2

<400> 197

Phe Met Phe Gln Glu Ala Leu Lys Leu
1               5

<210> 198
<211> 9
<212> PRT
<213> Artificial

<220>
<223> fragment of SEQ ID NO:2

<400> 198

Leu Leu Ile Ile Val Leu Gly Val Ile
1               5

<210> 199
<211> 9
<212> PRT
<213> Artificial

<220>
<223> fragment of SEQ ID NO:2

<400> 199

Tyr Ile Leu Val Thr Ala Leu Gly Leu
1               5

<210> 200
<211> 9
<212> PRT
<213> Artificial

<220>
<223> fragment of SEQ ID NO:2

<400> 200

His Tyr Glu Phe Leu Trp Gly Ser Lys
1               5

<210> 201
<211> 9
<212> PRT
<213> Artificial

<220>
<223> fragment of SEQ ID NO:2

<400> 201

Ser Val Tyr Tyr Thr Leu Trp Ser Gln
1               5

<210>  202
<211>  9
<212>  PRT
<213>  Artificial

<220>
<223>  fragment of SEQ ID NO:2

<400>  202

Glu Val Asp Pro Ala Gly His Ser Tyr
1               5

<210>  203
<211>  9
<212>  PRT
<213>  Artificial

<220>
<223>  fragment of SEQ ID NO:2

<400>  203

Thr Gln Asp Trp Val Gln Glu Asn Tyr
1               5

<210>  204
<211>  9
<212>  PRT
<213>  Artificial

<220>
<223>  fragment of SEQ ID NO:2

<400>  204

Met Leu Glu Ser Val Ile Lys Asn Tyr
1               5

<210>  205
<211>  9
<212>  PRT
<213>  Artificial

<220>
<223>  fragment of SEQ ID NO:2

<400>  205

Ala Ser Ser Ser Ile Ser Val Tyr Tyr
1               5

<210>  206
<211>  9
<212>  PRT
<213>  Artificial

<220>
<223>  fragment of SEQ ID NO:2

<400> 206

Thr Ser Tyr Glu Lys Val Ile Asn Tyr
1 5

<210> 207
<211> 9
<212> PRT
<213> Artificial

<220>
<223> fragment of SEQ ID NO:2

<400> 207

Trp Val Gln Glu Asn Tyr Leu Glu Tyr
1 5

<210> 208
<211> 9
<212> PRT
<213> Artificial

<220>
<223> fragment of SEQ ID NO:2

<400> 208

Leu Val His Phe Leu Leu His Lys Tyr
1 5

<210> 209
<211> 9
<212> PRT
<213> Artificial

<220>
<223> fragment of SEQ ID NO:2

<400> 209

Ser Val Ile Lys Asn Tyr Lys Arg Tyr
1 5

<210> 210
<211> 9
<212> PRT
<213> Artificial

<220>
<223> fragment of SEQ ID NO:2

<400> 210

Ser Ser Asp Ser Lys Glu Glu Glu Val
1 5

<210> 211
<211> 9
<212> PRT
<213> Artificial

<220>

<223> fragment of SEQ ID NO:2

<400> 211

Gly Ala Ser Ser Ser Ile Ser Val Tyr
1 5

<210> 212
<211> 9
<212> PRT
<213> Artificial

<220>
<223> fragment of SEQ ID NO:2

<400> 212

Ser Val Asp Pro Ala Gln Leu Glu Phe
1 5

<210> 213
<211> 9
<212> PRT
<213> Artificial

<220>
<223> fragment of SEQ ID NO:2

<400> 213

Gly Thr Asp Val Lys Glu Val Asp Pro
1 5

<210> 214
<211> 9
<212> PRT
<213> Artificial

<220>
<223> fragment of SEQ ID NO:2

<400> 214

Glu Ala Leu Ser Val Met Gly Val Tyr
1 5

<210> 215
<211> 9
<212> PRT
<213> Artificial

<220>
<223> fragment of SEQ ID NO:2

<400> 215

Tyr Val Gly Lys Glu His Met Phe Tyr
1 5

<210> 216
<211> 9
<212> PRT
<213> Artificial

<220>
<223> fragment of SEQ ID NO:2

<400> 216

Val Pro Gly Ser Asp Pro Ala His Tyr
1 5

<210> 217
<211> 9
<212> PRT
<213> Artificial

<220>
<223> fragment of SEQ ID NO:2

<400> 217

Gly Ser Asp Pro Ala His Tyr Glu Phe
1 5

<210> 218
<211> 9
<212> PRT
<213> Artificial

<220>
<223> fragment of SEQ ID NO:2

<400> 218

Ser Lys Ala His Ala Glu Thr Ser Tyr
1 5

<210> 219
<211> 9
<212> PRT
<213> Artificial

<220>
<223> fragment of SEQ ID NO:2

<400> 219

Leu Asn Ala Arg Glu Pro Ile Cys Tyr
1 5

<210> 220
<211> 9
<212> PRT
<213> Artificial

<220>
<223> fragment of SEQ ID NO:2

<400> 220

Glu Pro Ile Cys Tyr Pro Ser Leu Tyr
1 5

<210> 221
<211> 9

<212> PRT
<213> Artificial

<220>
<223> fragment of SEQ ID NO:2

<400> 221

Tyr Gly Glu Pro Arg Lys Leu Leu Thr
1               5

<210> 222
<211> 9
<212> PRT
<213> Artificial

<220>
<223> fragment of SEQ ID NO:2

<400> 222

Ala Leu Lys Leu Lys Val Ala Glu Leu
1               5

<210> 223
<211> 9
<212> PRT
<213> Artificial

<220>
<223> fragment of SEQ ID NO:2

<400> 223

Ala Leu Leu Ile Ile Val Leu Gly Val
1               5

<210> 224
<211> 9
<212> PRT
<213> Artificial

<220>
<223> fragment of SEQ ID NO:2

<400> 224

Leu Leu Ile Ile Val Leu Gly Val Ile
1               5

<210> 225
<211> 9
<212> PRT
<213> Artificial

<220>
<223> fragment of SEQ ID NO:2

<400> 225

Lys Val Ala Glu Leu Val His Phe Leu
1               5

<210> 226
<211> 9
<212> PRT
<213> Artificial

<220>
<223> fragment of SEQ ID NO:2

<400> 226

Leu Ile Ile Val Leu Gly Val Ile Leu
1 5

<210> 227
<211> 9
<212> PRT
<213> Artificial

<220>
<223> fragment of SEQ ID NO:2

<400> 227

Tyr Ile Leu Val Thr Ala Leu Gly Leu
1 5

<210> 228
<211> 9
<212> PRT
<213> Artificial

<220>
<223> fragment of SEQ ID NO:2

<400> 228

Ala Leu Ser Val Met Gly Val Tyr Val
1 5

<210> 229
<211> 9
<212> PRT
<213> Artificial

<220>
<223> fragment of SEQ ID NO:2

<400> 229

Lys Val Ile Asn Tyr Leu Val Met Leu
1 5

<210> 230
<211> 9
<212> PRT
<213> Artificial

<220>
<223> fragment of SEQ ID NO:2

<400> 230

Val Leu Gly Glu Glu Gln Glu Gly Val
1 5

<210> 231
<211> 9
<212> PRT
<213> Artificial

<220>
<223> fragment of SEQ ID NO:2

<400> 231

Phe Met Phe Gln Glu Ala Leu Lys Leu
1 5

<210> 232
<211> 9
<212> PRT
<213> Artificial

<220>
<223> fragment of SEQ ID NO:2

<400> 232

Lys Ala Ala Leu Leu Ile Ile Val Leu
1 5

<210> 233
<211> 9
<212> PRT
<213> Artificial

<220>
<223> fragment of SEQ ID NO:2

<400> 233

Ser Met Leu Gly Asp Gly His Ser Met
1 5

<210> 234
<211> 9
<212> PRT
<213> Artificial

<220>
<223> fragment of SEQ ID NO:2

<400> 234

Ser Leu Tyr Glu Glu Val Leu Gly Glu
1 5

<210> 235
<211> 9
<212> PRT
<213> Artificial

<220>
<223> fragment of SEQ ID NO:2

<400> 235

Thr Lys Ala Glu Met Leu Glu Ser Val
1               5

<210> 236
<211> 9
<212> PRT
<213> Artificial

<220>
<223> fragment of SEQ ID NO:2

<400> 236

Ile Ile Val Leu Gly Val Ile Leu Thr
1               5

<210> 237
<211> 9
<212> PRT
<213> Artificial

<220>
<223> fragment of SEQ ID NO:2

<400> 237

Val Ile Trp Glu Ala Leu Ser Val Met
1               5

<210> 238
<211> 9
<212> PRT
<213> Artificial

<220>
<223> fragment of SEQ ID NO:2

<400> 238

Phe Leu Trp Gly Ser Lys Ala His Ala
1               5

<210> 239
<211> 9
<212> PRT
<213> Artificial

<220>
<223> fragment of SEQ ID NO:2

<400> 239

Ser Met Pro Lys Ala Ala Leu Leu Ile
1               5

<210> 240
<211> 9
<212> PRT
<213> Artificial

<220>
<223> fragment of SEQ ID NO:2

<400> 240

Val Met Leu Asn Ala Arg Glu Pro Ile
1               5

<210> 241
<211> 9
<212> PRT
<213> Artificial

<220>
<223> fragment of SEQ ID NO:2

<400> 241

Asp Leu Glu Ala Gln Gly Glu Asp Leu
1               5

<210> 242
<211> 9
<212> PRT
<213> Artificial

<220>
<223> fragment of SEQ ID NO:2

<400> 242

Ile Val Leu Gly Val Ile Leu Thr Lys
1               5

<210> 243
<211> 9
<212> PRT
<213> Artificial

<220>
<223> fragment of SEQ ID NO:2

<400> 243

Gln Val Ile Phe Gly Thr Asp Val Lys
1               5

<210> 244
<211> 9
<212> PRT
<213> Artificial

<220>
<223> fragment of SEQ ID NO:2

<400> 244

Ser Val Met Gly Val Tyr Val Gly Lys
1               5

<210> 245
<211> 9
<212> PRT
<213> Artificial

<220>

<223> fragment of SEQ ID NO:2

<400> 245

Phe Leu Leu His Lys Tyr Arg Val Lys
1 5

<210> 246
<211> 9
<212> PRT
<213> Artificial

<220>
<223> fragment of SEQ ID NO:2

<400> 246

Lys Leu Lys Val Ala Glu Leu Val His
1 5

<210> 247
<211> 9
<212> PRT
<213> Artificial

<220>
<223> fragment of SEQ ID NO:2

<400> 247

Glu Val Asp Pro Ala Gly His Ser Tyr
1 5

<210> 248
<211> 9
<212> PRT
<213> Artificial

<220>
<223> fragment of SEQ ID NO:2

<400> 248

Glu Leu Val His Phe Leu Leu His Lys
1 5

<210> 249
<211> 9
<212> PRT
<213> Artificial

<220>
<223> fragment of SEQ ID NO:2

<400> 249

Lys Val Ile Asn Tyr Leu Val Met Leu
1 5

<210> 250
<211> 9
<212> PRT
<213> Artificial

<220>
<223> fragment of SEQ ID NO:2

<400> 250

Ser Val Asp Pro Ala Gln Leu Glu Phe
1 5

<210> 251
<211> 9
<212> PRT
<213> Artificial

<220>
<223> fragment of SEQ ID NO:2

<400> 251

Val Ile Phe Gly Lys Ala Ser Glu Phe
1 5

<210> 252
<211> 9
<212> PRT
<213> Artificial

<220>
<223> fragment of SEQ ID NO:2

<400> 252

Glu Val Ile Trp Glu Ala Leu Ser Val
1 5

<210> 253
<211> 9
<212> PRT
<213> Artificial

<220>
<223> fragment of SEQ ID NO:2

<400> 253

Asp Leu Gly Leu Met Gly Ala Gln Glu
1 5

<210> 254
<211> 9
<212> PRT
<213> Artificial

<220>
<223> fragment of SEQ ID NO:2

<400> 254

Glu Val Ser Ala Ala Gly Ser Ser Ser
1 5

<210> 255
<211> 9

<212> PRT
<213> Artificial

<220>
<223> fragment of SEQ ID NO:2

<400> 255

Ser Val Ile Lys Asn Tyr Lys Arg Tyr
1               5

<210> 256
<211> 9
<212> PRT
<213> Artificial

<220>
<223> fragment of SEQ ID NO:2

<400> 256

Pro Val Ile Phe Gly Lys Ala Ser Glu
1               5

<210> 257
<211> 9
<212> PRT
<213> Artificial

<220>
<223> fragment of SEQ ID NO:2

<400> 257

Leu Val Thr Ala Leu Gly Leu Ser Cys
1               5

<210> 258
<211> 9
<212> PRT
<213> Artificial

<220>
<223> fragment of SEQ ID NO:2

<400> 258

Leu Leu Ile Ile Val Leu Gly Val Ile
1               5

<210> 259
<211> 9
<212> PRT
<213> Artificial

<220>
<223> fragment of SEQ ID NO:2

<400> 259

Ala Leu Ser Val Met Gly Val Tyr Val
1               5

<210> 260
<211> 9
<212> PRT
<213> Artificial

<220>
<223> fragment of SEQ ID NO:2

<400> 260

Trp Val Gln Glu Asn Tyr Leu Glu Tyr
1 5

<210> 261
<211> 9
<212> PRT
<213> Artificial

<220>
<223> fragment of SEQ ID NO:2

<400> 261

Ala Leu Lys Leu Lys Val Ala Glu Leu
1 5

<210> 262
<211> 9
<212> PRT
<213> Artificial

<220>
<223> fragment of SEQ ID NO:2

<400> 262

Ser Val Met Gly Val Tyr Val Gly Lys
1 5

<210> 263
<211> 9
<212> PRT
<213> Artificial

<220>
<223> fragment of SEQ ID NO:2

<400> 263

Ile Val Leu Gly Val Ile Leu Thr Lys
1 5

<210> 264
<211> 9
<212> PRT
<213> Artificial

<220>
<223> fragment of SEQ ID NO:2

<400> 264

Gln Val Ile Phe Gly Thr Asp Val Lys
1 5

<210> 265
<211> 9
<212> PRT
<213> Artificial

<220>
<223> fragment of SEQ ID NO:2

<400> 265

Glu Thr Thr Ser Ser Ser Asp Ser Lys
1 5

<210> 266
<211> 9
<212> PRT
<213> Artificial

<220>
<223> fragment of SEQ ID NO:2

<400> 266

Glu Leu Val His Phe Leu Leu His Lys
1 5

<210> 267
<211> 9
<212> PRT
<213> Artificial

<220>
<223> fragment of SEQ ID NO:2

<400> 267

Ser Val Asp Pro Ala Gln Leu Glu Phe
1 5

<210> 268
<211> 9
<212> PRT
<213> Artificial

<220>
<223> fragment of SEQ ID NO:2

<400> 268

Ala Ser Ser Ser Ile Ser Val Tyr Tyr
1 5

<210> 269
<211> 9
<212> PRT
<213> Artificial

<220>
<223> fragment of SEQ ID NO:2

<400> 269

Ser Ser Val Asp Pro Ala Gln Leu Glu
1               5

<210> 270
<211> 9
<212> PRT
<213> Artificial

<220>
<223> fragment of SEQ ID NO:2

<400> 270

Phe Leu Leu His Lys Tyr Arg Val Lys
1               5

<210> 271
<211> 9
<212> PRT
<213> Artificial

<220>
<223> fragment of SEQ ID NO:2

<400> 271

Glu Ser Val Ile Lys Asn Tyr Lys Arg
1               5

<210> 272
<211> 9
<212> PRT
<213> Artificial

<220>
<223> fragment of SEQ ID NO:2

<400> 272

Gly Thr Asp Val Lys Glu Val Asp Pro
1               5

<210> 273
<211> 9
<212> PRT
<213> Artificial

<220>
<223> fragment of SEQ ID NO:2

<400> 273

Ala Glu Met Leu Glu Ser Val Ile Lys
1               5

<210> 274
<211> 9
<212> PRT
<213> Artificial

<220>
<223> fragment of SEQ ID NO:2

<400> 274

Ala Ser Glu Phe Met Gln Val Ile Phe
1 5

<210> 275
<211> 9
<212> PRT
<213> Artificial

<220>
<223> fragment of SEQ ID NO:2

<400> 275

His Met Phe Tyr Gly Glu Pro Arg Lys
1 5

<210> 276
<211> 9
<212> PRT
<213> Artificial

<220>
<223> fragment of SEQ ID NO:2

<400> 276

Pro Ser Leu Tyr Glu Glu Val Leu Gly
1 5

<210> 277
<211> 9
<212> PRT
<213> Artificial

<220>
<223> fragment of SEQ ID NO:2

<400> 277

Ser Ser Ser Asp Ser Lys Glu Glu Glu
1 5

<210> 278
<211> 9
<212> PRT
<213> Artificial

<220>
<223> fragment of SEQ ID NO:2

<400> 278

Val Thr Lys Ala Glu Met Leu Glu Ser
1 5

<210> 279
<211> 9
<212> PRT
<213> Artificial

<220>

<223> fragment of SEQ ID NO:2

<400> 279

Leu Ser Cys Asp Ser Met Leu Gly Asp
1               5

<210> 280
<211> 9
<212> PRT
<213> Artificial

<220>
<223> fragment of SEQ ID NO:2

<400> 280

Ser Met Pro Lys Ala Ala Leu Leu Ile
1               5

<210> 281
<211> 9
<212> PRT
<213> Artificial

<220>
<223> fragment of SEQ ID NO:2

<400> 281

Glu Val Ile Trp Glu Ala Leu Ser Val
1               5

<210> 282
<211> 9
<212> PRT
<213> Artificial

<220>
<223> fragment of SEQ ID NO:2

<400> 282

Phe Tyr Gly Glu Pro Arg Lys Leu Leu
1               5

<210> 283
<211> 9
<212> PRT
<213> Artificial

<220>
<223> fragment of SEQ ID NO:2

<400> 283

Val Tyr Val Gly Lys Glu His Met Phe
1               5

<210> 284
<211> 9
<212> PRT
<213> Artificial

<220>
<223> fragment of SEQ ID NO:2

<400> 284

Ser Tyr Glu Lys Val Ile Asn Tyr Leu
1               5

<210> 285
<211> 9
<212> PRT
<213> Artificial

<220>
<223> fragment of SEQ ID NO:2

<400> 285

Val Tyr Tyr Thr Leu Trp Ser Gln Phe
1               5

<210> 286
<211> 9
<212> PRT
<213> Artificial

<220>
<223> fragment of SEQ ID NO:2

<400> 286

Asn Tyr Lys Arg Tyr Phe Pro Val Ile
1               5

<210> 287
<211> 9
<212> PRT
<213> Artificial

<220>
<223> fragment of SEQ ID NO:2

<400> 287

Met Phe Tyr Gly Glu Pro Arg Lys Leu
1               5

<210> 288
<211> 9
<212> PRT
<213> Artificial

<220>
<223> fragment of SEQ ID NO:2

<400> 288

Val Ala Glu Leu Val His Phe Leu Leu
1               5

<210> 289
<211> 9

<212> PRT
<213> Artificial

<220>
<223> fragment of SEQ ID NO:2

<400> 289

Val Asp Pro Ala Gly His Ser Tyr Ile
1 5

<210> 290
<211> 9
<212> PRT
<213> Artificial

<220>
<223> fragment of SEQ ID NO:2

<400> 290

Ser Tyr Ile Leu Val Thr Ala Leu Gly
1 5

<210> 291
<211> 9
<212> PRT
<213> Artificial

<220>
<223> fragment of SEQ ID NO:2

<400> 291

Ser Met Pro Lys Ala Ala Leu Leu Ile
1 5

<210> 292
<211> 9
<212> PRT
<213> Artificial

<220>
<223> fragment of SEQ ID NO:2

<400> 292

Val Met Leu Asn Ala Arg Glu Pro Ile
1 5

<210> 293
<211> 9
<212> PRT
<213> Artificial

<220>
<223> fragment of SEQ ID NO:2

<400> 293

Lys Val Ala Glu Leu Val His Phe Leu
1 5

<210> 294
<211> 9
<212> PRT
<213> Artificial

<220>
<223> fragment of SEQ ID NO:2

<400> 294

Arg Tyr Phe Pro Val Ile Phe Gly Lys
1 5

<210> 295
<211> 9
<212> PRT
<213> Artificial

<220>
<223> fragment of SEQ ID NO:2

<400> 295

His Ser Met Pro Lys Ala Ala Leu Leu
1 5

<210> 296
<211> 9
<212> PRT
<213> Artificial

<220>
<223> fragment of SEQ ID NO:2

<400> 296

Asn Tyr Leu Glu Tyr Arg Gln Val Pro
1 5

<210> 297
<211> 9
<212> PRT
<213> Artificial

<220>
<223> fragment of SEQ ID NO:2

<400> 297

Ser Asp Pro Ala His Tyr Glu Phe Leu
1 5

<210> 298
<211> 9
<212> PRT
<213> Artificial

<220>
<223> fragment of SEQ ID NO:2

<400> 298

Ser Ser Ile Ser Val Tyr Tyr Thr Leu
1 5

<210> 299
<211> 9
<212> PRT
<213> Artificial

<220>
<223> fragment of SEQ ID NO:2

<400> 299

Val Ile Phe Gly Lys Ala Ser Glu Phe
1 5

<210> 300
<211> 9
<212> PRT
<213> Artificial

<220>
<223> fragment of SEQ ID NO:2

<400> 300

Lys Ala Ser Glu Phe Met Gln Val Ile
1 5

<210> 301
<211> 9
<212> PRT
<213> Artificial

<220>
<223> fragment of SEQ ID NO:2

<400> 301

Leu Leu Ile Ile Val Leu Gly Val Ile
1 5

<210> 302
<211> 9
<212> PRT
<213> Artificial

<220>
<223> fragment of SEQ ID NO:2

<400> 302

Glu Leu Val His Phe Leu Leu His Lys
1 5

<210> 303
<211> 9
<212> PRT
<213> Artificial

<220>
<223> fragment of SEQ ID NO:2

<400> 303

Glu Thr Thr Ser Ser Ser Asp Ser Lys
1               5

<210>  304
<211>  9
<212>  PRT
<213>  Artificial

<220>
<223>  fragment of SEQ ID NO:2

<400>  304

Ile Val Leu Gly Val Ile Leu Thr Lys
1               5

<210>  305
<211>  9
<212>  PRT
<213>  Artificial

<220>
<223>  fragment of SEQ ID NO:2

<400>  305

Ser Val Met Gly Val Tyr Val Gly Lys
1               5

<210>  306
<211>  9
<212>  PRT
<213>  Artificial

<220>
<223>  fragment of SEQ ID NO:2

<400>  306

Glu Ser Val Ile Lys Asn Tyr Lys Arg
1               5

<210>  307
<211>  9
<212>  PRT
<213>  Artificial

<220>
<223>  fragment of SEQ ID NO:2

<400>  307

Gln Val Ile Phe Gly Thr Asp Val Lys
1               5

<210>  308
<211>  9
<212>  PRT
<213>  Artificial

<220>
<223>  fragment of SEQ ID NO:2

<400> 308

Glu Phe Met Phe Gln Glu Ala Leu Lys
1 5

<210> 309
<211> 9
<212> PRT
<213> Artificial

<220>
<223> fragment of SEQ ID NO:2

<400> 309

Ala Ala Leu Leu Ile Ile Val Leu Gly
1 5

<210> 310
<211> 9
<212> PRT
<213> Artificial

<220>
<223> fragment of SEQ ID NO:2

<400> 310

Glu Thr Ser Tyr Glu Lys Val Ile Asn
1 5

<210> 311
<211> 9
<212> PRT
<213> Artificial

<220>
<223> fragment of SEQ ID NO:2

<400> 311

Lys Val Ile Asn Tyr Leu Val Met Leu
1 5

<210> 312
<211> 9
<212> PRT
<213> Artificial

<220>
<223> fragment of SEQ ID NO:2

<400> 312

Arg Val Lys Glu Pro Val Thr Lys Ala
1 5

<210> 313
<211> 9
<212> PRT
<213> Artificial

<220>

<223> fragment of SEQ ID NO:2

<400> 313

Val Leu Gly Val Ile Leu Thr Lys Asp
1 5

<210> 314
<211> 9
<212> PRT
<213> Artificial

<220>
<223> fragment of SEQ ID NO:2

<400> 314

Glu Ala Leu Lys Leu Lys Val Ala Glu
1 5

<210> 315
<211> 9
<212> PRT
<213> Artificial

<220>
<223> fragment of SEQ ID NO:2

<400> 315

Lys Ala Ala Leu Leu Ile Ile Val Leu
1 5

<210> 316
<211> 9
<212> PRT
<213> Artificial

<220>
<223> fragment of SEQ ID NO:2

<400> 316

Ile Ile Val Leu Gly Val Ile Leu Thr
1 5

<210> 317
<211> 9
<212> PRT
<213> Artificial

<220>
<223> fragment of SEQ ID NO:2

<400> 317

Cys Ala Pro Glu Glu Val Ile Trp Glu
1 5

<210> 318
<211> 9
<212> PRT
<213> Artificial

<220>
<223> fragment of SEQ ID NO:2

<400> 318

Glu His Met Phe Tyr Gly Glu Pro Arg
1 5

<210> 319
<211> 9
<212> PRT
<213> Artificial

<220>
<223> fragment of SEQ ID NO:2

<400> 319

Asn Tyr Leu Val Met Leu Asn Ala Arg
1 5

<210> 320
<211> 9
<212> PRT
<213> Artificial

<220>
<223> fragment of SEQ ID NO:2

<400> 320

Asp Leu Gly Leu Met Gly Ala Gln Glu
1 5

<210> 321
<211> 9
<212> PRT
<213> Artificial

<220>
<223> fragment of SEQ ID NO:2

<400> 321

Glu Met Leu Glu Ser Val Ile Lys Asn
1 5

<210> 322
<211> 15
<212> PRT
<213> Artificial

<220>
<223> fragment of SEQ ID NO:2

<400> 322

Pro Ala His Tyr Glu Phe Leu Trp Gly Ser Lys Ala His Ala Glu
1 5 10 15

<210> 323
<211> 15

<212> PRT
<213> Artificial

<220>
<223> fragment of SEQ ID NO:2

<400> 323

Gln Leu Glu Phe Met Phe Gln Glu Ala Leu Lys Leu Lys Val Ala
1 5 10 15

<210> 324
<211> 15
<212> PRT
<213> Artificial

<220>
<223> fragment of SEQ ID NO:2

<400> 324

Gly His Ser Tyr Ile Leu Val Thr Ala Leu Gly Leu Ser Cys Asp
1 5 10 15

<210> 325
<211> 15
<212> PRT
<213> Artificial

<220>
<223> fragment of SEQ ID NO:2

<400> 325

Ile Asn Tyr Leu Val Met Leu Asn Ala Arg Glu Pro Ile Cys Tyr
1 5 10 15

<210> 326
<211> 15
<212> PRT
<213> Artificial

<220>
<223> fragment of SEQ ID NO:2

<400> 326

Tyr Leu Glu Tyr Arg Gln Val Pro Gly Ser Asp Pro Ala His Tyr
1 5 10 15

<210> 327
<211> 15
<212> PRT
<213> Artificial

<220>
<223> fragment of SEQ ID NO:2

<400> 327

Lys Arg Tyr Phe Pro Val Ile Phe Gly Lys Ala Ser Glu Phe Met
1 5 10 15

<210> 328
<211> 15
<212> PRT
<213> Artificial

<220>
<223> fragment of SEQ ID NO:2

<400> 328

Ala Ala Leu Leu Ile Ile Val Leu Gly Val Ile Leu Thr Lys Asp
1 5 10 15

<210> 329
<211> 15
<212> PRT
<213> Artificial

<220>
<223> fragment of SEQ ID NO:2

<400> 329

Gly Glu Asp Leu Gly Leu Met Gly Ala Gln Glu Pro Thr Gly Glu
1 5 10 15

<210> 330
<211> 15
<212> PRT
<213> Artificial

<220>
<223> fragment of SEQ ID NO:2

<400> 330

Ser Glu Phe Met Gln Val Ile Phe Gly Thr Asp Val Lys Glu Val
1 5 10 15

<210> 331
<211> 15
<212> PRT
<213> Artificial

<220>
<223> fragment of SEQ ID NO:2

<400> 331

Glu Val Ile Trp Glu Ala Leu Ser Val Met Gly Val Tyr Val Gly
1 5 10 15

<210> 332
<211> 15
<212> PRT
<213> Artificial

<220>
<223> fragment of SEQ ID NO:2

<400> 332

Gln Glu Ala Leu Lys Leu Lys Val Ala Glu Leu Val His Phe Leu
1 5 10 15

<210> 333
<211> 15
<212> PRT
<213> Artificial

<220>
<223> fragment of SEQ ID NO:2

<400> 333

Glu Thr Ser Tyr Glu Lys Val Ile Asn Tyr Leu Val Met Leu Asn
1 5 10 15

<210> 334
<211> 15
<212> PRT
<213> Artificial

<220>
<223> fragment of SEQ ID NO:2

<400> 334

Pro Ile Cys Tyr Pro Ser Leu Tyr Glu Glu Val Leu Gly Glu Glu
1 5 10 15

<210> 335
<211> 15
<212> PRT
<213> Artificial

<220>
<223> fragment of SEQ ID NO:2

<400> 335

Ile Lys Asn Tyr Lys Arg Tyr Phe Pro Val Ile Phe Gly Lys Ala
1 5 10 15

<210> 336
<211> 15
<212> PRT
<213> Artificial

<220>
<223> fragment of SEQ ID NO:2

<400> 336

Tyr Lys Arg Tyr Phe Pro Val Ile Phe Gly Lys Ala Ser Glu Phe
1 5 10 15

<210> 337
<211> 15
<212> PRT
<213> Artificial

<220>
<223> fragment of SEQ ID NO:2

<400> 337

Tyr Phe Pro Val Ile Phe Gly Lys Ala Ser Glu Phe Met Gln Val
1 5 10 15

<210> 338
<211> 15
<212> PRT
<213> Artificial

<220>
<223> fragment of SEQ ID NO:2

<400> 338

Lys Ala Ala Leu Leu Ile Ile Val Leu Gly Val Ile Leu Thr Lys
1 5 10 15

<210> 339
<211> 15
<212> PRT
<213> Artificial

<220>
<223> fragment of SEQ ID NO:2

<400> 339

Lys Glu His Met Phe Tyr Gly Glu Pro Arg Lys Leu Leu Thr Gln
1 5 10 15

<210> 340
<211> 15
<212> PRT
<213> Artificial

<220>
<223> fragment of SEQ ID NO:2

<400> 340

Glu Lys Val Ile Asn Tyr Leu Val Met Leu Asn Ala Arg Glu Pro
1 5 10 15

<210> 341
<211> 15
<212> PRT
<213> Artificial

<220>
<223> fragment of SEQ ID NO:2

<400> 341

Leu Gly Leu Met Gly Ala Gln Glu Pro Thr Gly Glu Glu Glu Glu
1 5 10 15

<210> 342
<211> 15
<212> PRT
<213> Artificial

<220>
<223> fragment of SEQ ID NO:2

<400> 342

Pro Ala Gln Leu Glu Phe Met Phe Gln Glu Ala Leu Lys Leu Lys
1 5 10 15

<210> 343
<211> 15
<212> PRT
<213> Artificial

<220>
<223> fragment of SEQ ID NO:2

<400> 343

Leu Glu Ser Val Ile Lys Asn Tyr Lys Arg Tyr Phe Pro Val Ile
1 5 10 15

<210> 344
<211> 15
<212> PRT
<213> Artificial

<220>
<223> fragment of SEQ ID NO:2

<400> 344

Val Met Gly Val Tyr Val Gly Lys Glu His Met Phe Tyr Gly Glu
1 5 10 15

<210> 345
<211> 15
<212> PRT
<213> Artificial

<220>
<223> fragment of SEQ ID NO:2

<400> 345

Gln Val Ile Phe Gly Thr Asp Val Lys Glu Val Asp Pro Ala Gly
1 5 10 15

<210> 346
<211> 15
<212> PRT
<213> Artificial

<220>
<223> fragment of SEQ ID NO:2

<400> 346

Glu His Met Phe Tyr Gly Glu Pro Arg Lys Leu Leu Thr Gln Asp
1 5 10 15

<210> 347
<211> 15
<212> PRT
<213> Artificial

<220>

<223> fragment of SEQ ID NO:2

<400> 347

Cys Asp Ser Met Leu Gly Asp Gly His Ser Met Pro Lys Ala Ala
1 5 10 15

<210> 348
<211> 15
<212> PRT
<213> Artificial

<220>
<223> fragment of SEQ ID NO:2

<400> 348

Lys Ala Glu Met Leu Glu Ser Val Ile Lys Asn Tyr Lys Arg Tyr
1 5 10 15

<210> 349
<211> 15
<212> PRT
<213> Artificial

<220>
<223> fragment of SEQ ID NO:2

<400> 349

Ala Leu Gly Leu Ser Cys Asp Ser Met Leu Gly Asp Gly His Ser
1 5 10 15

<210> 350
<211> 15
<212> PRT
<213> Artificial

<220>
<223> fragment of SEQ ID NO:2

<400> 350

Gly Val Ile Leu Thr Lys Asp Asn Cys Ala Pro Glu Glu Val Ile
1 5 10 15

<210> 351
<211> 15
<212> PRT
<213> Artificial

<220>
<223> fragment of SEQ ID NO:2

<400> 351

Arg Lys Leu Leu Thr Gln Asp Trp Val Gln Glu Asn Tyr Leu Glu
1 5 10 15

<210> 352
<211> 15
<212> PRT
<213> Artificial

<220>
<223> fragment of SEQ ID NO:2

<400> 352

Ala Leu Lys Leu Lys Val Ala Glu Leu Val His Phe Leu Leu His
1 5 10 15

<210> 353
<211> 15
<212> PRT
<213> Artificial

<220>
<223> fragment of SEQ ID NO:2

<400> 353

Val Ala Glu Leu Val His Phe Leu Leu His Lys Tyr Arg Val Lys
1 5 10 15

<210> 354
<211> 15
<212> PRT
<213> Artificial

<220>
<223> fragment of SEQ ID NO:2

<400> 354

Ala Ala Leu Leu Ile Ile Val Leu Gly Val Ile Leu Thr Lys Asp
1 5 10 15

<210> 355
<211> 15
<212> PRT
<213> Artificial

<220>
<223> fragment of SEQ ID NO:2

<400> 355

Lys Leu Lys Val Ala Glu Leu Val His Phe Leu Leu His Lys Tyr
1 5 10 15

<210> 356
<211> 15
<212> PRT
<213> Artificial

<220>
<223> fragment of SEQ ID NO:2

<400> 356

Val His Phe Leu Leu His Lys Tyr Arg Val Lys Glu Pro Val Thr
1 5 10 15

<210> 357
<211> 15

<212> PRT
<213> Artificial

<220>
<223> fragment of SEQ ID NO:2

<400> 357

Tyr Phe Pro Val Ile Phe Gly Lys Ala Ser Glu Phe Met Gln Val
1 5 10 15

<210> 358
<211> 15
<212> PRT
<213> Artificial

<220>
<223> fragment of SEQ ID NO:2

<400> 358

Lys Glu His Met Phe Tyr Gly Glu Pro Arg Lys Leu Leu Thr Gln
1 5 10 15

<210> 359
<211> 15
<212> PRT
<213> Artificial

<220>
<223> fragment of SEQ ID NO:2

<400> 359

His Cys Lys Pro Asp Glu Asp Leu Glu Ala Gln Gly Glu Asp Leu
1 5 10 15

<210> 360
<211> 15
<212> PRT
<213> Artificial

<220>
<223> fragment of SEQ ID NO:2

<400> 360

Ser Ile Ser Val Tyr Tyr Thr Leu Trp Ser Gln Phe Asp Glu Gly
1 5 10 15

<210> 361
<211> 15
<212> PRT
<213> Artificial

<220>
<223> fragment of SEQ ID NO:2

<400> 361

Gln Leu Glu Phe Met Phe Gln Glu Ala Leu Lys Leu Lys Val Ala
1 5 10 15

<210> 362
<211> 15
<212> PRT
<213> Artificial

<220>
<223> fragment of SEQ ID NO:2

<400> 362

Glu His Met Phe Tyr Gly Glu Pro Arg Lys Leu Leu Thr Gln Asp
1 5 10 15

<210> 363
<211> 15
<212> PRT
<213> Artificial

<220>
<223> fragment of SEQ ID NO:2

<400> 363

Glu Thr Ser Tyr Glu Lys Val Ile Asn Tyr Leu Val Met Leu Asn
1 5 10 15

<210> 364
<211> 15
<212> PRT
<213> Artificial

<220>
<223> fragment of SEQ ID NO:2

<400> 364

Lys Leu Lys Val Ala Glu Leu Val His Phe Leu Leu His Lys Tyr
1 5 10 15

<210> 365
<211> 15
<212> PRT
<213> Artificial

<220>
<223> fragment of SEQ ID NO:2

<400> 365

Val Lys Glu Val Asp Pro Ala Gly His Ser Tyr Ile Leu Val Thr
1 5 10 15

<210> 366
<211> 15
<212> PRT
<213> Artificial

<220>
<223> fragment of SEQ ID NO:2

<400> 366

Ile Ile Val Leu Gly Val Ile Leu Thr Lys Asp Asn Cys Ala Pro
1 5 10 15

<210> 367
<211> 15
<212> PRT
<213> Artificial

<220>
<223> fragment of SEQ ID NO:2

<400> 367

Ile Ser Val Tyr Tyr Thr Leu Trp Ser Gln Phe Asp Glu Gly Ser
1 5 10 15

<210> 368
<211> 15
<212> PRT
<213> Artificial

<220>
<223> fragment of SEQ ID NO:2

<400> 368

Tyr Thr Leu Trp Ser Gln Phe Asp Glu Gly Ser Ser Ser Gln Glu
1 5 10 15

<210> 369
<211> 15
<212> PRT
<213> Artificial

<220>
<223> fragment of SEQ ID NO:2

<400> 369

Glu Phe Met Phe Gln Glu Ala Leu Lys Leu Lys Val Ala Glu Leu
1 5 10 15

<210> 370
<211> 15
<212> PRT
<213> Artificial

<220>
<223> fragment of SEQ ID NO:2

<400> 370

Tyr Lys Arg Tyr Phe Pro Val Ile Phe Gly Lys Ala Ser Glu Phe
1 5 10 15

<210> 371
<211> 15
<212> PRT
<213> Artificial

<220>
<223> fragment of SEQ ID NO:2

<400> 371

Lys Arg Tyr Phe Pro Val Ile Phe Gly Lys Ala Ser Glu Phe Met
1               5                   10                  15

<210> 372
<211> 15
<212> PRT
<213> Artificial

<220>
<223> fragment of SEQ ID NO:2

<400> 372

Pro Val Ile Phe Gly Lys Ala Ser Glu Phe Met Gln Val Ile Phe
1               5                   10                  15

<210> 373
<211> 15
<212> PRT
<213> Artificial

<220>
<223> fragment of SEQ ID NO:2

<400> 373

Gly His Ser Tyr Ile Leu Val Thr Ala Leu Gly Leu Ser Cys Asp
1               5                   10                  15

<210> 374
<211> 15
<212> PRT
<213> Artificial

<220>
<223> fragment of SEQ ID NO:2

<400> 374

Glu Val Ile Trp Glu Ala Leu Ser Val Met Gly Val Tyr Val Gly
1               5                   10                  15

<210> 375
<211> 15
<212> PRT
<213> Artificial

<220>
<223> fragment of SEQ ID NO:2

<400> 375

Pro Ala His Tyr Glu Phe Leu Trp Gly Ser Lys Ala His Ala Glu
1               5                   10                  15

<210> 376
<211> 15
<212> PRT
<213> Artificial

<220>
<223> fragment of SEQ ID NO:2

<400> 376

Pro Ile Cys Tyr Pro Ser Leu Tyr Glu Glu Val Leu Gly Glu Glu
1               5                   10                  15

<210> 377
<211> 15
<212> PRT
<213> Artificial

<220>
<223> fragment of SEQ ID NO:2

<400> 377

Pro Ser Leu Tyr Glu Glu Val Leu Gly Glu Glu Gln Glu Gly Val
1               5                   10                  15

<210> 378
<211> 15
<212> PRT
<213> Artificial

<220>
<223> fragment of SEQ ID NO:2

<400> 378

Glu Glu Glu Val Ser Ala Ala Gly Ser Ser Ser Pro Pro Gln Ser
1               5                   10                  15

<210> 379
<211> 15
<212> PRT
<213> Artificial

<220>
<223> fragment of SEQ ID NO:2

<400> 379

Ser Ser Ser Ile Ser Val Tyr Tyr Thr Leu Trp Ser Gln Phe Asp
1               5                   10                  15

<210> 380
<211> 15
<212> PRT
<213> Artificial

<220>
<223> fragment of SEQ ID NO:2

<400> 380

Ser Ser Ser Val Asp Pro Ala Gln Leu Glu Phe Met Phe Gln Glu
1               5                   10                  15

<210> 381
<211> 15
<212> PRT
<213> Artificial

<220>

<223> fragment of SEQ ID NO:2

<400> 381

Pro Ala Gln Leu Glu Phe Met Phe Gln Glu Ala Leu Lys Leu Lys
1 5 10 15

<210> 382
<211> 15
<212> PRT
<213> Artificial

<220>
<223> fragment of SEQ ID NO:2

<400> 382

Val Thr Ala Leu Gly Leu Ser Cys Asp Ser Met Leu Gly Asp Gly
1 5 10 15

<210> 383
<211> 15
<212> PRT
<213> Artificial

<220>
<223> fragment of SEQ ID NO:2

<400> 383

Val Leu Gly Val Ile Leu Thr Lys Asp Asn Cys Ala Pro Glu Glu
1 5 10 15

<210> 384
<211> 15
<212> PRT
<213> Artificial

<220>
<223> fragment of SEQ ID NO:2

<400> 384

Glu Phe Met Phe Gln Glu Ala Leu Lys Leu Lys Val Ala Glu Leu
1 5 10 15

<210> 385
<211> 15
<212> PRT
<213> Artificial

<220>
<223> fragment of SEQ ID NO:2

<400> 385

Phe Pro Val Ile Phe Gly Lys Ala Ser Glu Phe Met Gln Val Ile
1 5 10 15

<210> 386
<211> 15
<212> PRT
<213> Artificial

<220>
<223> fragment of SEQ ID NO:2

<400> 386

Met Gln Val Ile Phe Gly Thr Asp Val Lys Glu Val Asp Pro Ala
1 5 10 15

<210> 387
<211> 15
<212> PRT
<213> Artificial

<220>
<223> fragment of SEQ ID NO:2

<400> 387

Glu Thr Ser Tyr Glu Lys Val Ile Asn Tyr Leu Val Met Leu Asn
1 5 10 15

<210> 388
<211> 15
<212> PRT
<213> Artificial

<220>
<223> fragment of SEQ ID NO:2

<400> 388

Pro Ile Cys Tyr Pro Ser Leu Tyr Glu Glu Val Leu Gly Glu Glu
1 5 10 15

<210> 389
<211> 15
<212> PRT
<213> Artificial

<220>
<223> fragment of SEQ ID NO:2

<400> 389

Glu Glu Glu Val Ser Ala Ala Gly Ser Ser Ser Pro Pro Gln Ser
1 5 10 15

<210> 390
<211> 15
<212> PRT
<213> Artificial

<220>
<223> fragment of SEQ ID NO:2

<400> 390

Ser Pro Gln Gly Gly Ala Ser Ser Ser Ile Ser Val Tyr Tyr Thr
1 5 10 15

<210> 391
<211> 15

<212> PRT
<213> Artificial

<220>
<223> fragment of SEQ ID NO:2

<400> 391

Ser Ile Ser Val Tyr Tyr Thr Leu Trp Ser Gln Phe Asp Glu Gly
1 5 10 15

<210> 392
<211> 15
<212> PRT
<213> Artificial

<220>
<223> fragment of SEQ ID NO:2

<400> 392

Ser Ser Ser Val Asp Pro Ala Gln Leu Glu Phe Met Phe Gln Glu
1 5 10 15

<210> 393
<211> 15
<212> PRT
<213> Artificial

<220>
<223> fragment of SEQ ID NO:2

<400> 393

Pro Val Ile Phe Gly Lys Ala Ser Glu Phe Met Gln Val Ile Phe
1 5 10 15

<210> 394
<211> 15
<212> PRT
<213> Artificial

<220>
<223> fragment of SEQ ID NO:2

<400> 394

Ser Glu Phe Met Gln Val Ile Phe Gly Thr Asp Val Lys Glu Val
1 5 10 15

<210> 395
<211> 15
<212> PRT
<213> Artificial

<220>
<223> fragment of SEQ ID NO:2

<400> 395

Val Lys Glu Val Asp Pro Ala Gly His Ser Tyr Ile Leu Val Thr
1 5 10 15

<210> 396
<211> 15
<212> PRT
<213> Artificial

<220>
<223> fragment of SEQ ID NO:2

<400> 396

Gly His Ser Met Pro Lys Ala Ala Leu Leu Ile Ile Val Leu Gly
1 5 10 15

<210> 397
<211> 15
<212> PRT
<213> Artificial

<220>
<223> fragment of SEQ ID NO:2

<400> 397

Lys Ala Ala Leu Leu Ile Ile Val Leu Gly Val Ile Leu Thr Lys
1 5 10 15

<210> 398
<211> 15
<212> PRT
<213> Artificial

<220>
<223> fragment of SEQ ID NO:2

<400> 398

Ile Ile Val Leu Gly Val Ile Leu Thr Lys Asp Asn Cys Ala Pro
1 5 10 15

<210> 399
<211> 15
<212> PRT
<213> Artificial

<220>
<223> fragment of SEQ ID NO:2

<400> 399

Glu Glu Val Ile Trp Glu Ala Leu Ser Val Met Gly Val Tyr Val
1 5 10 15

<210> 400
<211> 15
<212> PRT
<213> Artificial

<220>
<223> fragment of SEQ ID NO:2

<400> 400

Asp Leu Gly Leu Met Gly Ala Gln Glu Pro Thr Gly Glu Glu Glu
1 5 10 15

<210> 401
<211> 15
<212> PRT
<213> Artificial

<220>
<223> fragment of SEQ ID NO:2

<400> 401

Thr Thr Ser Ser Ser Asp Ser Lys Glu Glu Glu Val Ser Ala Ala
1 5 10 15

<210> 402
<211> 15
<212> PRT
<213> Artificial

<220>
<223> fragment of SEQ ID NO:2

<400> 402

Tyr Lys Arg Tyr Phe Pro Val Ile Phe Gly Lys Ala Ser Glu Phe
1 5 10 15

<210> 403
<211> 15
<212> PRT
<213> Artificial

<220>
<223> fragment of SEQ ID NO:2

<400> 403

His Tyr Glu Phe Leu Trp Gly Ser Lys Ala His Ala Glu Thr Ser
1 5 10 15

<210> 404
<211> 15
<212> PRT
<213> Artificial

<220>
<223> fragment of SEQ ID NO:2

<400> 404

Lys Leu Lys Val Ala Glu Leu Val His Phe Leu Leu His Lys Tyr
1 5 10 15

<210> 405
<211> 15
<212> PRT
<213> Artificial

<220>
<223> fragment of SEQ ID NO:2

<400> 405

Lys Arg Tyr Phe Pro Val Ile Phe Gly Lys Ala Ser Glu Phe Met
1 5 10 15

<210> 406
<211> 15
<212> PRT
<213> Artificial

<220>
<223> fragment of SEQ ID NO:2

<400> 406

Pro Ala His Tyr Glu Phe Leu Trp Gly Ser Lys Ala His Ala Glu
1 5 10 15

<210> 407
<211> 15
<212> PRT
<213> Artificial

<220>
<223> fragment of SEQ ID NO:2

<400> 407

Tyr Leu Glu Tyr Arg Gln Val Pro Gly Ser Asp Pro Ala His Tyr
1 5 10 15

<210> 408
<211> 15
<212> PRT
<213> Artificial

<220>
<223> fragment of SEQ ID NO:2

<400> 408

Gln Val Ile Phe Gly Thr Asp Val Lys Glu Val Asp Pro Ala Gly
1 5 10 15

<210> 409
<211> 15
<212> PRT
<213> Artificial

<220>
<223> fragment of SEQ ID NO:2

<400> 409

Ala Glu Leu Val His Phe Leu Leu His Lys Tyr Arg Val Lys Glu
1 5 10 15

<210> 410
<211> 15
<212> PRT
<213> Artificial

<220>
<223> fragment of SEQ ID NO:2

<400> 410

Ala Glu Met Leu Glu Ser Val Ile Lys Asn Tyr Lys Arg Tyr Phe
1 5 10 15

<210> 411
<211> 15
<212> PRT
<213> Artificial

<220>
<223> fragment of SEQ ID NO:2

<400> 411

Glu Ser Val Ile Lys Asn Tyr Lys Arg Tyr Phe Pro Val Ile Phe
1 5 10 15

<210> 412
<211> 15
<212> PRT
<213> Artificial

<220>
<223> fragment of SEQ ID NO:2

<400> 412

Cys Asp Ser Met Leu Gly Asp Gly His Ser Met Pro Lys Ala Ala
1 5 10 15

<210> 413
<211> 15
<212> PRT
<213> Artificial

<220>
<223> fragment of SEQ ID NO:2

<400> 413

Trp Glu Ala Leu Ser Val Met Gly Val Tyr Val Gly Lys Glu His
1 5 10 15

<210> 414
<211> 15
<212> PRT
<213> Artificial

<220>
<223> fragment of SEQ ID NO:2

<400> 414

Glu Lys Val Ile Asn Tyr Leu Val Met Leu Asn Ala Arg Glu Pro
1 5 10 15

<210> 415
<211> 15
<212> PRT
<213> Artificial

<220>

<223> fragment of SEQ ID NO:2

<400> 415

Glu Phe Met Phe Gln Glu Ala Leu Lys Leu Lys Val Ala Glu Leu
1 5 10 15

<210> 416
<211> 15
<212> PRT
<213> Artificial

<220>
<223> fragment of SEQ ID NO:2

<400> 416

Glu His Met Phe Tyr Gly Glu Pro Arg Lys Leu Leu Thr Gln Asp
1 5 10 15

<210> 417
<211> 15
<212> PRT
<213> Artificial

<220>
<223> fragment of SEQ ID NO:2

<400> 417

His Met Phe Tyr Gly Glu Pro Arg Lys Leu Leu Thr Gln Asp Trp
1 5 10 15

<210> 418
<211> 15
<212> PRT
<213> Artificial

<220>
<223> fragment of SEQ ID NO:2

<400> 418

Val Ile Asn Tyr Leu Val Met Leu Asn Ala Arg Glu Pro Ile Cys
1 5 10 15

<210> 419
<211> 15
<212> PRT
<213> Artificial

<220>
<223> fragment of SEQ ID NO:2

<400> 419

Ile Asn Tyr Leu Val Met Leu Asn Ala Arg Glu Pro Ile Cys Tyr
1 5 10 15

<210> 420
<211> 15
<212> PRT
<213> Artificial

<220>
<223> fragment of SEQ ID NO:2

<400> 420

Gly Glu Asp Leu Gly Leu Met Gly Ala Gln Glu Pro Thr Gly Glu
1 5 10 15

<210> 421
<211> 15
<212> PRT
<213> Artificial

<220>
<223> fragment of SEQ ID NO:2

<400> 421

Met Gly Val Tyr Val Gly Lys Glu His Met Phe Tyr Gly Glu Pro
1 5 10 15

<210> 422
<211> 15
<212> PRT
<213> Artificial

<220>
<223> fragment of SEQ ID NO:2

<400> 422

Ser Ser Ser Ile Ser Val Tyr Tyr Thr Leu Trp Ser Gln Phe Asp
1 5 10 15

<210> 423
<211> 15
<212> PRT
<213> Artificial

<220>
<223> fragment of SEQ ID NO:2

<400> 423

Tyr Glu Lys Val Ile Asn Tyr Leu Val Met Leu Asn Ala Arg Glu
1 5 10 15

<210> 424
<211> 15
<212> PRT
<213> Artificial

<220>
<223> fragment of SEQ ID NO:2

<400> 424

Leu Ser Val Met Gly Val Tyr Val Gly Lys Glu His Met Phe Tyr
1 5 10 15

<210> 425
<211> 15

<212> PRT
<213> Artificial

<220>
<223> fragment of SEQ ID NO:2

<400> 425

His Phe Leu Leu His Lys Tyr Arg Val Lys Glu Pro Val Thr Lys
1 5 10 15

<210> 426
<211> 15
<212> PRT
<213> Artificial

<220>
<223> fragment of SEQ ID NO:2

<400> 426

Glu Ser Val Ile Lys Asn Tyr Lys Arg Tyr Phe Pro Val Ile Phe
1 5 10 15

<210> 427
<211> 15
<212> PRT
<213> Artificial

<220>
<223> fragment of SEQ ID NO:2

<400> 427

Ser Tyr Ile Leu Val Thr Ala Leu Gly Leu Ser Cys Asp Ser Met
1 5 10 15

<210> 428
<211> 15
<212> PRT
<213> Artificial

<220>
<223> fragment of SEQ ID NO:2

<400> 428

Asp Ser Met Leu Gly Asp Gly His Ser Met Pro Lys Ala Ala Leu
1 5 10 15

<210> 429
<211> 15
<212> PRT
<213> Artificial

<220>
<223> fragment of SEQ ID NO:2

<400> 429

Ala Ala Leu Leu Ile Ile Val Leu Gly Val Ile Leu Thr Lys Asp
1 5 10 15

<210> 430
<211> 15
<212> PRT
<213> Artificial

<220>
<223> fragment of SEQ ID NO:2

<400> 430

Ala Pro Glu Glu Val Ile Trp Glu Ala Leu Ser Val Met Gly Val
1 5 10 15

<210> 431
<211> 15
<212> PRT
<213> Artificial

<220>
<223> fragment of SEQ ID NO:2

<400> 431

Glu Glu Val Ile Trp Glu Ala Leu Ser Val Met Gly Val Tyr Val
1 5 10 15

<210> 432
<211> 15
<212> PRT
<213> Artificial

<220>
<223> fragment of SEQ ID NO:2

<400> 432

Gln Leu Glu Phe Met Phe Gln Glu Ala Leu Lys Leu Lys Val Ala
1 5 10 15

<210> 433
<211> 15
<212> PRT
<213> Artificial

<220>
<223> fragment of SEQ ID NO:2

<400> 433

Lys Leu Lys Val Ala Glu Leu Val His Phe Leu Leu His Lys Tyr
1 5 10 15

<210> 434
<211> 15
<212> PRT
<213> Artificial

<220>
<223> fragment of SEQ ID NO:2

<400> 434

Val Ala Glu Leu Val His Phe Leu Leu His Lys Tyr Arg Val Lys
1 5 10 15

<210> 435
<211> 15
<212> PRT
<213> Artificial

<220>
<223> fragment of SEQ ID NO:2

<400> 435

Lys Glu Pro Val Thr Lys Ala Glu Met Leu Glu Ser Val Ile Lys
1 5 10 15

<210> 436
<211> 15
<212> PRT
<213> Artificial

<220>
<223> fragment of SEQ ID NO:2

<400> 436

Ile Lys Asn Tyr Lys Arg Tyr Phe Pro Val Ile Phe Gly Lys Ala
1 5 10 15

<210> 437
<211> 15
<212> PRT
<213> Artificial

<220>
<223> fragment of SEQ ID NO:2

<400> 437

Lys Asn Tyr Lys Arg Tyr Phe Pro Val Ile Phe Gly Lys Ala Ser
1 5 10 15

<210> 438
<211> 15
<212> PRT
<213> Artificial

<220>
<223> fragment of SEQ ID NO:2

<400> 438

Asp Pro Ala Gly His Ser Tyr Ile Leu Val Thr Ala Leu Gly Leu
1 5 10 15

<210> 439
<211> 15
<212> PRT
<213> Artificial

<220>
<223> fragment of SEQ ID NO:2

<400> 439

```
Gly His Ser Met Pro Lys Ala Ala Leu Leu Ile Ile Val Leu Gly
1               5                   10                  15


<210>  440
<211>  15
<212>  PRT
<213>  Artificial

<220>
<223>  fragment of SEQ ID NO:2

<400>  440

Ala Leu Leu Ile Ile Val Leu Gly Val Ile Leu Thr Lys Asp Asn
1               5                   10                  15


<210>  441
<211>  15
<212>  PRT
<213>  Artificial

<220>
<223>  fragment of SEQ ID NO:2

<400>  441

Leu Leu Ile Ile Val Leu Gly Val Ile Leu Thr Lys Asp Asn Cys
1               5                   10                  15
```